# EUROPÄISCHE PATENTSCHRIFT

(11) **EP 4 069 079 B1**
(45) Veröffentlichungstag und Bekanntmachung des Hinweises auf die Patenterteilung: **02.09.2026**
(21) Anmeldenummer: 20816488.9
(22) Anmeldetag: 04.12.2020
(51) Int. Cl.: A61B 5/11, A61B 5/00, A63B 26/00

(54) **GERÄT, KIT SOWIE COMPUTERPROGRAMPRODUKT FÜR EIN SELBSTÄNDIGES, ADAPTIVES GLEICHGEWICHTSTRAINING**
DEVICE, KIT AND COMPUTER PROGRAM PRODUCT FOR INDEPENDENT ADAPTIVE BALANCE TRAINING
DISPOSITIF, KIT ET PRODUIT DE PROGRAMME INFORMATIQUE POUR APPRENTISSAGE D'ÉQUILIBRE ADAPTATIF INDÉPENDANT

(30) Priorität: 06.12.2019 EP 19214099
(43) Veröffentlichungstag der Anmeldung: 12.10.2022
(73) Patentinhaber: DizzyCure GmbH, 14195 Berlin (DE)
(72) Erfinder: ERNST, Arneborg, 14169 Berlin (DE); BASTA, Dietmar, 14656 Brieselang (DE)
(74) Vertreter: Hertin und Partner Rechts- und Patentanwälte PartG mbB
(86) Internationale Anmeldenummer: PCT/EP2020/084628
(87) Internationale Veröffentlichungsnummer: WO 2021/110913

(56) Entgegenhaltungen:
- EP-A1- 2 445 405
- AU-A1- 2017 200 112
- US-A1- 2010 076 278
- US-A1- 2012 094 814
- US-A1- 2018 140 902

## Beschreibung

Die Erfindung betrifft bevorzugt eine Vorrichtung zur durchührung eines adaptiven Gleichgewichtstrainings. Die Vorrichtung wird durch die angehängten Ansprüche definiert. Ein Kit und Computerprogrammprodukt zur Installation auf einer Vorrichtung zur Durchführung eines adaptiven Gleichgewichtstrainings werder bevorzugt ebenfalls bereitgestellt.

### Hintergrund und Stand der Technik

Im Gleichgewichtssystem des Menschen ist die Lage des Körperschwerpunktes die geregelte Größe. Dafür werden multimodale Informationen verarbeitet. Diese werden z. B. in den propriozeptiven, somatosensorischen, visuellen und auditorischen Eingängen aufgenommen. Die gesamte Verarbeitung der sensorischen Informationen und die Aktivierung der entsprechenden motorischen Efferenzen dient letztendlich dazu, den Körperschwerpunkt unter verschiedenen sensomotorischen Konditionen in einem engen Bereich zu halten. Wird das realisiert, stürzt der Mensch nicht. Bei Defiziten hinsichtlich der sensorischen Inputs, der zentralnervösen Signalverarbeitung oder bei der Auslösung der motorischen Stellreaktionen erhöht sich das Sturzrisiko.

Durch ein spezifisches Gleichgewichtstraining kann oft ein Funktionsverlust kompensiert und damit das Sturzrisiko verringert werden.

Die WO 2007/115565 offenbart beispielsweise eine mobile Gleichgewichtsprothese sowie eine Verwendung derselben für ein Gleichgewichtstraining. Hierbei wird mittels Gyrometer oder Beschleunigungssensoren die Veränderung einer Körperposition dreidimensional im Raum als Veränderung der Winkelgeschwindigkeit von Vorwärts-, Rückwärts- und/oder Seitwärtsbewegungen des Körpers bestimmt. Bei Überschreitung von auf den Bewegungsablauf bezogener Grenzen werden Aktoren zur Ausgabe eines Feedbacksignals aktiviert. Die Grenzen basieren auf alters- und geschlechtsspezifischen Normwerten. Nachteilig an der bekannten Vorrichtung ist, dass die alters- und geschlechtsspezifischen Normwerte zur Etablierung der Feedbackschwelle nur unzureichend auf die individuellen Bedürfnisse der Patienten angepasst werden können. So ist zu beobachten, dass für manche Patienten ein deutlich zu häufiges oder zu seltenes Feedbacksignal ausgegeben wird, wodurch sich der Therapieerfolg vermindert. Teilweise kann dies durch ein manuelles Nachregulieren individueller Feedbackschwellen kompensiert werden. Eine erfolgreiche manuelle Nachregulation ist jedoch aufwendig und Bedarf erfahrenem, geschultem und somit kostenintensivem medizinischen Personal. Zudem ist zu beobachten, dass die manuelle Nachregulation sehr subjektiv und häufig ungenau durchgeführt wird. Dadurch erhöht sich die Varianz des Therapieerfolgs, wodurch die durchschnittliche Effektivität der Anwendung aktuell deutlich geringer ist als methodisch möglich.

Die selbstständige Anwendung in einer Einrichtung oder im häuslichen Umfeld ist aufgrund der fehlenden spezifischen Nachregulation der Feedbackschwellen durch geschultes, medizinisches Personal nicht möglich. Außerdem fehlt aktuell die objektive Überprüfbarkeit der korrekten Durchführung des Gleichgewichtstrainings und eine daraus resultierende Interaktion mit dem Patienten.

Eine weitere Vorrichtung zur Ermittlung von Beinahestürzen wird in der Schrift EP 2 445 405 A1 offenbart.

Es besteht mithin ein Bedarf an Verbesserung oder alternativen Lösungen zur Durchführung eines Gleichgewichtstrainings.

### Aufgabe der Erfindung

Aufgabe der Erfindung ist es, eine Vorrichtung zur Durchführung eines Gleichgewichtstrainings bereitzustellen, welche die Nachteile des Standes der Technik nicht aufweist. Insbesondere war es eine Aufgabe der Erfindung, eine Vorrichtung für ein individualisiertes, und effizientes Gleichgewichtstraining bereitzustellen, welches mit geringem Aufwand, z. B. zumindest teilweise automatisiert, durchführbar ist, eine hohe Compliance aufweist und einen guten Therapieerfolg gewährleistet.

### Zusammenfassung

Gelöst wird die Aufgabe durch die Merkmale der unabhängigen Ansprüche. Vorteilhafte Ausgestaltungen der Erfindung sind in den abhängigen Ansprüchen beschrieben.

In einem ersten Aspekt betrifft die Offenlegung eine Vorrichtung zur Durchführung eines adaptiven Gleichgewichtstrainings umfassend
a) eine Prozessoreinheit
b) mindestens einen Sensor zur Messung der Veränderung einer Körperposition und/oder eines Bewegungsmusters des Trägers der Vorrichtung, welcher Messdaten an die Prozesseinheit überträgt, wobei die Prozessoreinheit konfiguriert ist aus den Messdaten Schwankungswerte zu bestimmen
c) ein Aufforderungsmodul zur Ausgabe von Anweisungen für ein Übungsprogramm umfassend mehrere Bewegungsmuster an den Träger der Vorrichtung
d) mindestens einen Signalgeber zur Ausgabe eines Feedbacksignals, wobei die Prozesseinheit konfiguriert ist, während eines Übungsprogrammes mittels des Signalgebers ein Feedbacksignal zu erzeugen, falls die Schwankungswerte eine Feedbackschwelle überschreiten
wobei die Prozessoreinheit dazu konfiguriert ist, während einer ersten Durchführung eines Übungsprogramms die Schwankungswerte für die Bewegungsmuster zu speichern und anhand der gespeicherten Schwankungswerte die Feedbackschwelle für eine spätere zweite Durchführung eines Übungsprogramms zu berechnen.

Die Vorrichtung zur Durchführung eines adaptiven Gleichgewichtstrainings kann im Aufbau beispielsweise durch eine Installation der Komponenten, wie z. B. Prozessoreinheit, Sensor, Aufforderungsmodul und/oder Signalgeber in einem gemeinsamen Gehäuse realisiert werden. Bevorzugt ist, dass das erfindungsgemäße Gerät außen am Körper des Trägers getragen wird, insbesondere nahe des Körperschwerpunktes. Z. B. kann eine Befestigung, insbesondere durch einen Hüftgürtel oder ein Klebepad direkt am Körper oder der Kleidung des Trägers verwendet werden. Das Gerät ist hierzu vorteilhafterweise durch eine ausreichend kompakte Bauweise und/oder ein relativ geringes Gewicht gekennzeichnet, so dass der Träger im Wesentlichen nicht durch die Vorrichtung bei der Ausführung des Übungsprogrammes behindert wird.

Eine Prozessoreinheit ist vorzugsweise eine Einheit zur Datenverarbeitung.

Die Prozessoreinheit umfasst bevorzugt einen Prozessor und/oder einen elektronischen (Daten)Speicher, welche insbesondere durch Datenübertragungsmittel, z. B. in Form von Signalleitungen für elektrische Signale, miteinander verbunden sein können.

Der Begriff Prozessor bezeichnet bevorzugt einen logischen Schaltkreis, welcher Daten bzw. elektrische Signale senden, empfangen und verarbeiten kann. Bevorzugte Prozessoren umfassen ohne Beschränkung eine integrierte Schaltung (IC), ein Anwendungsspezifische integrierte Schaltungen (ASIC), ein Field Programmable Gate Array (FPGA), einen Mikroprozessor, einen Mikrocomputer, eine speicherprogrammierbare Steuerung und/oder eine sonstige elektronische, bevorzugt programmierbare, Schaltung.

Die Prozessoreinheit ist bevorzugt geeignet zum Einlesen, zur Verarbeitung und/oder zur Ausgabe elektronischer Signale, welche durch die Konfiguration der Prozessoreinheit bestimmt werden.

Der Ausdruck, die Prozessoreinheit ist konfiguriert zur Ausführung bestimmter Verfahrensschritte oder Berechnungen meint bevorzugt, dass auf der Prozessoreinheit, eine Software, Firmware bzw. ein Computerprogramm gespeichert vorliegt, welches Befehle umfasst, die Verfahrens- bzw. Berechnungsschritte ausführen.

Der Ausdruck Prozessor ist dazu konfiguriert, während einer ersten Durchführung eines Übungsprogrammes die Schwankungswerte für die Bewegungsmuster zu speichern und anhand der gespeicherten Schwankungswerte die Feedbackschwelle für eine spätere zweite Durchführung eines Feedbackprogramms festzulegen, meint somit bevorzugt, dass ein entsprechendes Computerprogramm (als Soft- oder Firmware) auf der Prozessoreinheit gespeichert vorliegt, und Befehle umfasst, um Schwankungswerte für die Bewegungsmuster während einer ersten Durchführung eines Übungsprogrammes zu speichern und anhand der gespeicherten Schwankungswerte die Feedbackschwelle für eine spätere zweite Durchführung eines Feedbackprogramms zu berechnen und festzulegen.

Der Fachmann erkennt, dass bevorzugte (Berechnungs)schritte oder Verfahrensschritte, welche hinsichtlich der Funktionsweise der erfindungsgemäßen Vorrichtung offenbart werden, bevorzugt als computerimplementiertes Verfahren auf der Prozessoreinheit gespeichert vorliegen und von dieser vorgenommen bzw. ausgeführt werden können.

Der mindestens eine Sensor zur Messung der Veränderung einer Körperposition und/oder eines Bewegungsmusters des Trägers der Vorrichtung kann insbesondere Gyrometer und/oder Beschleunigungssensoren umfassen. Die Messung wird bevorzugt durch einen Transfer der gemessenen physikalischen Größe(n) in geeignete und/oder standardisierte elektronische Signale realisiert, welche die gemessenen Größen in geeigneter Weise repräsentieren und bevorzugt durch die Prozessoreinheit eingelesen und/oder verarbeitet werden können. Der Sensor kann bevorzugt durch geeignete Datenübertragungsmittel mit der Prozessoreinheit verbunden sein. Es kann sich auch bevorzugt um ein integriertes Bauteil handeln, welches die Prozessoreinheit und den mindestens einen Sensor umfasst.

Bei der Durchführung eines adaptiven Gleichgewichtstrainings misst der Sensor die Veränderung einer Körperposition und/oder eines Bewegungsmusters und überträgt die Messdaten an die Prozesseinheit. Die Messung kann beispielsweise Geschwindigkeitsdaten und/oder Beschleunigungsdaten in drei Dimensionen umfassen, aus welchen dann eine Körperposition, bevorzugt relativ zu einer Körperposition zu Anfang eines Messprozesses, bzw. ein Bewegungsmuster bestimmt werden kann. Bevorzugt wird der Sensor dafür im Wesentlichen ortsfest am Körper getragen und misst somit Veränderungen der Position des Körpers bzw. Bewegungsmuster des Körpers direkt über eine Veränderung der eigenen Position bzw. durch eigene Relativbewegungen im Hinblick auf ein initiales Koordinatensystem.

Die Prozessoreinheit ist konfiguriert, aus den Messdaten Schwankungswerte zu bestimmen. Schwankungswerte umfassen vorzugsweise eine Schwankungsauslenkung, Schwankungsbeschleunigung oder eine Schwankungsgeschwindigkeit. Sie können insbesondere eine Winkelgeschwindigkeit in Bezug auf Vorwärts-, Rückwärts- und Seitwärtsbewegungen des Trägers und für jede der Vorwärts-, Rückwärts- und Seitwärtsbewegungen umfassen. Eine Schwankung ist somit insbesondere jede Abweichung von der Ruheposition eines Körperbereiches insbesondere des Körperschwerpunktes. Zur Festlegung einer idealen Position mit normalen Schwankungen kann bspw. die Bewegung bzw. Position einer Vielzahl gesunder Probanden gemittelt werden.

Bevorzugt können Schwankungen lineare bzw. translative Bewegungen umfassen. Diese können beispielsweise durch ein Accelerometer (Beschleunigungsmesser) gemessen werden und können Geschwindigkeiten und/oder Beschleunigungen umfassen.

Es kann ebenso bevorzugt sein, dass eine Schwankung durch eine Rotationsbewegung um mindestens eine von drei aufeinander senkrecht stehenden Achsen, vorzugsweise den Drehachsen, beschrieben wird, welche bevorzugt durch den Körperschwerpunkt des Trägers laufen. Insbesondere können diese Achsen Longitudinalachse, Transversalachse und/oder Sagittalachse des Körpers des Trägers umfassen. Somit ist eine Schwankung bevorzugt beschrieben durch mindestens eine Winkelgeschwindigkeit und oder eine Veränderung mindestens einer Winkelgeschwindigkeit (bzw. Winkelbeschleunigung) um eine (Dreh-) Achse. Solche Rotationsbewegungen können z. B. durch Gyrometer gemessen werden.

Zur Bestimmung der Schwankungswerte aus den Messdaten werden die Messdaten der Prozessoreinheit zugeführt, bevorzugt über einen Signaleingang der Prozessoreinheit. Der Prozessor ist so eingerichtet, dass er aus diesen Messdaten die Schwankungswerte bestimmen kann. Dies kann bspw. durch eine richtige Interpretation der Messdaten und/oder Zuordnung der Messdaten zu Schwankungswerten erfolgen, z. B. anhand einer direkten Auswertung der Messwerte, anhand von gespeicherten Zuordnungstabelle oder anhand einer Berechnung mithilfe festgelegter Regeln durch die Prozessoreinheit, wobei die festgelegten Regeln bevorzugt durch physikalische Gesetze, insbesondere durch die Gesetze der Kinematik und/oder durch heuristische Regeln bestimmt werden. Es kann bevorzugt sein, dass für alle Übungen ideale Bewegungsmuster gespeichert vorliegen und/oder berechnet werden können und die Prozessoreinheit durch einen Vergleich der gemessenen Bewegungsmuster mit den idealen Bewegungsmustern aus den Abweichungen Schwankungswerte berechnet werden. Insbesondere sind die Schwankungswerte die gemessenen linearen und/oder rotatorischen Bewegungen.

Es kann vorzugsweise der Median 50 der gemessenen Schwankungswerte pro Zeiteinheit und/oder pro Schwankungsrichtung (z. B. links, rechts, vorwärts, rückwärts bzw. seitlich, vor/zurück) berechnet werden, wobei dann der Median 50 den berechneten Schwankungswert bildet. Eine Zeiteinheit kann beispielsweise durch die Länge einer (bevorzugt von der Vorrichtung erkannten oder typischen) Übungseinheit, eines Übungsprogramms oder eines Bewegungsmusters vorgegeben werden. Ebenso kann eine Zeiteinheit eine vorgegebene zeitliche Dauer umfassen und z. B. Größenordnungen von 1 µs (Mikrosekunde), 10 µs, 100 µs, 1 ms (Millisekunde), 10 ms, 100 ms, 1 s (Sekunde), 10 s oder 100 s umfassen.

Die Berechnung der Schwankungswerte kann z. B. eine Zeitreihe der gemessenen Schwankungswerte (bzw. der Median 50 der gemessenen Schwankungswerte) pro Richtung (links, rechts, vorne, hinten) oder Schwankungsachse (z. B. Achse 1: Schwankungen nach vorne oder hinten, Achse 2: Schwankungen nach links oder rechts) umfassen.

Das Aufforderungsmodul zur Ausgabe von Anweisungen für ein Übungsprogramm umfasst mehrere Bewegungsmuster des Trägers der Vorrichtung und kann bspw. eine optische Anzeige und/oder einen Lautsprecher enthalten. Das Aufforderungsmodul muss für die Ausgabe der Anweisungen bevorzugt Informationen verarbeiten und entsprechend den Informationen Anweisungen ausgeben. Dies kann insbesondere in Zusammenarbeit mit der Prozessoreinheit und/oder unter Berücksichtigung der Messdaten des mindestens einen Sensors geschehen. Die zu verarbeitenden Informationen und/oder ausgegebenen Anweisungen können z. B. folgende umfassen:
- Startzeitpunkt des Übungsprogramms;
- Handelt es sich um die erste oder eine spätere, bspw. zweite Durchführung des Übungsprogramms;
- Art und Anzahl der für das Übungsprogramm zu absolvierenden Übungen;
- Festgelegte oder individuell einstellbare Pausenzeiten zwischen zwei Übungen;
- Allgemeine und spezifische Anweisungen für die zu absolvierenden Übungen;
- Bewegungsmuster der Übungen des Übungsprogramms;
- Vom Träger absolvierte Bewegungsmuster;
- Bereits absolvierte und noch zu absolvierende Übungen für das Übungsprogramm;
- Dauer des Übungsprogramms;
- Endzeitpunkt und/oder noch zu absolvierende Dauer eines begonnenen Übungsprogrammes.

Das Aufforderungsmodul umfasst bevorzugt eine Informationsschnittstelle mit dem Träger, durch die Ausgabe der Anweisungen an diesen vermittelt werden können, z. B. einen Lautsprecher und/oder eine optische Anzeige. Das Aufforderungsmodul umfasst bevorzugt des Weiteren Mittel zur Verarbeitung bzw. zur Bestimmung der vorstehend genannten Informationen. Beispielsweise kann die Vorrichtung ein Mittel zur Eingabe des Startzeitpunkts, bevorzugt durch ein Eingabemodul, umfassen. Dabei kann es sich z. B. um einen Startknopf oder ein Mittel zu einer Eingabe der gewünschten Uhrzeit des Starts und/oder der Zeitdauer bis zum Start handeln. Diese Eingabe muss bevorzugt dazu führen, dass das Aufforderungsmodul zum gewünschten Startzeitpunkt die entsprechende Ausgabe vornimmt, z. B. "Beginnen Sie nun mit Übung 1". Damit diese Funktion erfüllt sein kann, kann bspw. die Eingabe des Startzeitpunkts einen Timer in Gang setzen, welcher zum Startzeitpunkt abläuft und ein elektronische Startsignal ausgibt, ferner müssen durch das Aufforderungsmodul bevorzugt Information abgerufen werden, z. B. aus einem elektronischen Speicher, wobei diese Informationen bspw. umfassen, welche Übungen in welcher Reihenfolge und in welcher Zeit absolviert werden sollen. Diese Informationen sollen dann vorzugsweise durch die Informationsschnittstelle an den Träger bereitgestellt werden. Dafür kann das Aufforderungsmodul einen elektronischen Prozessor umfassen, welche elektronische Signale je nach Konfiguration bzw. Programmierung einlesen, verarbeiten und/oder ausgeben kann. Dieser Prozessor kann durch die Prozessoreinheit umfasst sein. Je nach Grad der Automatisierung der Vorrichtung kann das Aufforderungsmodul bevorzugt automatisch nach Beendigung einer Übung den Beginn einer neuen Übung anweisen und/oder Informationen auswerten, welche Übungen mit welcher Qualität gerade absolviert werden. Dies kann insbesondere durch ein Zusammenwirken der Prozessoreinheit und dem mindestens einen Sensor erfolgen. Dabei können die Anweisungen auch ein Feedback zur Qualität der absolvierten Übung und/oder konkrete Anweisungen zur Verbesserung der Übung umfassen. Bevorzugt kann das angewiesene Übungsprogramm durch die Prozessoreinheit bestimmt werden, bspw. aufgrund der bei einem absolvierten initialen Übungsprogramm bestimmten Schwankungswerte bei verschiedenen Übungen.

Der Signalgeber zur Ausgabe eines Feedbacksignals umfasst beispielsweise einen Lautsprecher oder eine optische Anzeige.

Die Prozesseinheit ist konfiguriert, während eines Übungsprogrammes mittels des Signalgebers ein Feedbacksignal zu erzeugen, falls die Schwankungswerte eine Feedbackschwelle überschreiten. Dafür werden die von der Prozessoreinheit bestimmten Schwankungswerte bevorzugt mit Schwellwerten bzw. Schwellenwerten verglichen, welche beispielsweise gespeichert vorliegen. Diese Schwellwerte werden insbesondere als Feedbackschwelle bezeichnet. Beim Überschreiten der Schwellwerte bzw. der Feedbackschwelle wird mittels des Signalgebers ein Feedbacksignal erzeugt, welches den Träger darüber informieren soll, dass Schwankungswerte überschritten wurden. Dies hat insbesondere eine Trainingsfunktion. Zum einen wird der Träger durch ein Signal dazu gebracht, der Durchführung der Übung erhöhte Aufmerksamkeit zu widmen. Des Weiteren lernt der Träger die erwünschten maximalen Schwankungswerte kennen. So kann im Folgenden ein Lernprozess stattfinden, indem der Träger versucht, diese maximalen Schwankungswerte nicht zu überschreiten und ein Feedbacksignal zu vermeiden. Darüber hinaus kann das Feedbacksignal auch eine Warnfunktion erfüllen, indem es den Träger warnt, dass die Schwankungswerte ein Ausmaß angenommen haben, welches eine sichere Durchführung der Übung gefährdet und z. B. ein Sturz des Trägers unmittelbar bevorstehen könnte. Ein Überschreiten der Feedbackschwelle bedeutet insbesondere, dass der Schwankungswert innerhalb der Auflösungsgenauigkeit der Schwankungswerte und der Feedbackschwelle größer ist als die Feedbackschwelle. Beispielsweise können Schwankungswerte und Feedbackschwellen elektronisch dargestellte Zahlenwerte oder Signalstärken umfassen, die (bzw. deren Beträge) miteinander verglichen werden.

Aufforderungsmodul und/oder Signalgeber sind bevorzugt im funktionellen Sinne zu verstehen, d. h. es werden hierdurch Mittel bereitgestellt, die geeignet sind, die spezifischen jeweiligen Funktionen zu erfüllen. Aufforderungsmodul und Signalgeber können bspw. dieselbe optische Anzeige umfassen, welche die Informationsschnittstellte dieser Mittel mit dem Träger darstellen kann und/oder über einen gemeinsamen Prozessor, z. B. durch die Prozessoreinheit gesteuert werden.

Die Prozessoreinheit ist dazu konfiguriert, während einer ersten Durchführung eines Übungsprogrammes die Schwankungswerte für die Bewegungsmuster zu speichern und anhand der gespeicherten Schwankungswerte die Feedbackschwelle für eine spätere zweite Durchführung eines Feedbackprogramms festzulegen. Bevorzugt wird dabei der Median 50 der Schwankungswerte (s. o.) gespeichert, welcher entweder bereits bestimmt wurde (s. o.) oder vor dem Abspeicherungsprozess wie vorstehend beschrieben aus den zuvor berechneten bzw. gemessenen Schwankungswerten bestimmt wird. Durch eine solche Anpassung der Feedbackschwellen können bspw. Trainingseffekte verbessert werden, da Anforderungen vorteilhafterweise individuell an den Träger angepasst werden können und/oder angehoben werden können, so dass ein Lernprozess hierdurch unterstützt wird. Die Feedbackschwelle gibt bevorzugt die Anforderungen an den Träger bei der Durchführung des Übungsprogramms wieder, da der Träger vorteilhafterweise vermeiden möchte, dass die Schwankungswerte die Feedbackschwelle überschreiten und ein Feedbacksignal erzeugt wird. Jedoch ist es sinnvoll, die hierdurch gegebenen Anforderungen den individuellen Fähigkeiten des Trägers anzupassen. Sind diese z. B. dergestalt, dass bei einer Übung die Schwankungswerte sehr oft überschritten werden, dann können sie nicht mehr als Orientierung dienen, welche Ausführung wünschenswert ist und welche nicht. Ein Lernen findet vorteilhafterweise dann statt, wenn der Träger sowohl ein Verständnis dafür entwickelt, welche Bewegungen richtig sind und welche falsch. Wird dem Träger jedoch z. B. bei jeder Bewegung durch ein Feedbacksignal vermittelt, dass diese nicht richtig ausgeführt wird, kann ein solches Verständnis nicht entstehen. Darüber hinaus würde der Träger hierdurch frustriert. Es hat sich insbesondere gezeigt, dass für einen Lernerfolg Frustration in hohem Maße schädlich ist und dazu führen kann, dass der Träger aufgibt und die Durchführung des Übungsprogramms komplett einstellt. Vorteilhafterweise wird für einen besonders verbesserten Lernerfolg ein Verhältnis von der Vermittlung eines Lernerfolgs, welches insbesondere das Belohnungszentrum des Gehirns stimuliert und z. B. durch Ausbleiben eines Feedbacksignals erfolgt sowie eines negativen Feedbacks, z. B. durch das Feedbacksignal angestrebt, bei dem das negative Feedback nicht überwiegt. Daher ist es vorteilhaft, wenn der Träger nur "ab und an" ein Feedbacksignal erhält, so dass er ein entsprechendes Verständnis entwickeln kann. Dies kann jedoch unabhängig von den Fähigkeiten des Trägers nur erreicht werden, wenn eine Anpassung des Feedbacksignals an dessen Fähigkeiten erfolgt.

Für die Berechnung der Feedbackschwelle müssen von der Prozessoreinheit vorzugsweise Informationen berücksichtigt werden, welche z. B. die Antworten auf folgende Fragen umfassen:
- Gehören Schwankungswerte gemessene/gespeicherte Schwankungswerte zu einer ersten oder zu einer zweiten, späteren Durchführung des Übungsprogrammes?
- Welche Übungen sind vom Übungsprogramm umfasst?
- Welche Bewegungsmuster umfassen die einzelnen Übungen?
- Welche der berechneten Schwankungswerte (s. o.) werden gespeichert? (Beispielsweise die komplette Zeitreihe der Schwankungswerte und/oder ggf. nur Maximalwerte der Schwankung pro Schwankungsbewegung in einer der genannten Richtungen bzw. auf einer der genannten Achsen und/oder pro Zeiteinheit?
- Welche der gespeicherten Schwankungswerte werden herangezogen und/oder wie wird daraus die Feedbackschwelle für eine spätere zweite Durchführung eines Übungsprogramms berechnet?

Beispielsweise können während einer Durchführung eines Übungsprogramms Schwankungswerte für eine zweite Durchführung berechnet werden, indem:
- ein Zählerelement die Anzahl der Durchführungen zählt und dadurch festgestellt wird, dass es sich um eine erste Durchführung handelt;
- durch eine Zeigerfunktion innerhalb eines Datenspeichers die berechneten Schwankungswerte einer Übung zugeordnet und in Form einer Zeitreihe entsprechend abgespeichert werden;
- vorzugsweise ein Abgleich eines gemessenen Bewegungsmusters mit einem abgespeicherten Bewegungsmuster erfolgt, um die Schwankungen spezifischen Bewegungen des Bewegungsmusters zuzuordnen;
- anhand eines abgespeicherten Algorithmus aus den Schwankungswerten Feedbackschwellen für eine spätere, zweite Durchführung berechnet werden. Dabei werden vorzugsweise für die spezifischen Bewegungen jeweils eigene Berechnungen durchgeführt.

Bei der späteren, zweiten Durchführung, welche z. B. durch das Zählerelement festgestellt wird, wird dann das Feedbacksignal erzeugt, falls die Schwankungswerte die berechnete Feedbackschwelle überschreiten.

Es kann bevorzugt sein, dass es für jede Übung eine feste Feedbackschwelle gibt, welche sich insbesondere aus einem oder mehreren gespeicherten Schwankungswert(en) pro Übung ergibt. In diesem Fall umfasst das Bewegungsmuster insbesondere die komplette Übung. Es kann jedoch auch bevorzugt sein, dass eine Übung, insbesondere eine komplexe Übung, in mehrere, separate Bewegungsmuster zerlegt wird, und für jedes dieser Bewegungsmuster mindestens ein Schwankungswert gespeichert und eine eigene Feedbackschwelle erstellt wird.

Bevorzugt wird für jede Übung eine Zeitreihe von Schwankungswerten erstellt und abgespeichert.

Die Begriffe "erste Durchführung" und "zweite Durchführung" eines Übungsprogrammes kennzeichnen insbesondere eine zeitliche Reihenfolge, dahingehend, dass während einer früheren Durchführung gespeicherte Schwankungswerte zur Berechnung der Feedbackschwelle für eine spätere Durchführung genutzt werden können. Bei der ersten Durchführung muss es sich mithin nicht um eine allererste Durchführung handeln. Zwischen einer ersten und zweiten Durchführung können auch weitere Durchführungen liegen, bevorzugt schließt sich die zweite Durchführung jedoch unmittelbar an eine erste Durchführung an, d.h. die Berechnung der Feedbackschwelle für eine zweite Durchführung basiert bevorzugt auf einer zuletzt vorherigen Durchführung.

Die Speicherung der Schwankungswerte erfolgt bevorzugt "während einer ersten Durchführung" des Übungsprogrammes. Eine Berechnung kann bevorzugt auch während der ersten Durchführung stattfinden. Dies ist jedoch vorzugsweise nicht eng im Sinne einer absoluten Gleichzeitigkeit zu verstehen. Vielmehr ist damit bevorzugt gemeint, dass innerhalb der Zeit, die die Vorrichtung für die Vornahme der erforderlichen Schritte typischerweise benötigt, eine Berechnung stattfindet. Diese Schritte umfassen insbesondere Übertragung der Messdaten an die Prozessoreinheit durch den Sensor, Bestimmung der Schwankungswerte aus den Messdaten durch die Prozessoreinheit, Speicherung der Schwankungswerte für die Bewegungsmuster und Berechnung der Feedbackschwelle anhand der gespeicherten Schwankungswerte durch die Prozessoreinheit. Die Berechnung kann somit auch nach der ersten Durchführung erfolgen, in jedem Fall sollte die Berechnung aber vor der zweiten Durchführung abgeschlossen sein, sodass die Feedbackschwellen bereitstehen.

Mit der beschriebenen Berechnung kann eine automatisierte Anpassung der Feedbackschwelle erreicht werden, welche einen verbesserten bzw. hohen Lernerfolg des Trägers bei der Durchführung der Übungen erzielt, ohne dass ein medizinisches bzw. vergleichbar geschultes Fachpersonal benötigt wird, welches den Träger bei der Durchführung des Übungsprogramms individuell betreut. Die Durchführung der Übungen kann bequem zu Hause durchgeführt werden. Es können Ressourcen gespart werden, welche vorher nötig waren, um sich an einem Ort zur Durchführung des Übungsprogramms unter fachlicher Aufsicht einzufinden. Dies sorgt für eine hohe Compliance bei der Durchführung des Übungsprogrammes, wodurch der Erfolg erheblich verbessert werden kann. Compliance bedeutet hier insbesondere, dass die Übungsprogramme mithilfe der Vorrichtung mit einer hohen Regelmäßigkeit, Motivation und/oder Zuverlässigkeit und einer geringen Abbruch- oder Unterbrechungsquote durchgeführt werden.

Es kann bevorzugt sein, ein Positiv-Feedbacksignal auszugeben, wenn die Schwankungswerte unterhalb der Feedbackschwelle bleiben. Dieses Positiv-Feedbacksignal kann sich beispielsweise dadurch vom Feedbacksignal unterscheiden, dass es auf den Träger angenehmer und oder unauffälliger wirkt. Als Beispiel kann das Positiv-Feedbacksignal eine optische Anzeige in einem dauerhaften Grünton umfassen, während das Feedbacksignal eine Art Warncharakter aufweist und ein rotes Blinken der optischen Anzeige und/oder einen Signalton umfasst. Ein Positiv-Feedbacksignal kann durch ein gezieltes Ansprechen des Belohnungssystems den Trainingserfolg steigern.

Eine erste Durchführung kann eine vom jeweiligen Träger erstmalige Durchführung des Übungsprogrammes darstellen. In diesem besonderen Fall wäre diese auch als allererste Durchführung zu bezeichnen. Es kann sich bei der ersten Durchführung auch um eine spätere, z. B. zweite Durchführung handeln, bspw. nach einer langen zeitlichen Unterbrechung des Trainings. Bevorzugt kann eine Art Reset durchgeführt werden, so dass eine Durchführung nach dem Reset eine erste Durchführung ist. Ebenso kann es sich bei der ersten Durchführung, um eine beliebige Durchführung handeln, welche zeitlich vor einer zweiten (späteren) Durchführung erfolgt und so wie beschrieben für die Festlegung einer Feedbackschwelle genutzt werden kann. Eine Vorrichtung kann vorzugsweise für unterschiedliche Träger unterschiedliche Profile vorhalten, welche von dem jeweiligen Träger aufgerufen werden können. Somit kann eine Durchführung für einen Träger in seinem Profil die erste Durchführung sein, während ein anderer Träger seine erste Durchführung bereits absolviert hat und diese Tatsache sich in dessen Profil widerspiegelt.

Bevorzugt handelt es sich bei der Berechnung der Feedbackschwelle für eine spätere zweite Durchführung um einen iterativen Prozess, bei der bei beliebigen aufeinanderfolgenden Durchführungen die vorhergehende Durchführung als erste Durchführung und die darauffolgende Durchführung als zweite Durchführung gilt, so dass die Feedbackschwelle (außer für die allererste Durchführung) jeweils aus der vorhergehenden Durchführung berechnet wird. So kann eine ständige Anpassung der Feedbackschwellen je nach bisherigem Trainingserfolg vorgenommen werden, durch die ein besonders verbesserter Trainingserfolg erzielt werden kann, der sogar synergistische Züge annehmen kann.

Die Feedbackschwelle für die erste Durchführung kann aufgrund vorher festgelegter, z. B. gespeicherter Schwellwerte eingestellt werden, insbesondere für eine allererste Durchführung.

Bevorzugt umfasst das Übungsprogramm 1 - 25 Übungen, besonders bevorzugt 10 - 20 Übungen und insbesondere 1 - 6 Übungen.

Überraschenderweise kann durch die automatisierte Anpassung der Feedbackschwellen wie beschrieben ein gleichwertiger bzw. verbesserter Trainingserfolg erzielt werden, wie bei einer Durchführung des Übungsprogramms unter Aufsicht von Fachpersonal. Daher können die Übungen nun bequem zuhause absolviert werden. Das Gerät stellt somit vorteilhafterweise eine Art bislang unbekannten Hometrainer für den Gleichgewichtssinn dar. Wenn darüber hinaus bevorzugt Daten an ein medizinisches Fachpersonal übermittelt werden können, stellt die Vorrichtung vorzugsweise eine bislang unbekannte Vorrichtung zur Durchführung von Telemedizin für den Gleichgewichtssinn dar.

Die Vorrichtung zeichnet sich dadurch aus, dass die Prozessoreinheit dazu konfiguriert ist während einer ersten Durchführung eines Übungsprogrammes die Schwankungswerte für die Bewegungsmuster zu speichern und anhand der gespeicherten Schwankungswerte die Feedbackschwelle für eine spätere zweite Durchführung eines Übungsprogramms zu berechnen. Hierdurch kann eine adaptive Anpassung der Feedbackschwellenwerte erfolgen, welche zu einem überraschend guten Therapieerfolg führt. Ein aufwendiges Nachjustieren durch medizinisches Personal ist nicht mehr notwendig. Stattdessen kann das mittels der Vorrichtung ermöglichte adaptive Gleichgewichtstraining beispielsweise auch selbstständig zu Hause durchgeführt werden. Die Regelung gewährleistet insbesondere, dass stets optimale, individualisierte Feedbackschwellen für die jeweiligen Übungen und Bewegungen eingestellt werden. Darüber hinaus kann mittels der Vorrichtung bevorzugt automatisiert eine Zusammenstellung eines individualisierten Übungsverlaufs erfolgen, wodurch sich der Therapieerfolg nochmals steigern lässt.

In einer bevorzugten Ausführungsform der Erfindung wird die Veränderung einer Körperposition und/oder eines Bewegungsmusters des Trägers der Vorrichtung dreidimensional im Raum als Winkelgeschwindigkeit und/oder der Veränderung der Winkelgeschwindigkeit und/oder der Beschleunigung von Vorwärts-, Rückwärts- und Seitwärtsbewegungen des Träger, bevorzugt nahe am Körperschwerpunkt des Trägers, bestimmt und umfassen die Schwankungswerte eine Schwankungsauslenkung oder eine Schwankungsgeschwindigkeit.

Eine Veränderung einer Körperposition und/oder des Bewegungsmusters des Trägers der Vorrichtung kann bevorzugt durch eine Kombination von Translationsbewegungen und Rotationsbewegungen beschrieben werden, z. B. durch die (lineare) Geschwindigkeit und/oder Beschleunigung des Körperschwerpunktes in drei Raumdimensionen und die Winkelgeschwindigkeit und/oder die Veränderung der Winkelgeschwindigkeit des Körperschwerpunktes in drei Raumdimensionen. Die Beschleunigung von Vorwärts-, Rückwärts- und Seitwärtsbewegungen meint bevorzugt die lineare Beschleunigung in drei Raumdimensionen im Sinne einer Translationsbewegung. Diese kann vorteilhafterweise durch senkrecht aufeinander stehende Achsen, z. B. lokal ortsfester Achsen und/oder körperfeste Achsen des Körpers beschrieben werden. Die Körperachsen verlaufen vorzugsweise durch den Körperschwerpunkt und werden insbesondere durch Longitudinalachse, Transversalachse und Sagittalachse aufgespannt. Anhand der vorgenannten Achsen lässt sich bevorzugt eine Vorwärts-, Rückwärts- und/oder Seitwärtsbewegung (Translationsbewegung) ebenso wie eine Dreh- bzw. Rotationsbewegung um diese Achsen, z. B. in Form von Roll-, Gier- und/oder Nickbewegung beschrieben werden. Diese Achsen sind bevorzugt geeignet, Winkelgeschwindigkeiten und deren Veränderungen zu beschreiben, indem bevorzugt eine Körperdrehung anhand von Drehungen um diese Achsen beschrieben wird. Dabei kann es bevorzugt sein, die Drehungen anhand Eulerscher Winkel darzustellen, z. B. in der Konvention der eigentlichen Eulerwinkel oder der Kardanwinkel bzw. Tait-Bryan-Winkel Konvention. Die Winkelgeschwindigkeit ist bevorzugt eine Größe, welche angibt, wie schnell sich ein Winkel mit der Zeit um eine Achse ändert. Insbesondere ist die Winkelgeschwindigkeit eine vektorielle Größe, wobei die Vektororientierung bevorzugt durch die Drehachse vorgegeben ist, wobei die Vektorrichtung vorzugsweise durch die rechte Daumenregel bzw. die rechte Handregel beschrieben wird.

Schwankungen können bevorzugt Translationen oder Rotationen sein. Translationen werden bevorzugt durch Accelerometer gemessen und können Beschleunigungen und/oder Geschwindigkeiten umfassen. Rotationen werden bevorzugt durch Gyrometer gemessen und können bevorzugt Winkelgeschwindigkeiten und/oder -beschleunigungen umfassen.

Somit können Schwankungen vorteilhafterweise durch Bestimmung der Winkelgeschwindigkeit bzw. deren Veränderung gemessen und/oder berechnet werden bzw. sind durch diese direkt vorgegeben, so dass eine Messung der Winkelgeschwindigkeit und/oder deren Veränderung direkt die Schwankung ergibt.

Die Winkelgeschwindigkeit wird vorzugsweise in den Einheiten Radiant pro Sekunde (rad/s) oder Grad pro Sekunde (°/s) ausgedrückt.

Die Schwankungswerte umfassen dabei bevorzugt eine Schwankungsauslenkung und/oder eine Schwankungsgeschwindigkeit. Die Schwankungsauslenkung umfasst dabei insbesondere einen Auslenkungswinkel im Sinne eines oben beschriebenen Drehwinkels um mindestens eine Achse. Die Schwankungsgeschwindigkeit umfasst dabei bevorzugt die Winkelgeschwindigkeit. Die Schwankungsauslenkung zu einem Zeitpunkt kann vorzugsweise durch eine anfängliche Schwankungsauslenkung und das zeitliche Integral von einem Anfangszeitpunkt bis zum gewünschten Zeitpunkt der Schwankungsgeschwindigkeit bestimmt werden. Die Schwankungsgeschwindigkeit zu einem Zeitpunkt kann vorzugsweise durch eine anfängliche Schwankungsgeschwindigkeit und das zeitliche Integral von einem Anfangszeitpunkt bis zum gewünschten Zeitpunkt der Veränderung der Schwankungsgeschwindigkeit bestimmt werden.

Durch diese Ausführungsform lässt sich eine Veränderung einer Körperposition, eines Bewegungsmusters, einer Schwankung und/oder einer Schwankungsauslenkung besonders präzise und gleichzeitig effizient beschreiben. So kann auch Rechenleistung der Prozessoreinheit gespart werden, insbesondere, wenn gleiche Achsen für die Vorwärts-, Rückwärts- und Seitwärtsbewegungen und die Veränderung der Winkelgeschwindigkeit verwendet werden.

In einer weiteren bevorzugten Ausführungsform der Erfindung entsprechen die Schwankungswerte einer Winkelgeschwindigkeit in Bezug auf Vorwärts-, Rückwärts- und Seitwärtsbewegungen des Trägers und für jede der Vorwärts-, Rückwärts- und Seitwärtsbewegungen wird eine Feedbackschwelle berechnet. Bei dieser Ausführungsform werden bevorzugt alle Bewegungen, welche zusätzlich zu einer translatorischen Bewegung in die genannten Richtungen gemessen werden, also insbesondere rotatorische Bewegungen, als Winkelgeschwindigkeit gemessen und einem Schwankungswert zugeordnet, vorzugsweise ohne weitere Nachbearbeitung. Vorzugsweise entsprechen also den Schwankungswerten Winkelgeschwindigkeiten, welche in Bezug auf Vorwärts-, Rückwärts- und Seitwärtsbewegungen gemessen werden.

Die Vorwärts-, Rückwärts und Seitwärtsbewegungen entsprechen vorzugsweise den Bewegungsmustern der Übungen des Übungsprogramms. Dabei können "Bewegungen" vorzugsweise in einem sehr allgemeinen Sinne als beschreibend für die zu absolvierenden Bewegungsmuster verstanden werden.

In diesem Sinne kann beispielsweise auch eine Ruheposition über einen gewissen Zeitraum als "Bewegung" in diesem Sinne verstanden werden. Vorzugsweise können anhand der Messung von Vorwärts-, Rückwärts- und Seitwärtsbewegungen Bewegungsmuster einer Übung identifiziert werden, bspw. durch einen Abgleich mit gespeicherten Bewegungsmustern durch die Prozessoreinheit und einer Berechnung von Wahrscheinlichkeiten, dass es sich bei dem gemessenen Bewegungsmuster um eine der gespeicherten Bewegungsmuster handelt.

Vorzugsweise können sodann den gemessenen Winkelgeschwindigkeiten bei den Bewegungsmustern Schwankungswerte zugeordnet werden und so anhand dieser Schwankungswerte spezifisch für die Bewegungsmuster (die Vorwärts- Rückwärts- und Seitwärtsbewegungen) Feedbackschwellen errechnet werden. So können sehr effizient für die individuellen Übungen und/oder Bewegungsmuster der Übungen und individuell für den Träger Feedbackschwellen bereitgestellt werden. Derart individuell für einzelne Übungen, Bewegungen und Träger abgestimmte Feedbackschwellen waren bislang auch durch sehr geschultes Fachpersonal nicht realisierbar. Hierdurch kann ein hoher Trainingserfolg mit minimalem Einsatz von Ressourcen erreicht werden.

In einer weiteren bevorzugten Ausführungsform der Erfindung liegen in der Prozessoreinheit maximale Normwerte von Schwankungswerten für jeweilige Bewegungsmuster gespeichert vor.

Maximale Normwerte werden im Sinne der Erfindung auch als Maximalwerte der Normwerte bezeichnet. Die Maximalwerte der Normwerte von Schwankungswerten (in Bezug auf eine Übung oder Bewegungsmuster) sind bevorzugt jene auftretenden höchsten Schwankungswerte nach links, rechts, vorn und hinten, welche innerhalb einer Altersgruppe gesunder Probanden des gleichen Geschlechts nach dem Ausschluss von Extremwerten auftreten. Hierbei kann beispielsweise ein maximaler Normwert von einem Probanden für eine Vorwärts/Rückwärts-Schwankung ermittelt werden, während ein weiterer maximaler Normwert für eine seitwärts Bewegung von einem anderen Probanden ermittelt wird. Insbesondere wird zur Bestimmung der Normwerte zunächst der Median 50 der Schwankungswerte für jeden Probanden errechnet und in einem weiteren Schritt werden dann aus diesen Werten (bevorzugt den Normwerten) die maximalen Werte bestimmt, welche die Maximalwerte der Normwerte darstellen.

Bevorzugt entsprechen die Normwerte dem Median 50 der Schwankungswerte der Probanden, welche vorzugsweise gesund sind.

Alle Probanden sind vorzugsweise gesund, sodass z. B. die maximalen Normwerte Schwankungswerte darstellen, mit denen ein beschwerde- und sturzfreier Alltag möglich ist. Gesund bezeichnet in diesem Zusammenhang, dass keine Abweichungen vom physiologischen Zustand vorliegen, die einen Einfluss auf die Kontrolle der Körperstabilität haben könnten. Dieser Zustand kann z. B. durch die Anwendung des Dizziness Handicap Inventory überprüft werden. Ein gesunder Proband im oben genannten Sinne würde dabei vorzugsweise einen Gesamtscore unter 7 erreichen (Kurre, A; van Gool, C J A W; Bastiaenen, C H G; Gloor-Juzi, T; Straumann, D; de Bruin, E D. 2009. Translation, cross-cultural adaptation and reliability of the German version of the dizziness handicap inventory. Otology & Neurotology, 30(3):359-367). Die alters- und geschlechtsspezifischen maximalen Normwerte für eine Übung werden somit bevorzugt an einer Gruppe von gesunden Probanden im Hinblick auf eine bestimmte Übung oder Bewegungsmuster ermittelt und approximieren jene maximalen Schwankungswerte, welche noch als gesund gelten können.

Bevorzugt werden hierfür Extremwerte aussortiert, weil sie derart stark abweichen, dass sie für die genannten Zwecke nicht brauchbar sind. Solche Extremwerte können sich beispielsweise durch individuelle Bewegungsabläufe ergeben, welche auch bei gesunden Probanden vorkommen können. Ein derartiger Extremwert, würde sich nicht als Approximation einer maximal (noch zulässigen) gesunden Schwankung eignen. Wie dem Fachmann bekannt können Extremwerte auf verschiedene Weise aussortiert werden.

Die maximalen Normwerte können z. B. wie obig beschrieben ermittelt und auf der Prozessoreinheit bzw. einem von dieser umfassten Speicher z. B. in elektronischer Form gespeichert vorliegen. Beispielhaft sei auf die Tabelle 1 verwiesen, welche Wertebereiche für maximale Normwerte angibt. Die maximalen Normwerte können beispielsweise für die Festlegung der Feedbackschwellen für eine (aller)erste Durchführung verwandt werden. In besonders bevorzugten Ausführungsformen wird die adaptive Berechnung der Feedbackschwellen für spätere Durchführungen anhand einer vorherigen ersten Durchführung durch eine Begrenzung anhand bekannter maximaler Normwerte optimiert.

In einer bevorzugten Ausführungsform der Erfindung ist die Prozessoreinheit konfiguriert während der ersten Durchführung des Übungsprogrammes die Feedbackschwelle anhand der maximalen Normwerte festzulegen. Beispielsweise kann die Feedbackschwelle direkt den maximalen Normwerten entsprechen oder aber den maximalen Normwerten multipliziert mit einem festgelegten oder variablen Faktor. Es können bevorzugterweise ebenso andere, komplexere Algorithmen zur Berechnung der Feedbackschwelle unter Verwendung der maximalen Normwerte verwendet werden. So können auch während einer ersten Durchführung des Übungsprogrammes, insbesondere während einer allerersten Durchführung, sinnvolle Feedbackschwellen bestimmt werden. Insbesondere geben die maximalen Normwerte für einen Großteil der Träger der Vorrichtung, welche unter Gleichgewichtsstörungen leiden, eine erste gute Feedbackschwelle zu Trainingszwecken ab.

In einer weiteren bevorzugten Ausführungsform der Erfindung ist die Prozessoreinheit konfiguriert vor einer zweiten Durchführung des Übungsprogrammes die Feedbackschwelle anhand der Schwankungswerte einer vorherigen ersten Übung zu berechnen, wobei die Feedbackschwelle bevorzugt einem Wert zwischen dem Median 40 aller Schwankungswerte und dem Median 80 aller Schwankungswertes, besonders bevorzugt dem Median 60 der Schwankungswerte entspricht.

Als Median 60 wird bevorzugt anhand der Schwankungswerte des ersten Durchgangs der Wert ermittelt, der die höchsten 40 % aller Werte einer Schwankungsrichtung und die geringsten 60 % aller Werte teilt. Dies entspricht bevorzugt dem "Median 60". Im Sinne der Erfindung bezeichnet der Median 60, bevorzugt einen Wert für den 60% der Schwankungswerte, insbesondere der Schwankungsgeschwindigkeit und/oder -auslenkung, unterhalb des Wertes liegen und 40% der Schwankungswerte oberhalb des Wertes liegen. Die Definition für einen Median 40, 50, 70, 80. 95 etc. ist analog. Dabei werden vorzugsweise die Schwankungen nicht bereits vorher durch die Prozessoreinheit gemittelt, sondern zunächst als Zeitreihe der gemessenen/berechneten Schwankungswerte gespeichert, wobei aus diesen später der entsprechende Median berechnet wird. Der Median kann dabei anhand der pro Zeiteinheit gespeicherten Schwankungswerte berechnet werden. Eine Zeiteinheit kann beispielsweise durch die Länge einer (bevorzugt von der Vorrichtung erkannten oder typischen) Länge einer Übung, eines Übungsprogramms oder eines Bewegungsmusters vorgegeben werden.

Im Sinne der Erfindung wird der mathematische "Median" somit als "Median 50" bezeichnet, da für diesen Wert 50% der Schwankungswerte ober- bzw. unterhalb des Median 50 liegen müssen. Für diese Ausführungsform wird insbesondere eine Zeitreihe von Schwankungswerten während der ersten Durchführung einer Übung des Übungsprogrammes aufgenommen. Diese können z. B. bezüglich ihrer Größe sortiert werden, so dass hinterher der Median entsprechend vorstehender Erläuterungen bestimmt werden kann.

Wenn die Anzahl der abgespeicherten Schwankungswerte keine exakte Aufteilung gemäß der genannten Regel zulässt, dann wird vorzugsweise der Schwankungswert herangezogen, z. B. als Median 60, welcher am ehesten von allen Werten eine ungefähre solche Aufteilung ermöglicht, z. B. in 60% der Werte unterhalb und 40 % oberhalb des Median 60. Bevorzugt können auch anhand der Schwankungswerte anhand von Extrapolation oder anhand von Näherungsverfahren eine im wesentlichen kontinuierliche Schwankungswertverteilung berechnet werden, welche eine Berechnung des jeweiligen Median ermöglicht.

Der Median kann insbesondere den Betrag des Median bezeichnen. Beim Betrag soll vorteilhafterweise nur die absolute Größe betrachtet werden, unabhängig von der Richtung oder einem je nach Richtung vergebenen Vorzeichen des Schwankungswertes. Insbesondere wird der Schwankungswert für jede Richtung, z. B. links, rechts, nach vorne und/oder nach hinten, einzeln aufgenommen bzw. separat abgespeichert, so dass keine verschiedenen Vorzeichen für entgegengesetzte Richtungen verwendet werden müssen, sondern eine absolute (positive) Größe bzw. der Betrag des Schwankungswertes.

Es war überraschend, dass anhand des Median 60 ein verbesserter Trainingserfolg erzielt werden konnte, welcher insbesondere für Durchführungen von Übungen zu Hause, ohne eine Betreuung durch Fachpersonal geeignet ist.

In einer weiteren bevorzugten Ausführungsform der Erfindung ist die Prozessoreinheit konfiguriert zu gewährleisten, dass die Feedbackschwelle stets zwischen 50% und 200% der maximalen Normwerte beträgt, indem die Feedbackschwelle auf einen Wert von 50% der maximalen Normwerte hochgesetzt wird, falls die zuvor berechnete Feedbackschwelle weniger als 50% der maximalen Normwerte betrug und die Feedbackschwelle auf einen Wert von 200% der maximalen Normwerte heruntergesetzt wird, falls die zuvor berechnete Feedbackschwelle mehr als 200% der maximalen Normwerte betrug.

Die vorteilhafte Verwendung des Median 60 zeigt bereits gute Ergebnisse.

In einer weiteren bevorzugten Ausführungsform der Erfindung ist die Prozessoreinheit konfiguriert zu gewährleisten, dass die Feedbackschwelle stets zwischen 50% und 200% der Maximalwerte der Normwerte (s. Tab. 1) beträgt.

Es wurde festgestellt, dass beste Ergebnisse erzielt werden können, wenn der als Feedbackschwelle berechnete Median 60 zwischen 50 und 200% des Maximalwertes der Normwerte der Schwankungsgeschwindigkeit oder -auslenkung der entsprechenden Alters- und Geschlechtsgruppe für die Bewegungen des Körpers in der betrachteten Richtung liegt.

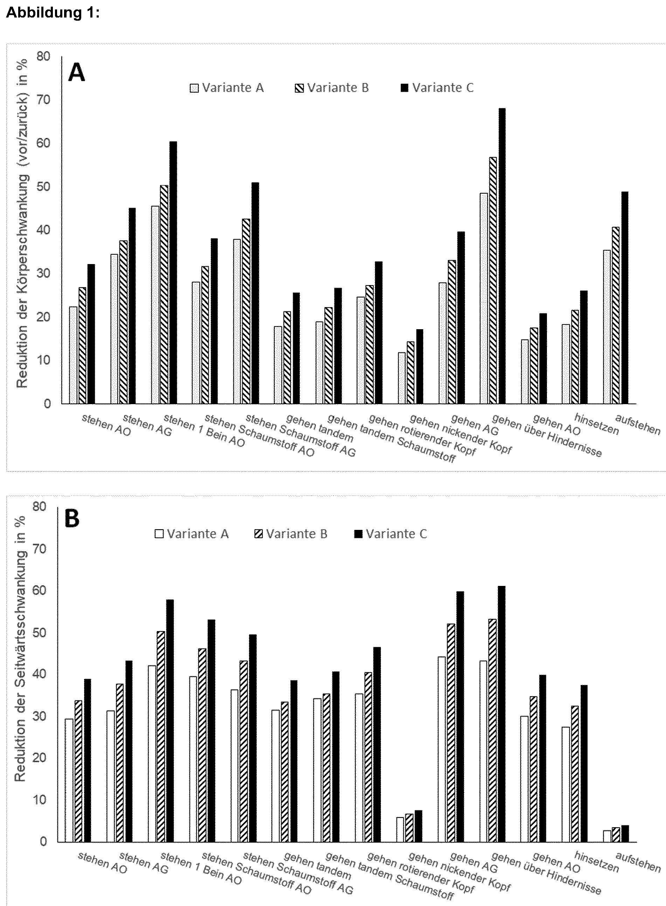

Wenn dem so ist, wird der Wert des Median 60 als Schwellenwert angenommen. Wenn der berechnete Wert größer ist als 200 % des Maximalwertes der Normwerte der Schwankungsgeschwindigkeit oder -auslenkung der entsprechenden Alters- und Geschlechtsgruppe für die Bewegungen des Körpers in der betrachteten Richtung, wird er auf diesen Wert heruntergesetzt und als Schwellenwert verwendet. Wenn der berechnete Wert kleiner ist als 50 % des Maximalwertes der Normwerte der Schwankungsgeschwindigkeit oder - auslenkung der entsprechenden Alters- und Geschlechtsgruppe für die Bewegungen des Körpers in der betrachteten Richtung, wird er auf diesen Wert heraufgesetzt und als Schwellenwert verwendet. Die maximalen Normwerte können entsprechend dem o. g. Vorgehen durch eine Messung der Normwerte einer Vielzahl gesunder Probanden ermittelt und auf dem Gerät abgespeichert werden. Wie in den Abbildung 1 gezeigt konnte hierdurch ein überraschend hoher Therapieerfolg verzeichnet werden. Die Festlegung der Feedbackschwellen mit dem bevorzugten Algorithmus stellt keine beliebige Auswahl, sondern vielmehr einen glücklichen Griff dar, welcher zu nicht erwartbaren Behandlungserfolgen führt. Die therapeutische Wirkung mittels der berechneten Feedbackschwellen ist hierbei sogar teilweise höher, als bei einer manuellen Festlegung der Feedbackschwellen durch ein geschultes Personal. Die automatisierte Festlegung der Feedbackschwellen erlaubt somit vorteilhaft eine kostensensitive telemedizinischen Betreuung, bei sogar gesteigerten Therapieergebnissen.

Die Berechnung erfolgt bevorzugt je Übung und Schwankungsrichtung an jedem Übungstag. Der Patient wird zudem bevorzugt mithilfe visueller oder akustischer Anweisungen, insbesondere durch das Ausgabemodul, durch das individuelle Übungsprogramm begleitet, wobei er durch Sprach-, Gesten-, Geräusch- oder Tasteingabe Zustimmung, Ablehnung oder Unterbrechung signalisieren kann.

In einer weiteren bevorzugten Ausführungsform der Erfindung ist der mindestens eine Signalgeber ausgesucht aus der Gruppe bestehend aus einem Lautsprecher, einer optischen Anzeige, einer Elektrode und einem Vibrationsstimulator.

Das Feedbacksignal wird dabei bevorzugt über den Lautsprecher, die optische Anzeige, der Elektrode und/oder den Vibrationsstimulator erzeugt und kann durch ein zeitiges Feedback besonders wirksam die Aufmerksamkeit des Trägers erhöhen. Ein Lautsprecher ist bevorzugt in die Vorrichtung integriert und erzeugt einen Signalton. Ein Signalton hat sich für eine besonders effektives Feedbacksignal als besonders geeignet erwiesen. Es hat sich gezeigt, dass Töne besonders gut von einem Träger aufgenommen werden und eine Gewöhnung an einen Signalton, welche eine verringerte Vigilanz zur Folge hätte, besonders selten ist. In einer besonders bevorzugten Variante wird die Lautstärke und/oder die Frequenz des akustischen Signales gesteigert je stärker die bestimmte Veränderung der Körperposition und/oder des Bewegungsmusters von der Feedbackschwelle abweicht.

Das Feedbacksignal kann (ebenso wie die Eingabeaufforderung) über eine Sprachausgabe erfolgen. Diese kann aus psychologischen Gründen als besonders angenehm und/oder wirksam empfunden werden und erhöht die Compliance.

Das Feedbacksignal kann (ebenso wie die Eingabeaufforderung) über ein Headset bzw. Hörgerät erfolgen. Gleichgewichtsstörungen gehen oft mit Störungen des Hörsinns einher. Daher kann es sinnvoll sein, das Feedbacksignal über ein Hörgerät auszugeben. Dabei kann eine Verbindung zwischen Hörgerät und Vorrichtung per Kabel und/oder drahtlos sein oder aber die Vorrichtung im Hörgerät und/oder Headset integriert sein.

Eine optische Anzeige ist bevorzugt eine Anzeigevorrichtung und/oder ein Display, auf der ein Feedbacksignal optisch, z. B. durch Darstellung eines Symbols (Warndreieck o. ä.) dargestellt werden kann. Dabei kann die Anzeige zusätzlich farblich gestaltet sein und/oder zur Warnung aufleuchten, bspw. in Signalrot. Die Anzeige ist dabei bevorzugt innerhalb des Blickfeldes des Trägers angebracht. Eine optische Anzeige kann auch projiziert werden, z. B. auf die Innenseite von Brillengläsern einer (Sonnen-) Brille. Optische Signale, welche durch eine Anzeige vermittelt werden, rufen eine besonders schnelle Reaktion hervor, wodurch das Feedback besonders effektiv ist.

Eine optische Anzeige kann ebenso bevorzugt eine Lichtquelle sein, bevorzugt eine Lichtquelle im Blickfeld des Trägers der Bestrahlungsvorrichtung, so dass bei Aktivität der Aktoren der Träger einen Lichtreiz wahrnimmt. Die Intensität des Lichtreizes oder aber die Farbe des Lichtreizes wird dabei in einer bevorzugten Variante, derart gewählt, dass diese eine höhere Signalwirkung aufweist, je stärker die bestimmte Veränderung der Körperposition und/oder des Bewegungsmusters von der Feedbackschwelle abweicht.

Ein Vibrationsstimulator löst bevorzugt eine mechanische Vibration aus, die auf einen Bereich der Körperoberfläche, bevorzugt auf die Haut eines Trägers übertragen werden kann. Der Stimulator kann dabei fest am Körper des Trägers befestigt sein, z. B. mit einem Band, einem Clip an der Hüfte oder einem Gürtel. Ebenso kann ein solcher Stimulator in eine Kleidung integriert sein. Ein Vibrationsstimulator löst auf besonders intuitive Weise eine Reaktion eines Trägers aus. Insbesondere können auch mehrere Stimulatoren verwendet werden, welche so an mehreren Körperstellen eines Trägers befestigt werden, dass durch einen taktilen Impuls und/oder eine Vibration eines geeigneten Stimulators von einem Träger direkt auf die jeweilige Schwankungsrichtung geschlossen werden kann, für die die Feedbackschwelle überschritten wurde. Z. B. können an der Hüfte vier Stimulatoren befestigt werden, welche gleichmäßig um die Hüfte verteilt werden. Dabei kann jeder vibrierende Stimulator einen Quadranten im Raum symbolisieren, in dessen Richtung eine akute Schwankung festgestellt wurde. Bspw. kann bei einer Schwankung in die vom Träger aus gesehene, linke vordere Richtung bevorzugt ein vorderer und ein seitlich links an der Hüfte befestigter Stimulator eine Vibration auslösen.

Bevorzugte Aktoren sind z.B. Unruhemotor 6CH-1201-WL-00, Namiko Corp., Tokyo. Die Drehzahl des Unruhemotors ist hierbei bevorzugt abhängig von der Frequenz der ausgegebenen Impulse. Die Zahl der Impulse kann dabei bevorzugt an die Größe der Schwankung angepasst, werden, welche sich z. B. durch Größe der Überschreitung der Stabilitätsgrenze bemessen kann. So kann eine besonders effektive und angepasste Warnung des Trägers vor Stürzen realisiert werden.

Weitere bevorzugte Aktoren sind galvanische Stimulatoren, insbesondere durch Elektroden, wobei die Stimulatoren durch elektrische Reizung auf der Körperoberfläche, durch elektrische Reizung von motorischen Nerven bzw. der Muskulatur und/oder durch elektrische Reizung von sensorischen Nerven bzw. Sinnesorganen oder Teilen dieser ausgeführt wird. Diese können besonders effektiv eine Reaktion bzw. erhöhte Aufmerksamkeit des Trägers auslösen.

In einer weiteren bevorzugten Ausführungsform der Erfindung ist das Feedbacksignal ausgewählt aus der Gruppe bestehend aus einem visuellem, einem taktilem, einem vibrotaktilem, einem galvanischem und einem gustatorischen Reiz. Diese Reize sind besonders effektiv, um die Aufmerksamkeit des Trägers zu gewinnen und den Trainingserfolg zu erhöhen. Es kann auch je nachdem, ob ein Träger unter bestimmten Sinnesstörungen leidet, eine gezielte Reizung des für den jeweiligen Träger am besten geeigneten Sinns erzielt werden und so die Vorrichtung besonders individualisiert werden. Gustatorische Reize können durch chemische und/oder elektrische Stimulation der Geschmacks- und/oder Geruchsnerven erzielt werden.

In einer weiteren bevorzugten Ausführungsform der Erfindung kann das Feedbacksignal in mehreren Intensitätsstufen ausgegeben werden, wobei bevorzugt eine einfache Überschreitung der Feedbackschwelle eine erste Stufe auslöst, eine 1,5fache Überschreitung der Feedbackschwelle eine zweite Stufe und eine zweifache Überschreitung der Feedbackschwelle eine dritte Stufe. Bevorzugt steigert sich die Intensität von der ersten zur dritten Stufe. Eine Steigerung kann linear zur Überschreitung oder auch exponentiell hierzu erfolgen. Besonders eine exponentielle Steigerung hat sich als effektiv erwiesen. So kann das Feedback angepasst werden, je nachdem wie stark eine Schwankung ausfällt. Es hat sich gezeigt, dass die Aufmerksamkeit des Trägers so am besten angesprochen werden kann, da das Feedback gut dosiert werden kann und u. a. dadurch kein zu starker Gewöhnungseffekt an ein immer gleich starkes Feedbacksignal eintritt, welcher kontraproduktiv wäre. Auch kann dem Träger ein besseres Gefühl für seine Bewegungen und die Stärke seiner Schwankungen vermittelt werden.

In einer weiteren bevorzugten Ausführungsform der Erfindung erfolgt die Ausgabe der Anweisungen für ein Übungsprogramm visuell oder akustisch. Insbesondere umfasst das Ausgabemodul einen Lautsprecher und/oder eine optische Anzeige. Eine optische Anzeige kann auch ein von einem Touchscreen, welches zusätzliche Interaktionsmöglichkeiten für den Träger bietet, umfasst sein. Durch Erzeugung eines Tones oder einer verbalen Aufforderung über einen Lautsprecher kann ein Träger besonders effektiv bei der Ausführung der Übung angewiesen werden bei einer gleichzeitig möglichst geringen Ablenkung, bspw. durch optische Reize. Eine optische Anzeige wiederum bietet die Möglichkeit, bestimmte Bewegungsformen durch eine optische Darstellung besonders eindrücklich zu vermitteln. Insbesondere können beide Ausgabeformen kombiniert werden, um ihre jeweiligen Vorteile in idealer Weise zu vereinen.

In einer weiteren bevorzugten Ausführungsform der Erfindung umfasst die Vorrichtung ein Eingabemodul zur Vornahme von Eingaben durch den Träger der Vorrichtung, welche zur Auswertung an die Prozessoreinheit übermittelt werden. So kann eine Durchführung eines Übungsprogrammes durch eine erhöhte Compliance weiter gesteigert werden. Durch die Möglichkeit der Interaktion fühlt sich der Träger in seinen individuellen Bedürfnissen besser wahrgenommen, wodurch seine Motivation bei der Durchführung des Übungsprogrammes gesteigert wird. Ein Eingabemodul umfasst vorzugsweise mindestens einen Taster, einen Knopf, einen Schalter, eine Kamera, ein Touchscreen und/oder ein Mikrofon.

In einer weiteren bevorzugten Ausführungsform der Erfindung umfassen die Eingaben Spracheingaben, Gesteneingaben, Geräuscheingaben oder Tasteingaben. Diese Formen der Eingaben lassen sich besonders leicht vom Träger veranlassen, auch während einer Durchführung einer Übungseinheit.

In einer weiteren bevorzugten Ausführungsform der Erfindung kann ein Träger der Vorrichtung mittels der Eingaben auf die Anweisungen zur Durchführung eines Übungsprograms reagieren, in dem der Träger bevorzugt eine Zustimmung, Ablehnung oder Unterbrechung signalisiert, und die Prozessoreinheit dafür konfiguriert ist, den Verlauf der Durchführung des Übungsprogrammes anhand der Eingaben anzupassen. Dies steigert die Motivation bei der Durchführung des Übungsprogramms und verbessert den Trainingserfolg.

In einer weiteren bevorzugten Ausführungsform der Erfindung ist die Prozesseinheit dazu konfiguriert während der Durchführung eines ersten Übungsprogrammes die Schwankungswerte für die Bewegungsmuster zu speichern und anhand der gespeicherte Schwankungswerte Übungen für ein späteres zweites Übungsprogramm auszuwählen. Insbesondere können dabei Übungen ausgewählt werden, bei welchen die Schwankungswerte besonders hoch sind und welche ein gesteigertes Übungsvolumen bzw. eine gesteigerte Übungsintensität erfordern. So wird das Training effizienter, es kann Zeit eingespart werden, indem nur individuell angepasst die für den Träger wesentlichen Übungen durchgeführt werden.

In einer bevorzugten Ausführungsform der Erfindung erfolgt die Auswahl der Übungen anhand eines Vergleichs der Schwankungswerte bei der Durchführung einer Batterie von Übungen mit Normwerten, wobei bevorzugt diejenigen Übungen ausgewählt werden, bei denen ein Median 50 der gemessenen Schwankungswerte höher ist als der Median 80 bis 98, insbesondere Median 95 der Normwerte für eine jeweilige Alters- oder Geschlechtsgruppe. Bevorzugt umfasst das erste oder auch das initiale Übungsprogramm eine Batterie bzw. eine Vielzahl Übungen beispielsweise 3, 5, 6, 7, 8, 9, 10 oder mehr von denen gemäß obig beschriebenen Kriterien diejenigen Übungen ausgewählt werden, bei denen die Schwankungswerte signifikant höher sind. Es hat sich gezeigt, dass hierdurch die Effizienz des Trainings enorm gesteigert werden kann.

Normwerte sind vorzugsweise Werte, die bei einer Durchführung der Bewegungsmuster der Übungen des Übungsprogramms durch eine statistisch relevante Zahl gesunder Probanden ermittelt bzw. gemessen werden. Für die Erstellung der Normwerte wird insbesondere der Median 50 der Schwankungswerte der Probanden im Hinblick auf ein bestimmtes Bewegungsmuster verwendet.

Von diesen Werten wird insbesondere der Median 95 aller vorliegenden Normwerte der Alters-, Geschlechts- und Übungsgruppe je Schwankungsrichtung (vgl. Tabelle 2), um wie beschrieben die Übungsauswahl zu treffen. Im Gegensatz zu den maximalen Normwerten, bezeichnet im Sinne der Erfindung die Normwerte der Schwankungswerte bevorzugt somit die statistisch am häufigsten auftretenden Schwankungswerte. Es werden somit vorteilhafterweise nicht maximale Schwankungswerte approximiert, die noch als gesund gelten können, sondern ein Abbild der tatsächlichen wahrscheinlichen Schwankungswerte innerhalb einer gesunden Population. Es wurde erkannt, dass für die Auswahl der Übungen der Median 95 der Normwerte (und nicht die maximalen Normwerte) zu besonders guten Ergebnissen führt.

Vorteilhaft kann das Übungsprogramm hier durch effektiv auf jene Übungen reduziert bzw. konzentriert werden, bei denen sich ein besonders hoher Behandlungserfolg einstellt. Die Auswahl kann, muss sich aber nicht mit jenen Übungen decken für die subjektiv ein erhöhter Bedarf besteht. Vielmehr kann überraschenderweise durch Auswahl eines geeigneten Sets an Übungen nach obigen Kriterien auch für andere Bewegungsmuster eine Verbesserung festgestellt werden.

In einer bevorzugten Ausführungsform werden anhand eines Vergleichs der Schwankungswerte bei der Durchführung einer Batterie von Übungen mit Normwerten für eine jeweilige Alters- oder Geschlechtsgruppe 1 bis 20, besondere bevorzugt 4 bis 8, am meisten bevorzugt 6 Übungen für ein Übungsprogramm ausgewählt.

In einer weiteren bevorzugten Ausführungsform der Erfindung liegen die Prozessoreinheit, der Sensor, das Aufforderungsmodul und der Signalgeber in einem gemeinsamen Gehäuse installiert vor und sind über elektrische Signalleitungen miteinander verbunden. So kann eine besonders kompakte und robuste Zusammenstellung der wesentlichen Komponenten der Vorrichtung realisiert werden.

In einer weiteren bevorzugten Ausführungsform der Erfindung umfasst die Prozessoreinheit einen Mikroprozessor und/oder einen elektronischen Speicher. Diese elektronischen Bauteile sind günstig, leistungsstark, robust und räumlich kompakt. Ein elektronischer Speicher kann Daten, z. B. zur Stabilitätsgrenze, besonders schnell aufrufen und verarbeiten bzw. abspeichern.

In einer weiteren bevorzugten Ausführungsform der Erfindung umfasst der mindestens eine Sensor zur Messung der Veränderung einer Körperposition und/oder eines Bewegungsmusters des Trägers der Vorrichtung Gyrometer und/oder Beschleunigungssensoren.

Diese haben sich als Sensor zur Messung der Veränderung einer Körperposition und/oder des Bewegungsmusters des Trägers als besonders sinnvoll erwiesen. Es kann bevorzugt sein, dass hier kommerziell erhältliche Gyrometer und/oder Beschleunigungssensoren verwendet werden, die kostengünstig sind und ihre Tauglichkeit bereits bei einer Vielzahl von Anwendungen unter Beweis gestellt haben, somit besonders robust und wartungsarm sind. Es kann diesbezüglich je nach Verwendung und Anforderungen auf verschiedene Technologien zurückgegriffen werden, die unterschiedliche Vorteile aufweisen. Es können als Gyrometer z. B. hochpräzise Sagnac-Interferometer verwendet werden. Aber auch der Einsatz günstiger und robuster MEMS-Technologie für Gyrometer und/oder Beschleunigungssensoren kann bevorzugt werden.

Dabei können standardisierte Komponenten, die ein standardisiertes Datenausgabeformat aufweisen, bevorzugt sein. Diese können besonders einfach und schnell in der Herstellung mit einer Regelungseinheit funktionell im Sinne der Erfindung verknüpft werden.

Auch integrierte Komponenten, welche die geeignete Anzahl an Gyrometern und/oder Beschleunigungssensoren integriert in einer Schaltung aufweisen, können bevorzugt sein für eine besonders kompakte und schnelle Vorrichtung.

In einer weiteren bevorzugten Ausführungsform der Erfindung weist der mindestens eine Sensor zur Messung der Veränderung einer Körperposition und/oder eines Bewegungsmusters des Trägers mehrere orthogonal zueinanderstehende Gyrometer und/oder Beschleunigungssensoren auf, welche die Veränderung der Winkelgeschwindigkeit und/oder die Beschleunigung von Vorwärts-, Rückwärts- und Seitwärtsbewegungen des Körpers bestimmen.

Durch mehrere, orthogonal zueinanderstehende Gyrometer und/oder Beschleunigungssensoren können Veränderungen der Winkelgeschwindigkeit und/oder der Beschleunigung von Vorwärts-, Rückwärts- und Seitwärtsbewegungen des Körpers verbessert festgestellt werden. Bevorzugt bilden die orthogonal zueinanderstehenden Gyrometer und/oder Beschleunigungssensoren bezüglich ihrer Orientierung die entsprechenden Achsen nach, bevorzugt Longitudinalachse, Transversalachse und Sagittalachse. Dadurch ist die Messung besonders effizient und schnell, auch aufgrund eingesparter Rechenleistung.

In einer bevorzugten Ausführungsform wird die Veränderung der Winkelgeschwindigkeit von Vorwärts-, Rückwärts- und Seitwärtsbewegungen des Körperschwerpunktes in 3 Dimensionen dabei von einem 3-Achsen-Gyrometer-Chip bestimmt. Der 3-Achsen-Gyrometer-Chip umfasst dabei bevorzugt drei Gyrometer, welche orthogonal zueinander angeordnet sind. Ein besonders bevorzugter 3-Achsen-Gyrometer-Chip ist der L3G4200D von STMicroelectronics. Gyrometer sind dabei bevorzugt als Geräte zu verstehen, welche Drehbewegungen insbesondere Winkelgeschwindigkeiten der Drehbewegungen messen können. Geeignete Gyrometer sind dem Fachmann bekannt und können kommerziell erworben werden. Insbesondere sind im Stand der Technik Gyrometer bekannt, welche die Corioliskraft, als Trägheitskraft in rotierenden Bezugsystemen, bestimmen. Dabei werden in diesen Gyrometern abhängig von der Veränderung der Corioliskraft während einer Bewegung Kapazitätsveränderungen registriert. Da die Corioliskraft in einer festen Beziehung zur Winkelgeschwindigkeit der Rotation steht, können die gemessenen Kapazitätsveränderungen mithilfe eines Mikroprozessors in numerische Werte der Winkelgeschwindigkeit umgewandelt werden. Ebenso können 3-Achsen-Gyrometer-Chips kommerziell erworben werden. Durch die orthogonale Anordnung von drei Gyrometern in den 3-Achsen-Gyrometer-Chips, kann die Winkelgeschwindigkeit einer Drehung des Gerätes bevorzugt zur Messung der Veränderung einer Körperposition und/oder des Bewegungsmusters in 3 Dimensionen, d.h. entlang von drei Bezugsachsen, bestimmt werden.

In einem weiteren Aspekt betrifft die Offenlegung eine Verwendung der Vorrichtung zur Durchführung eines Übungsprogrammes.

Der durchschnittliche Fachmann erkennt, dass Vorteile, Merkmale, Definitionen sowie Ausführungsformen der erfindungsgemäßen Vorrichtung ebenso für die erfindungsgemäße Verwendung gelten.

In einem weiteren Aspekt betrifft die Offenlegung ein Verfahren, bevorzugt ein computerimplementiertes Verfahren, zur Durchführung eines adaptiven Gleichgewichtstrainings umfassend eine erste Durchführung eines Übungsprogrammes und eine zweite spätere Durchführung des Übungsprogrammes, aufweisend folgende Schritte:
- Ausgabe von Anweisungen für das Übungsprogramm umfassend mehrere Bewegungsmuster durch ein Aufforderungsmodul, bevorzugt durch eine optische Anzeige und/oder einen Lautsprecher;
- Messung der Veränderung einer Körperposition und/oder eines Bewegungsmusters des Trägers der Vorrichtung durch mindestens einen Sensor während der Durchführung des Übungsprogramms und Bestimmung der Schwankungswerte aus den Messdaten;
- Ausgabe eines Feedbacksignals durch einen Signalgeber falls die Schwankungswerte eine Feedbackschwelle überschreiten, bevorzugt durch mindestens einen Lautsprecher, eine optischen Anzeige, mindestens Elektrode, vorzugsweise mehrere Elektroden und/oder mindestens einen Vibrationsstimulator, bevorzugt mehrere Vibrationsstimulatoren;
wobei während der ersten Durchführung des Übungsprogrammes die Schwankungswerte für die Bewegungsmuster gespeichert werden und die Feedbackschwelle für die zweite Durchführung des Übungsprogrammes anhand der gespeicherten Schwankungswerte berechnet werden.

Die in diesem Dokument beschriebene Vorrichtung ist bevorzugt eine Vorrichtung mit mindestens einem Sensor, einem Aufforderungsmodul und einem Signalgeber, welche geeignet ist, die Schritte des vorgenannten Verfahrens auszuführen.

In einem weiteren Aspekt betrifft die Erfindung ein Kit zur Durchführung eines adaptiven Gleichgewichtstrainings wie durch Anspruch 14 ausgeführt.

In einem weiteren Aspekt betrifft die Erfindung ein Computerprogrammprodukt zur Installation auf einer Vorrichtung wie durch Anspruch 15 ausgeführt.

Der durchschnittliche Fachmann erkennt, dass Vorteile, Merkmale, Definitionen sowie Ausführungsformen der erfindungsgemäßen Vorrichtung ebenso für das erfindungsgemäße Verfahren, den Kit sowie das Computerprogrammprodukt gelten, und umgekehrt.

Bei der in dem Dokument beschriebene Vorrichtung handelt es sich bevorzugt um eine eigens zudem Zweck bereitgestellte Vorrichtung. Die Vorrichtung kann im Aufbau beispielsweise durch eine Installation der Komponenten, wie z. B. Prozessoreinheit, Sensor, Aufforderungsmodul und/oder Signalgeber in einem gemeinsamen Gehäuse realisiert werden. Bevorzugt ist ein kompakter Aufbau mit einem möglichst geringen Gewicht, wobei die Vorrichtung bevorzugt außen am Körper des Trägers getragen wird, insbesondere nahe des Körperschwerpunktes. Eine Befestigung der Vorrichtung kann insbesondere durch einen Hüftgürtel oder ein Klebepad direkt am Körper oder der Kleidung des Trägers vorgesehen.

Von den Erfindern wurde zudem erkannt, dass das erfindungsgemäße adaptive Gleichgewichtstraining insbesondere auch mit Mobilgeräten aus dem Stand der Technik durchgeführt, sofern diese mittels einer eigens dazu eingerichteter Software bzw. Computerprogrammprodukt (,app') zu diesem Zweck konfiguriert werden.

Unter einem Mobilgerät werden bevorzugt mobile Endgeräte verstanden, die aufgrund ihrer Größe und ihres Gewichts ohne größere körperliche Anstrengung tragbar und somit mobil einsetzbar sind. Bevorzugt handelt es sich um elektronische Endgeräte für mobile, netzunabhängige Daten-, Sprach- und Bildkommunikation, Navigation oder dergleichen. Besonders bevorzugte Mobilgeräte sind beispielsweise ein Smartphone, ein Tablet-Computer, und/oder eine Smartwatch sein.

Vorteilhaft umfassen derartige Mobilgeräte bereits einige Grundkomponenten, welche sich bei entsprechender Konfiguration mittels einer Software zur Ausführung des erfindungsgemäßen adaptiven Gleichgewichtstraining eignen. Demnach weisen Mobilgeräte standardmäßig eine Prozesseinheit auf sowie geeignete Aufforderungsmodule, beispielsweise einen Lautsprecher zur Ausgabe akustischer Anweisungen oder ein Display zur Ausgabe optischer Anweisungen oder Eingabemodule (u.a. Mikrofon mit Spracherkennung oder Touchdisplay). Für einen Signalgeber zur Ausgabe eines Feedbacksignals können in derartigen Mobilgeräte insbesondere ein Vibrationsmechanismus zur Ausgabe eines taktilen Feedbacksignals, ein Lautsprecher zur Ausgabe eines akustischen Feedbacksignals oder aber ein Display zur Ausgabe eines optischen Feedbacksignals genutzt werden.

Zudem sind in einer Vielzahl der Mobilgeräte ebenfalls standardmäßig ein Sensor zur Messung der Veränderung einer Körperposition und/oder eines Bewegungsmusters des Trägers der Vorrichtung. Smartphones weisen heutzutage beispielsweise Accelerometer und/oder Gyrometer, insbesondere auch 3-Achsen-Gyrometer, auf, welche die Lageveränderung des Gerätes fortwährend messen können. Bei geeigneter Anbringung der Mobilgeräte nahe am Körperschwerpunkt beispielsweise mittels eines Gurtes an der Hüfte, kann vorteilhaft eine Messung der Veränderung einer Körperposition und/oder eines Bewegungsmusters des Trägers mittels des Mobilgerätes durchgeführt werden.

Die Messdaten des Sensors über die Veränderung der Position und/oder Orientierung des Mobilgerätes werden vorzugsweise, vom Betriebssystem des Mobilgerätes bereitgestellt.

Ein Betriebssystem bezieht sich vorzugsweise auf die Software, die mit der Hardware des Geräts kommuniziert und es anderen Programmen, wie beispielsweise der Software (,app') ermöglicht, auf dem Gerät zu laufen.

Beispiele für Betriebssysteme umfassen Apples iOS für iPhone, iPad und iPod Touch, Windows oder Android für den Betrieb verschiedener Smartphones, Tablet-Computer oder Media-Player. Betriebssysteme steuern und überwachen die Hardware der Mobilgeräte, beispielsweise des Lautsprechers, Mikrofone, Displays, Sensoren oder Vibratoren. Vorzugsweise stellen Betriebssysteme Messdaten über die Position und/oder Orientierung des Mobilgerätes bereits, welche mittels des erfindungsgemäßem Computerprogrammprodukt ausgewertet werden können, um Schwankungswerte zu erhalten und daraus Feedbackgrenzen zu ermitteln und Feedbacksignale bei einer Überschreitung auszugeben.

Das Computerprogrammprodukt bzw. die Software (,app') kann in jeder Programmiersprache oder modellbasierten Entwicklungsumgebung geschrieben werden, wie beispielsweise C/C++, C#, Objective-C, Java, Basic/VisualBasic oder Kotlin. Der Computercode kann Unterprogramme umfassen, die in einer proprietären Computersprache geschrieben sind, die spezifisch für das Auslesen oder die Steuerung oder einer anderen Hardwarekomponente des Gerätes vorgesehen sind.

Die Software bzw. das Computerprogrammprodukt umfasst bevorzugt Befehle zur Durchführung des beschriebenen adaptiven Gleichgewichtstraining, wobei das Computerprogrammprodukt auf die vom Betriebssystem bereitgestellten Messdaten zugreifen kann bzw. über das Betriebssystem Hardware des Gerätes (beispielsweise einen Vibrator als Signalgeber für ein Feedbacksignal) ansteuern kann.

Vorteilhaft kann somit durch Bereitstellung des Computerprogrammproduktes eine generisches Mobilgerät für das erfindungsgemäße adaptive Gleichgewichtstraining eingerichtet werden. Zusätzlich Hardware ist hierbei nicht erforderlich. Die Ausgabe von Anweisungen zur Durchführung der Übung, Messung der Veränderung einer Körperposition und/oder eines Bewegungsmusters des Trägers der Vorrichtung während der Übung, Bestimmung von Schwankungswerte, Auswertung der Ergebnisse und erfindungsgemäße Erzeugung eines Feedbacksignals kann vorteilhaft mit Hilfe von "Bordmitteln" (z.B. Sprachausgabe, Ton, Vibration etc.) erfolgen.

Die bevorzugter Ausführungsform stellt mithin eine besonders wirtschaftliche Lösung dar.

### DETAILLIERTE BESCHREIBUNG

Im Folgenden soll die Erfindung anhand von Beispielen näher erläutert werden, ohne auf diese beschränkt zu sein.

Eine besonders vorteilhafte Regelung für ein adaptives Gleichgewichtstraining unter Anpassung der Feedbackschwellen und ggf. Zusammenstellung eines Übungsprogramms wird in der folgenden beispielhaften Beschreibung detaillierter beschrieben, ohne hierauf beschränkt zu sein.

Die Vorrichtung umfasst bevorzugt einer Sensoreinheit, einer Prozesseinheit und Feedbackgeber. Die Sensoreinheit misst die Körperbewegung bevorzugt in mindestens zwei Raumachsen mithilfe von Gyrometern oder Accelerometern. Die Prozesseinheit berechnet Feedbackschwellen und -stufen bevorzugt anhand des hier beschriebenen Algorithmus und gibt dem Patienten Übungsanweisungen (z. B. verbal, visuell). Die Feedbackgeber geben dem Patienten ein Feedback, wenn seine Körperschwankung in der entsprechenden Richtung die berechnete Feedbackschwelle überschreitet.

Das Übungsprogramm umfasst bevorzugt mehrere Durchgänge der ausgewählten Übungen, bevorzugt an einem Trainingstag. Im ersten Durchgang des ersten Trainingstages werden bevorzugt die Maximalwerte der Normwerte der Schwankungsgeschwindigkeit oder -auslenkung der entsprechenden Alters- und Geschlechtsgruppe für die Bewegungen des Körpers nach links, rechts, vorn und hinten verwendet. Die Maximalwerte der Normwerte einer Übung sind die, bei Absolvierung der Übung, auftretenden höchsten Schwankungswerte nach links, rechts, vorn und hinten innerhalb einer Altersgruppe gesunder Probanden des gleichen Geschlechts nach dem Ausschluss von Extremwerten. Extremwerte werden bevorzugt aussortiert, weil sie derart stark abweichen, dass sie für die genannten Zwecke nicht brauchbar sind. Solche Extremwerte können sich beispielsweise durch ein sehr individuelles Bewegungsmuster ergeben, welches auch bei gesunden Probanden vorkommen kann.

Die Maximalwerte der Normwerte können die in der Tabelle 1 dargestellten Wertebereiche annehmen, welche bei einer statistisch relevanten Probandengruppe gemessen wurden. Beim zweiten Durchgang eines jeden Trainingstages wird bevorzugt anhand der Schwankungswerte des ersten Durchgangs für jede Übung und Schwankungsrichtung der Wert ermittelt, der die höchsten 40 % aller Werte einer Schwankungsrichtung und die geringsten 60 % aller Werte teilt. Dies entspricht bevorzugt dem "Median 60". Im Sinne der Erfindung bezeichnet der Median 60, bevorzugt einen Wert für den 60% der Schwankungswerte, insbesondere der Schwankungsgeschwindigkeit und/oder -auslenkung, unterhalb des Wertes liegen und 40% der Schwankungswerte oberhalb des Wertes liegen. Die Definition für einen Median 40, 50, 70, 80. 95 etc. ist analog. Im Sinne der Erfindung wird der mathematische "Median" somit als "Median 50" bezeichnet, da für diesen Wert 50% der Schwankungswerte ober- bzw. unterhalb des Median 50 liegen müssen.

Die Verwendung des Median 60 zeigt bereits gute Ergebnisse. Es wurde jedoch festgestellt, dass beste Ergebnisse erzielt werden können, wenn dieser Wert zwischen 50 und 200% des Maximalwertes der Normwerte der Schwankungsgeschwindigkeit oder -auslenkung der entsprechenden Alters- und Geschlechtsgruppe für die Bewegungen des Körpers in der betrachteten Richtung liegt, s. z. B. Tabelle 1. Wenn dem so ist, wird der Wert als Schwellenwert angenommen. Wenn der berechnete Wert größer ist als 200 % des Maximalwertes der Normwerte der Schwankungsgeschwindigkeit oder -auslenkung der entsprechenden Alters- und Geschlechtsgruppe für die Bewegungen des Körpers in der betrachteten Richtung, wird er auf diesen Wert heruntergesetzt und als Schwellenwert verwendet. Wenn der berechnete Wert kleiner ist als 50 % des Maximalwertes der Normwerte der Schwankungsgeschwindigkeit oder - auslenkung der entsprechenden Alters- und Geschlechtsgruppe für die Bewegungen des Körpers in der betrachteten Richtung, wird er auf diesen Wert heraufgesetzt und als Schwellenwert verwendet. Tabelle 1 definiert bevorzugte Wertebereiche für Maximalwerte der Normwerte der Schwankungsgeschwindigkeit, vorteilhaft kann jeder Wert innerhalb der genannten Grenzen verwandt werden. Besonders gute Ergebnisse werden mit dem Mittelwert der jeweiligen Bereiche erzielt. Für die Gruppe männlich stehend mit offenen Augen im Altersbereich 55-59 wird für eine Schwankung seitwärts beispielsweise ein Bereich von 0,13 °/s bis 0,67 °/s genannt. Geeignete Maximalwerte können somit 0,13 °/s, 0,14 °/s, 0,15 °/s, 0,16 °/s 0,62 °/s, 0,63 °/s, 0,64 °/s, 0,65 °/s, 0,66 °/s und 0,67 °/s sein, besonders bevorzugt 0,4 °/s.

Die Berechnung erfolgt bevorzugt je Übung und Schwankungsrichtung an jedem Übungstag. Der Patient wird zudem bevorzugt mithilfe visueller oder akustischer Anweisungen, insbesondere durch das Ausgabemodul, durch das individuelle Übungsprogramm begleitet, wobei er durch Sprach-, Gesten-, Geräusch- oder Tasteingabe Zustimmung, Ablehnung oder Unterbrechung signalisieren kann.

In einer bevorzugten Ausführungsform der Erfindung wird bei einer Durchführung eines Übungsprogramms anhand einer Ähnlichkeitsanalyse mit einer vorhergehenden Durchführung des Übungsprogrammes eine Rückmeldung über die korrekte Ausführung der Übungen des Übungsprogrammes ausgegeben.

Insbesondere ist die Prozessoreinheit dazu konfiguriert, während einer vorhergehenden Durchführung eines Übungsprogrammes die Bewegungsmuster und/oder die Schwankungswerte zu speichern und bei einer Durchführung des Übungsprogramms anhand einer Ähnlichkeitsanalyse der gespeicherten Bewegungsmuster und/oder der gespeicherten Schwankungswerte eine Rückmeldung über die korrekte Ausführung der Übungen des Übungsprogrammes ausgegeben.

Bei einer vorhergehenden Durchführung kann es sich bevorzugt um eine erste Durchführung handeln.

Bei einer vorhergehenden Durchführung kann es sich insbesondere um eine unter Aufsicht vollzogenen Durchführung handeln.

Bei einer Durchführung eines Übungsprogramms kann es sich um eine zweite Durchführung handeln.

Bei einer Durchführung eines Übungsprogramms kann es sich um eine aktuelle Durchführung handeln.

Eine Ähnlichkeitsanalyse umfasst vorzugsweise eine Berechnung und/oder Analyse der Abweichung der Bewegungsmuster und/oder der Schwankungswerte bei der Durchführung des Übungsprogramms mit der vorhergehenden Durchführung des Übungsprogramms.

Die Vorrichtung verwendet bevorzugt die aufgezeichneten Schwankungsdaten und/oder zusätzlich aufgezeichnete Bilddaten für eine Ähnlichkeitsanalyse der absolvierten Übungen mit bevorzugt zuvor unter Aufsicht vollzogenen Messungen der Körperschwankung und gibt dem Patienten aufgrund der Korrelation beider Datenmengen Rückmeldung über die korrekte Ausführung der einzelnen Übungen. Dazu kann z. B. das Ergebnis der Korrelationsanalyse in Form einer Bewertung angezeigt (gut gemacht (r=0.8), bitte wiederholen (r=0.4), wobei r den Korrelationskoeffizienten darstellt).

Die Auswertung der Bilddaten erfolgt bevorzugt mithilfe der kameragestützten Bewegungserfassung von Kopf und Rumpf sowie der Extremitäten. Dabei wird die x-y-Position der Körperteile in den einzelnen Bilddaten berechnet und mit der Position in früheren Aufnahmen (mit korrekter Übungsausführung) verglichen. In einer bevorzugten Ausführung der Erfindung werden die Bilddaten zunächst für die Erstellung eines Avatars verwendet und die Lageveränderung der Körperteile des Avatars für die Einschätzung der korrekten Übungsausführung verwendet.

In einer bevorzugten Ausführung der Erfindung wird ein individuelles Übungsprogramm auf Grundlage des Betrages des Median 95 der Schwankungsgeschwindigkeit oder -auslenkung für die Seitwärts- und Vorwärts-/Rückwärts-Achse während der Absolvierung einer Batterie von Stand- und Gangübungen erstellt.

Dabei werden bevorzugt die Schwankungswerte mit vorab hinterlegten Normdaten der Schwankungsgeschwindigkeit oder -auslenkung verglichen und nur die Übungen für das Übungsprogramm verwendet, bei denen der Betrag des Median 50 der Schwankungsgeschwindigkeit oder -auslenkung höher ist als der Median 95 der entsprechenden, in der bevorzugt für jedes Geschlecht in 5 Jahresabschnitten bezüglich des Lebensalters zusammengefassten Normwertdaten für die seitwärts- oder vorwärts/rückwärts-Achse. Der Median 95 der Normdaten (berechnet über alle Median 50 Werte der Schwankungsgeschwindigkeit für die Seitwärts- und Vorwärts-/Rückwärts-Achse bei gesunden Probanden bzw. dessen Betrag) können bei gesunden Probanden des gleichen Geschlechts für eine bevorzugte Batterie von Stand- und Gangübungen nach dem Ausschluss von Extremwerten (s. o.) altersabhängig die in **Tabelle 2** dargestellten Wertebereiche annehmen.

Während der Übung verwendet das Gerät bevorzugt die Maximalwerte der Normwerte der Schwankungsgeschwindigkeit oder -auslenkung der entsprechenden Alters- und Geschlechtsgruppe für die Bewegungen des Körpers nach links, rechts, vorn und hinten als Schwellenwerte für ein sensorisches Feedback.

Das Feedbacksignal kann beispielsweise ein visueller, akustischer, taktiler, vibrotaktiler, galvanischer oder gustatorischer Reiz sein. In einer bevorzugten Ausführungsform wird das Feedback je nach Überschreitung der Schwelle in 3 Stärkestufen ausgegeben (Stufe 1 bei einfacher Überschreitung, Stufe 2 bei 1,5-facher Überschreitung, Stufe 3 bei 2-facher Überschreitung). Liegt die mittlere Schwankungsgeschwindigkeit oder -auslenkung in einer Richtung (links, rechts, vor oder zurück) innerhalb eines Zeitintervalls (bevorzugt eine Sekunde) zwischen dem Einfachen und bis zum Anderthalbfachen des berechneten Feedbackwerts, wird mit geringerer Intensität stimuliert. Liegt diese zwischen dem Anderthalbfachen und bis zum Doppelten des berechneten Feedbackwerts wird mit mittlerer Intensität stimuliert. Bei einer noch größeren Schwankungsgeschwindigkeit oder -auslenkung erfolgt die Aktivierung des Feedbacksignals mit hoher Intensität.

### Beispielhafte Anwendung der Erfindung

Ein Patient klagt über Stand- oder Gangunsicherheit bzw. Gleichgewichtsstörungen. Zur Ermittlung seiner spezifischen Defizite hinsichtlich der Körperbalance absolviert der Patient zunächst eine Batterie von Stand- und Gangaufgaben unter verschiedenen sensorischen Bedingungen. So steht er z. B. auf einer Schaumstoffmatte mit offenen und geschlossenen Augen, geht mit seiner Präferenzgeschwindigkeit über eine Distanz mit offenen oder geschlossenen Augen usw. Durch die Kombination von sensorischen und motorischen Aufgaben wird eine große Bandbreite von alltagsrelevanten Konditionen absolviert. Das erfindungsgemäße Gerät ist währenddessen außen am Körper, nahe des Körperschwerpunktes, befestigt. Für die Befestigung wird z. B. ein Hüftgürtel oder Klebepad verwendet. Im Gerät bestimmen bevorzugt Accelerometer und/oder Gyrometer fortwährend die Parameter der Lageveränderung des Körperschwerpunktes. Das sind insbesondere die Schwankungsgeschwindigkeit und die Beschleunigung/Auslenkung seitwärts sowie vor- und rückwärts.

Diese Parameter werden neben der Ganggeschwindigkeit für die Analyse über einen Zeitraum von z. B. 20 s (bei allen Stehübungen) oder über die gesamte Dauer der Absolvierung der Gehstrecke (bei allen Gehübungen) ausgewertet, indem zunächst der Median 50 der Beträge aller gemessenen bzw. berechneten Schwankungswerte in der Seitwärtsrichtung sowie in der Vorwärts-/Rückwärtsrichtung berechnet wird. Beide Werte werden dann mit den entsprechenden alters- und geschlechtsrelationierten Normdaten verglichen, die zuvor an gesunden Probanden mit ähnlichem Körperbau und aus gleicher Ethnie erhoben wurden. Dazu wurde z. B. der Median 50 (bzw. dessen Betrag) der Schwankungswerte (z. B. Schwankungsgeschwindigkeit) bei mindestens 15 geeigneten Probanden während der Absolvierung der oben genannten Batterie von Stand- und Gangaufgaben bestimmt. Für jede Übung und jede Schwankungsrichtung (Seitwärts- und Vorwärts-/Rückwärts-Achse) wird dann der Median 95 (bzw. dessen Betrag) aus den Ergebnissen (Median 50) aller geeigneten Probanden berechnet. Die daraus resultierenden Werte sollten je nach Ethnie und Körperbau der Probanden Werte zwischen den entsprechenden von/bis-Angaben in Tabelle 2 annehmen. Liegen die Ergebnisse des Patienten über den so ermittelten Normdaten, ist eine zu geringe Kontrolle der Körperbalance in dieser Übung anzunehmen.

In einer besonders bevorzugten Variante der Erfindung berechnet das erfindungsgemäße Gerät einen Balancescore, der die Gleichgewichtsleistung des Patienten über alle absolvierten Übungen einschätzt. Dazu wird das Messergebnis der Schwankungen (Median 50 aller Werte je Übung und Achse) in ein prozentuales Verhältnis zu den oben beschriebenen Normdaten gesetzt, über alle Übungen (14 im hier dargestellten Beispiel) aufaddiert, mit einhundert multipliziert und durch die Anzahl der Übungen, multipliziert mit 400, geteilt. Für den Patienten nicht korrekt absolvierbare Übungen (d. h. solche Übungen, die er körperlich nicht schafft) fließen mit dem doppelten Wert der Normdaten in die Berechnung ein. Absolviert der Patient z. B. im beschriebenen Fall insgesamt alle 14 Übungen, wird der Median 50 der Beträge aller Werte einer Schwankungsachse (seitwärts oder vor/zurück) jeder Übung mit 100 multipliziert und durch den entsprechenden Normwert aus den Normdaten geteilt. Die 28 Werte (jeweils einmal die Schwankungen seitwärts und einmal vor-zurück) werden nun addiert und mit 100 multipliziert. Das Ergebnis wird durch 14 (beispielhafte Anzahl der absolvierten Übungen), multipliziert mit 400, geteilt. Der so erhaltene Balancescore liegt zwischen 0 und 100 und kann zusammenfassend mithilfe folgender Formel berechnet werden: $Balancescore = \frac{\left({\sum }_{i} pi +{\sum }_{i} ri\right) ∗ 100}{n ∗ 400}$ wobei: *p* bevorzugt der Schwankung vor-zurück geteilt durch den Normwert in % entspricht *(i* ist vorzugsweise der Index der jeweiligen Übung), r entspricht bevorzugt der Schwankung seitwärts geteilt durch den Normwert in % und n entspricht vorzugsweise der Anzahl der getesteten Übungen.

Zeigt der Patient eine zu geringe Kontrolle der Körperbalance in einer Übung nach oben beschriebener Auswertung oder einen Balancescore über alle Übungen von bevorzugt mehr als 50, so bereitet das erfindungsgemäße Gerät ein individuelles Gleichgewichtstraining automatisch vor. Dafür werden Übungen aus der absolvierten Batterie von Stand- und Gangaufgaben für das Trainingsprogramm zusammengestellt, in denen der Patient eine zu geringe Kontrolle der Körperbalance zeigt. Die Maximalwerte der Normwerte werden für diese Übungen bevorzugt als Schwellenwerte für das Feedbacksignal im ersten Trainingsdurchgang festgelegt. Damit sind die Maximalwerte des Median 50 der Beträge der Schwankungswerte je Richtung (rechts, links, vorn, hinten) innerhalb der Schwankungsachsen (seitwärts und vor-/rückwärts) von gesunden Probanden gemeint (Erstellung der Normdaten s. ebenfalls oben). Diese Werte sollten je nach Ethnie und Körperbau der Probanden Werte zwischen den entsprechenden von/bis-Angaben in **Tabelle 1** annehmen. Entsprechend der Richtungszuordnung erhält der Patient bei Überschreitung des Schwellenwertes ein Feedbacksignal (z. B. einen Vibrationsimpuls) in der jeweiligen Richtung.

Während der Übungsdurchführung zeichnet das erfindungsgemäße Gerät fortwährend die Parameter der Lageveränderungen des Körperschwerpunktes auf. Da das Training der gleichen Übung bevorzugt mehrmals am Tag und auch an mehreren darauffolgenden Tagen durchgeführt wird, werden jeweils aus der letzten Aufzeichnung der Lageveränderungen des Körperschwerpunktes die Schwellenwerte (rechts, links, vorn, hinten) für das Feedbacksignal im nächsten Übungsdurchgang errechnet. Zunächst berechnet das erfindungsgemäße Gerät den Median 60 der Beträge der Schwankungsgeschwindigkeit jeder Schwankungsrichtung (rechts, links, vor, zurück). Untersuchungen konnten nachweisen, dass einige Patienten bei Verwendung dieser Werte als Feedbackschwellen sehr gut vom Training profitierten (Abb. 1, Variante B). Andere Patienten verringerten hingegen nicht sehr deutlich ihre Körperschwankungen infolge des Trainings (Abb. 1b). Der Median 60 der Beträge der Schwankungsgeschwindigkeit jeder Schwankungsrichtung bei den Patienten, deren Feedbackschwelle durch die Vorrichtung (Abb.1, Variante C) zwischen 50% und 200% des Maximalwertes der Normwerte der Schwankungsgeschwindigkeit gelegt wurde, konnten deutlich stärker vom Training profitieren. Deshalb wird im erfindungsgemäßen Gerät vor jeder Aktualisierung der Schwellenwerte für den nächsten Übungsdurchgang geprüft, ob diese im oben genannten Bereich liegen. Ist das nicht der Fall, werden sie bei Unterschreitung durch das Setzen an die untere und bei Überschreitung durch das Setzen an die obere Grenze korrigiert.

Wenn z. B. bei einem 70-jährigen Mann der Median 60 der Beträge der Schwankungsgeschwindigkeit aller Werte nach rechts beim Stehen mit offenen Augen 1,0 °/s beträgt und der Maximalwert der Normwerte der Schwankungsgeschwindigkeit seiner Ethnie und seines Körperbaus mit 0,9 °/s festgestellt wurde, kann der Schwellenwert auf 1,0 °/s festgelegt werden, da 1,0 °/s zwischen 0,5 °/s und 1,8 °/s (50 % bzw. 200 % des Maximalwertes der Normwerte der Schwankungsgeschwindigkeit) liegt. Würde der oben genannte Wert des 70-jährigen Mannes nicht 1,0 °/s sondern 2,0 °/s betragen, wird er auf einen Schwellenwert von 1,8 °/s korrigiert. Wird für den Patienten ein Median 60 der Beträge der Schwankungsgeschwindigkeit aller Werte nach rechts beim Stehen mit offenen Augen von nur 0,4 °/s festgestellt, wird als Schwellenwert für die Aktivierung des Feedbacksignals 0,5 °/s verwendet.

Somit adaptiert das erfindungsgemäße Gerät die Schwellenwerte des Feedbacksignals während des gesamten Trainingsprogramms an die Performance des Patienten. Zusätzlich wird der Patient mithilfe der Modulation der Stärke des Feedbacksignals zur Verbesserung der Performance motiviert. Liegt die mittlere Schwankung nach rechts im oben beschriebenen Beispiel eines 70-jährigen Mannes mit einem Schwellenwert von 1,0 °/s innerhalb eines Zeitintervalls (bevorzugt eine Sekunde), z. B. bei 2,1 °/s, wird mit höchster Intensität stimuliert. Senkt sich die mittlere Schwankung z. B. auf 1,9 °/s ab, wird mit einer mittleren Intensität stimuliert. Reduziert sich die Schwankung weiter auf z. B. 1,4 °/s, erhält der Patient nur eine geringe Stimulation. Bei zunehmender Schwankung wird die Stimulation in gleicher Abstufung entsprechend erhöht.

Die im erfindungsgemäßen Gerät gespeicherten Schwankungsdaten und/oder Bildmaterialien der Übungsdurchgänge werden nach dem Training bzw. nach jedem Übungsdurchgang dafür genutzt, den Patienten über die korrekte Durchführung des Übungsablaufes zu informieren. Dazu führt das erfindungsgemäße Gerät eine Ähnlichkeitsanalyse des Schwankungsmusters nach rechts, links, vorn und hinten durch, indem es den Grad der Übereinstimmung der gespeicherten Werte des zu kontrollierenden Übungsdurchgangs mit dem ersten, bevorzugt unter fachlicher Aufsicht absolvierten Übungsdurchgang, korreliert. Das Ergebnis der Analyse wird dem Patienten mitgeteilt. So kann bei einem schlechten Ergebnis der Ähnlichkeitsanalyse die Empfehlung gegeben werden, die Übung zu wiederholen. Wird eine hohe Ähnlichkeit festgestellt, ist der Patient zu belobigen.

Nach dem letzten Training absolviert der Patient nochmals eine Batterie von Stand- und Gangaufgaben unter verschiedenen sensorischen Bedingungen, ohne ein Feedbacksignal zu erhalten, wobei das erfindungsgemäße Gerät, wie oben beschrieben, die Körperbalance ermittelt.

Durch den Vergleich der Ergebnisse vor und nach dem Training wird der Trainingserfolg festgestellt.

### Mögliche Übungsformen

Die im Folgenden geschilderten 14 Übungsformen sind beispielhaft.

### Stehend auf beiden Beinen - Augen offen:

Das Subjekt muss auf beiden Beinen stehen, die Augen sollten auf einen fixen Punkt vor ihm/ihr für 20 Sekunden gerichtet sein. Die Füße sollen parallel in einer Distanz von 15 cm voneinander positioniert werden.

### Stehend auf beiden Beinen - Augen geschlossen:

Wie oben, nur mit geschlossenen Augen.

### Stehend auf einem Bein - Augen offen

Das Subjekt steht auf einem Bein, die Augen nach vorne gerichtet und auf einen Punkt oder Objekt auf Augenhöhe fixiert. Am Beginn der Messung soll der Patient sein schwächeres Bein für 20 Sekunden vom Boden heben.

### 8 Ferse-zu-Zehen Schritte - Augen offen

Das Subjekt soll acht Schritte machen, indem die Ferse eines Fußes direkt vor den großen Zeh des anderen Fußes gesetzt wird.

### Stehend mit beiden Beinen auf einer Schaumstoffmatte - Augen offen

Wie die erste Übung oben, jedoch steht der Patient diesmal auf einer Schaummatte.

### Stehend mit beiden Beinen auf einer Schaumstoffmatte - Augen geschlossen

Wie vorhergehend, jedoch mit geschlossenen Augen.

### 8 Ferse-zu-Zehen Schritte auf einer Schaumstoffmatte - Augen offen

Wie vorstehend beschrieben, nur auf einer Schaumstoffmatte.

### 3 Meter Gehen bei normaler Geschwindigkeit

Der Patient soll 3 Meter bei seiner normalen Gehgeschwindigkeit gehen und dabei geradeaus schauen.

### 3 Meter Gehen bei normaler Geschwindigkeit und gleichzeitiger Drehbewegung des Kopfes (einmal pro genommenen Schritt)

Wie vorstehend, jedoch soll der Patient seinen Kopf einmal pro Schritt kreisförmig bis zur drehen.

### 3 Meter Gehen bei normaler Geschwindigkeit und gleichzeitiger Nickbewegung des Kopfes (einmal pro genommenen Schritt)

Wie vorstehend beschrieben, jedoch mit einer vollständigen Nickbewegung statt Drehbewegung pro Schritt.

### 3 Meter gehen bei Normalgeschwindigkeit - Augen geschlossen

Wie oben, nur mit geschlossenen Augen.

### Gehen bei gleichzeitiger Überwindung von Barrieren (24cm hoch, 1m Abstand)

Der Patient steigt jeweils mit dem linken Fuß über insgesamt 4 Barrieren und nimmt einen Schritt mit dem rechten Fuß zwischen den Barrieren. Der Fuß muss über die Barriere gehoben werden und darf nicht seitlich an der Barriere vorbei geschwenkt werden.

### Hinsetzen

Das Subjekt wird mit dem Rücken zu einem Stuhl platziert und soll sich ohne Hilfsmittel und ohne Sichtkontakt mit dem Stuhl hinsetzen

### Aufstehen

Das Subjekt sitzt auf einen normalen Stuhl und soll ohne Hilfsmittel aufstehen.

### Kurzbeschreibung der Abbildung

- **Abb. 1A**: Reduktion der Vorwärts- und Rückwärtsschwankungen nach 10-tägiger Übung ohne und mit einer bevorzugten Vorrichtung gemäß verschiedener Ausführungsformen der Erfindung.
- **Abb. 1B**: Reduktion der Seitwärtsschwankungen nach 10 tägiger Übung ohne und mit bevorzugter Vorrichtung gemäß verschiedener Ausführungsformen der Erfindung

### Ausführliche Beschreibung der Abbildung

**Abb. 1A** zeigt die Reduktion der Vorwärts- und Rückwärtsschwankungen unter Übung ohne (Variante A) und mit bevorzugter Vorrichtung gemäß verschiedener Ausführungsformen der Erfindung (Varianten B und C). "AO" steht für eine Durchführung mit Augen offen, "AG" für eine Durchführung mit Augen geschlossen.

Variante A zeigt die Reduktion der Körperschwankung nach einem 10-tägigen Gleichgewichtstraining in einem klinischen Setup gemäß dem Stand der Technik, das bedeutet insbesondere ein Training unter klinischer Aufsicht ohne erfindungsgemäße Vorrichtung.

Variante B zeigt die Durchführung des identischen Trainingsprogramms, jedoch unter Verwendung der Vorrichtung, wobei während einer ersten Durchführung eines Übungsprogrammes die Schwankungswerte für die Bewegungsmuster gespeichert werden und anhand der gespeicherten Schwankungswerte die Feedbackschwelle für eine spätere zweite Durchführung eines Übungsprogramms berechnet wurde. Es wurden die aktuellen Feedbackschwellen immer durch die jeweilig vorherigen, gespeicherten Schwankungswerte berechnet. Hier wurde eine Ausführungsform gewählt, bei der die Feedbackschwelle dem Median 60 der zuvor gespeicherten Schwankungswerte entspricht. Es zeigt sich bereits hier eine deutlich verbesserte Reduktion im Vergleich zum klassischen durch Medizinpersonal betreutem Training.

Die derart bestimmten Feedbackschwellen sind besser geeignet, um schnelle Fortschritte beim Gleichgewichtstraining zu erzielen, als es bei einem klinischen Setup durch einen geschulten Betreuer erreicht werden kann, der die Probanden bei ihren Übungen korrigiert und anleitet. Zudem lässt sich das Training mittels der Vorrichtung selbstständig zu Hause durchführen, sodass eine unkomplizierte Integration in den Alltag der Probanden möglich ist, sodass sich der Behandlungserfolg nochmals erhöht.

Variante C unterscheidet sich von Variante B zusätzlich dadurch, dass die Feedbackschwellen für jede Schwankungsrichtung sich zwischen 50% und 200% des Maximalwertes der Normwerte der Schwankungsgeschwindigkeit befinden müssen. Es kann eine weitere Verbesserung erzielt werden, welche vermutlich besonders deutlich bei Probanden mit starken Schwankungen oder bei für einzelne Probanden besonders schwierigen Übungen ausfällt.

**Abb.** 1B zeigt die Reduktion der Seitwärtsschwankungen unter Übung ohne (Variante A) und mit Vorrichtung gemäß der oben beschriebenen Ausführungsformen (Varianten B und C).

Es wurden Vergleichsgruppen einer statistisch relevanten Gruppengröße (> 14 Probanden) mit ähnlichen Pathologien bezüglich ihrer Gleichgewichtsprobleme verglichen.

Die Daten zeigen, dass unter einer erfindungsgemäß bevorzugten Berechnung der Feedbackschwellen und Begrenzung der Feedbackschwelle zwischen 50% und 200% der maximalen Normwerte der Schwankungsgeschwindigkeit besonders gute Ergebnisse erzielt werden können. Besonders vorteilhaft ist, dass ausgezeichnete Therapieerfolge mittels einer derart automatisierten Berechnung und Anpassung der Feedbackschwellen erfolgen kann, ohne dass medizinisches Fachpersonal individualisierte Anpassungen vornehmen muss. Vielmehr ist der vorgeschlagene Algorithmus zur adaptiven Berechnung und Festlegung der Feedbackschwellen für sukzessive Übungen äußerst robust und führt zuverlässig zu einem Behandlungserfolg bei diversen Patientengruppen.

**Tabelle 1: Bereich der Maximalwerte der Normwerte für die Schwankungsgeschwindigkeit des Körperschwerpunktes (Winkelgeschwindigkeit in °/s) bei verschiedenen Altersgruppen und unterschiedlichen Übungen/Aufgaben sowie beider Geschlechter (AO = Augen offen, AG = Augen geschlossen). Es werden rotatorische Schwankungen betrachtet.**

| | | Alter | | rechts | | links | | vorwärts | | rückwä rts | |
|---|---|---|---|---|---|---|---|---|---|---|---|
| Geschlecht | Aufgabe | von | bis | von | bis | von | bis | von | bis | von | bis |
| männlich | stehen AO | 15 | 19 | 0,43 | 2,16 | 0,22 | 1,10 | 0,43 | 2,16 | 0,36 | 1,80 |
| männlich | stehen AO | 20 | 24 | 0,58 | 2,89 | 0,27 | 1,35 | 0,54 | 2,70 | 0,40 | 2,00 |
| männlich | stehen AO | 25 | 29 | 0,53 | 2,63 | 0,23 | 1,14 | 0,43 | 2,16 | 0,49 | 2,45 |
| männlich | stehen AO | 30 | 34 | 0,47 | 2,37 | 0,19 | 0,94 | 0,32 | 1,62 | 0,58 | 2,90 |
| männlich | stehen AO | 35 | 39 | 0,48 | 2,39 | 0,15 | 0,77 | 0,30 | 1,50 | 0,60 | 2,98 |
| männlich | stehen AO | 40 | 44 | 0,47 | 2,34 | 0,23 | 1,17 | 0,27 | 1,36 | 0,58 | 2,88 |
| männlich | stehen AO | 45 | 49 | 0,44 | 2,21 | 0,32 | 1,58 | 0,29 | 1,45 | 0,49 | 2,46 |
| männlich | stehen AO | 50 | 54 | 0,42 | 2,09 | 0,40 | 1,98 | 0,31 | 1,54 | 0,41 | 2,03 |
| männlich | stehen AO | 55 | 59 | 0,60 | 2,99 | 0,34 | 1,70 | 0,52 | 2,61 | 0,90 | 4,50 |
| männlich | stehen AO | 60 | 64 | 0,52 | 2,60 | 0,33 | 1,66 | 0,44 | 2,19 | 0,65 | 3,26 |
| männlich | stehen AO | 65 | 69 | 0,58 | 2,88 | 0,41 | 2,04 | 0,84 | 4,19 | 0,98 | 4,89 |
| männlich | stehen AO | 70 | 74 | 0,59 | 2,93 | 0,43 | 2,17 | 0,54 | 2,72 | 0,86 | 4,31 |
| männlich | stehen AO | 75 | 79 | 1,02 | 5,12 | 0,91 | 4,55 | 1,15 | 5,76 | 1,04 | 5,22 |
| männlich | stehen AO | 80 | 84 | 0,66 | 3,30 | 0,46 | 2,31 | 0,29 | 1,43 | 0,41 | 2,03 |
| männlich | stehen AO | 85 | 90 | 0,72 | 3,62 | 0,53 | 2,64 | 0,35 | 1,76 | 0,46 | 2,31 |
| männlich | stehen AG | 15 | 19 | 0,69 | 3,44 | 0,52 | 2,60 | 0,48 | 2,39 | 0,84 | 4,22 |
| männlich | stehen AG | 20 | 24 | 0,90 | 4,50 | 0,33 | 1,66 | 0,62 | 3,09 | 0,95 | 4,73 |
| männlich | stehen AG | 25 | 29 | 0,66 | 3,31 | 0,62 | 3,08 | 0,60 | 2,99 | 0,89 | 4,43 |
| männlich | stehen AG | 30 | 34 | 0,79 | 3,96 | 0,54 | 2,70 | 0,61 | 3,03 | 0,98 | 4,88 |
| männlich | stehen AG | 35 | 39 | 0,43 | 2,14 | 0,15 | 0,76 | 0,37 | 1,85 | 0,54 | 2,72 |
| männlich | stehen AG | 40 | 44 | 0,57 | 2,87 | 0,18 | 0,91 | 0,36 | 1,79 | 0,72 | 3,59 |
| männlich | stehen AG | 45 | 49 | 0,64 | 3,20 | 0,32 | 1,59 | 0,46 | 2,31 | 0,72 | 3,62 |
| männlich | stehen AG | 50 | 54 | 0,43 | 2,14 | 0,37 | 1,85 | 0,34 | 1,70 | 0,61 | 3,07 |
| männlich | stehen AG | 55 | 59 | 0,45 | 2,25 | 0,41 | 2,05 | 0,26 | 1,29 | 0,74 | 3,69 |
| männlich | stehen AG | 60 | 64 | 0,56 | 2,82 | 0,54 | 2,69 | 0,37 | 1,84 | 0,65 | 3,26 |
| männlich | stehen AG | 65 | 69 | 0,54 | 2,70 | 0,53 | 2,66 | 0,76 | 3,81 | 1,00 | 5,01 |
| männlich | stehen AG | 70 | 74 | 0,56 | 2,81 | 0,40 | 1,99 | 0,47 | 2,37 | 0,67 | 3,37 |
| männlich | stehen AG | 75 | 79 | 0,72 | 3,60 | 0,47 | 2,35 | 0,87 | 4,34 | 0,89 | 4,44 |
| männlich | stehen AG | 80 | 84 | 0,59 | 2,96 | 0,54 | 2,69 | 0,35 | 1,76 | 0,40 | 1,98 |
| männlich | stehen AG | 85 | 90 | 0,75 | 3,74 | 0,51 | 2,53 | 0,52 | 2,59 | 0,63 | 3,13 |
| | | | | | | | | | | | |

| | | Alter | | rechts | | links | | vorwärts | | rückwärts | |
|---|---|---|---|---|---|---|---|---|---|---|---|
| Geschlecht | Aufgabe | von | bis | von | bis | von | bis | von | bis | von | bis |
| männlich | stehen 1 Bein AO | 15 | 19 | 1,62 | 8,09 | 1,62 | 8,09 | 1,94 | 9,71 | 1,82 | 9,08 |
| männlich | stehen 1 Bein AO | 20 | 24 | 1,55 | 7,77 | 1,64 | 8,20 | 1,98 | 9,92 | 1,94 | 9,71 |
| männlich | stehen 1 Bein AO | 25 | 29 | 1,41 | 7,03 | 1,08 | 5,38 | 1,27 | 6,36 | 1,37 | 6,87 |
| männlich | stehen 1 Bein AO | 30 | 34 | 1,35 | 6,76 | 0,84 | 4,22 | 0,86 | 4,30 | 1,32 | 6,58 |
| männlich | stehen 1 Bein AO | 35 | 39 | 1,96 | 9,79 | 1,49 | 7,44 | 1,62 | 8,09 | 1,51 | 7,55 |
| männlich | stehen 1 Bein AO | 40 | 44 | 1,45 | 7,24 | 1,53 | 7,63 | 1,71 | 8,54 | 2,13 | 10,65 |
| männlich | stehen 1 Bein AO | 45 | 49 | 2,37 | 11,87 | 2,32 | 11,58 | 2,56 | 12,80 | 2,25 | 11,25 |
| männlich | stehen 1 Bein AO | 50 | 54 | 3,02 | 15,09 | 2,98 | 14,88 | 2,80 | 14,01 | 3,69 | 18,43 |
| männlich | stehen 1 Bein AO | 55 | 59 | 3,02 | 15,09 | 4,19 | 20,95 | 2,57 | 12,87 | 3,07 | 15,34 |
| männlich | stehen 1 Bein AO | 60 | 64 | 4,12 | 20,60 | 4,77 | 23,84 | 3,39 | 16,97 | 3,24 | 16,20 |
| männlich | stehen 1 Bein AO | 65 | 69 | 5,33 | 26,67 | 5,11 | 25,54 | 4,86 | 24,32 | 5,00 | 24,99 |
| männlich | stehen 1 Bein AO | 70 | 74 | 3,94 | 19,70 | 4,40 | 21,98 | 4,49 | 22,44 | 4,33 | 21,64 |
| männlich | stehen 1 Bein AO | 75 | 79 | 6,20 | 30,98 | 5,11 | 25,55 | 4,26 | 21,31 | 3,83 | 19,17 |
| männlich | stehen 1 Bein AO | 80 | 84 | 5,14 | 25,71 | 4,31 | 21,54 | 3,58 | 17,91 | 2,66 | 13,29 |
| männlich | stehen 1 Bein AO | 85 | 90 | 4,85 | 24,23 | 5,29 | 26,43 | 3,32 | 16,59 | 2,77 | 13,85 |
| männlich | stehen 1 Bein AG | 15 | 19 | 4,40 | 22,02 | 4,48 | 22,40 | 7,03 | 35,16 | 5,95 | 29,75 |
| männlich | stehen 1 Bein AG | 20 | 24 | 4,46 | 22,32 | 5,28 | 26,41 | 6,37 | 31,84 | 5,77 | 28,85 |
| männlich | stehen 1 Bein AG | 25 | 29 | 4,62 | 23,09 | 4,21 | 21,05 | 5,69 | 28,43 | 5,85 | 29,23 |
| männlich | stehen 1 Bein AG | 30 | 34 | 5,67 | 28,35 | 6,68 | 33,39 | 6,41 | 32,03 | 6,50 | 32,51 |
| männlich | stehen 1 Bein AG | 35 | 39 | 8,29 | 41,43 | 7,06 | 35,30 | 8,14 | 40,70 | 8,70 | 43,50 |
| männlich | stehen 1 Bein AG | 40 | 44 | 6,99 | 34,95 | 6,60 | 32,99 | 7,46 | 37,30 | 7,62 | 38,12 |
| männlich | stehen 1 Bein AG | 45 | 49 | 8,58 | 42,88 | 8,58 | 42,91 | 10,64 | 53,19 | 10,49 | 52,44 |
| männlich | stehen 1 Bein AG | 50 | 54 | 5,73 | 28,65 | 6,12 | 30,59 | 4,52 | 22,60 | 4,80 | 23,98 |
| männlich | stehen 1 Bein AG | 55 | 60 | 19,15 | 95,77 | 18,99 | 94,96 | 19,86 | 99,31 | 18,41 | 92,03 |
| männlich | gehen tandem | 15 | 19 | 3,76 | 18,80 | 4,02 | 20,12 | 6,66 | 33,30 | 4,35 | 21,77 |
| männlich | gehen tandem | 20 | 24 | 5,20 | 25,98 | 4,15 | 20,75 | 5,38 | 26,91 | 7,14 | 35,68 |
| männlich | gehen tandem | 25 | 29 | 7,13 | 35,64 | 4,22 | 21,10 | 5,21 | 26,04 | 8,97 | 44,86 |
| männlich | gehen tandem | 30 | 34 | 5,99 | 29,94 | 5,34 | 26,69 | 5,59 | 27,97 | 8,27 | 41,35 |
| männlich | gehen tandem | 35 | 39 | 6,37 | 31,85 | 4,02 | 20,10 | 5,24 | 26,20 | 8,05 | 40,25 |
| männlich | gehen tandem | 40 | 44 | 6,65 | 33,23 | 4,24 | 21,18 | 5,62 | 28,09 | 9,39 | 46,97 |
| männlich | gehen tandem | 45 | 49 | 6,69 | 33,43 | 5,21 | 26,04 | 6,44 | 32,18 | 10,53 | 52,67 |
| männlich | gehen tandem | 50 | 54 | 6,20 | 30,98 | 6,16 | 30,82 | 5,09 | 25,44 | 5,98 | 29,88 |
| männlich | gehen tandem | 55 | 59 | 6,87 | 34,33 | 7,12 | 35,61 | 5,62 | 28,10 | 10,33 | 51,66 |
| männlich | gehen tandem | 60 | 64 | 6,12 | 30,61 | 4,60 | 23,01 | 5,74 | 28,71 | 7,88 | 39,40 |
| männlich | gehen tandem | 65 | 69 | 6,84 | 34,18 | 5,79 | 28,97 | 8,21 | 41,06 | 9,95 | 49,75 |
| männlich | gehen tandem | 70 | 74 | 5,58 | 27,89 | 5,34 | 26,69 | 6,53 | 32,63 | 8,52 | 42,60 |
| männlich | gehen tandem | 75 | 79 | 5,81 | 29,07 | 5,22 | 26,11 | 8,08 | 40,40 | 7,95 | 39,77 |
| männlich | gehen tandem | 80 | 84 | 7,53 | 37,64 | 8,73 | 43,63 | 6,58 | 32,91 | 6,14 | 30,72 |
| männlich | gehen tandem | 85 | 90 | 7,49 | 37,47 | 6,99 | 34,95 | 5,91 | 29,57 | 7,33 | 36,65 |
| männlich | stehen Schaumstoff AO | 15 | 19 | 0,84 | 4,22 | 0,42 | 2,09 | 0,80 | 4,02 | 0,74 | 3,71 |
| männlich | stehen Schaumstoff AO | 20 | 24 | 0,79 | 3,97 | 0,34 | 1,69 | 0,63 | 3,16 | 0,78 | 3,92 |
| männlich | stehen Schaumstoff AO | 25 | 29 | 0,71 | 3,56 | 0,37 | 1,86 | 0,65 | 3,24 | 1,06 | 5,31 |
| männlich | stehen Schaumstoff AO | 30 | 34 | 1,13 | 5,67 | 0,63 | 3,14 | 1,13 | 5,66 | 1,32 | 6,58 |
| männlich | stehen Schaumstoff AO | 35 | 39 | 0,78 | 3,90 | 0,29 | 1,43 | 0,53 | 2,64 | 0,54 | 2,72 |
| männlich | stehen Schaumstoff AO | 40 | 44 | 0,74 | 3,69 | 0,54 | 2,69 | 0,40 | 1,99 | 0,76 | 3,81 |
| männlich | stehen Schaumstoff AO | 45 | 49 | 0,80 | 4,01 | 0,42 | 2,11 | 0,69 | 3,45 | 1,03 | 5,17 |
| männlich | stehen Schaumstoff AO | 50 | 54 | 0,52 | 2,59 | 0,48 | 2,42 | 0,39 | 1,94 | 0,83 | 4,15 |
| männlich | stehen Schaumstoff AO | 55 | 59 | 0,73 | 3,64 | 0,55 | 2,73 | 0,38 | 1,90 | 1,18 | 5,89 |
| männlich | stehen Schaumstoff AO | 60 | 64 | 0,80 | 3,98 | 0,48 | 2,39 | 0,57 | 2,83 | 1,01 | 5,03 |
| männlich | stehen Schaumstoff AO | 65 | 69 | 0,84 | 4,20 | 0,64 | 3,22 | 0,91 | 4,55 | 1,15 | 5,74 |
| männlich | stehen Schaumstoff AO | 70 | 74 | 1,00 | 4,98 | 0,70 | 3,49 | 0,89 | 4,45 | 1,45 | 7,24 |
| männlich | stehen Schaumstoff AO | 75 | 79 | 0,98 | 4,92 | 0,72 | 3,58 | 1,06 | 5,32 | 1,19 | 5,96 |
| männlich | stehen Schaumstoff AO | 80 | 84 | 1,04 | 5,22 | 1,16 | 5,78 | 0,70 | 3,52 | 0,86 | 4,29 |
| männlich | stehen Schaumstoff AO | 85 | 90 | 1,20 | 5,99 | 1,36 | 6,81 | 0,91 | 4,56 | 0,93 | 4,67 |
| | | | | | | | | | | | |

| | | Alter | | rechts | | links | | vorwärts | | rückwärts | |
|---|---|---|---|---|---|---|---|---|---|---|---|
| Geschleht | Aufgabe | von | bis | von | bis | von | bis | von | bis | von | bis |
| männlich | stehen Schaumstoff AG | 15 | 19 | 0,72 | 3,59 | 0,46 | 2,31 | 0,76 | 3,78 | 0,65 | 3,27 |
| männlich | stehen Schaumstoff AG | 20 | 24 | 0,71 | 3,57 | 0,44 | 2,19 | 0,59 | 2,94 | 0,66 | 3,31 |
| männlich | stehen Schaumstoff AG | 25 | 29 | 0,81 | 4,05 | 0,53 | 2,64 | 0,71 | 3,53 | 1,10 | 5,50 |
| männlich | stehen Schaumstoff AG | 30 | 34 | 0,68 | 3,41 | 0,26 | 1,30 | 0,50 | 2,48 | 1,18 | 5,92 |
| männlich | stehen Schaumstoff AG | 35 | 39 | 0,67 | 3,37 | 0,16 | 0,82 | 0,46 | 2,29 | 0,63 | 3,16 |
| männlich | stehen Schaumstoff AG | 40 | 44 | 0,88 | 4,40 | 0,29 | 1,46 | 0,37 | 1,84 | 0,89 | 4,43 |
| männlich | stehen Schaumstoff AG | 45 | 49 | 1,21 | 6,07 | 0,41 | 2,04 | 1,38 | 6,90 | 1,13 | 5,64 |
| männlich | stehen Schaumstoff AG | 50 | 54 | 0,65 | 3,25 | 0,56 | 2,82 | 0,44 | 2,22 | 1,07 | 5,36 |
| männlich | stehen Schaumstoff AG | 55 | 59 | 0,92 | 4,60 | 0,72 | 3,60 | 0,54 | 2,70 | 1,19 | 5,96 |
| männlich | stehen Schaumstoff AG | 60 | 64 | 1,20 | 6,01 | 0,89 | 4,47 | 0,80 | 3,98 | 1,25 | 6,26 |
| männlich | stehen Schaumstoff AG | 65 | 69 | 1,26 | 6,31 | 0,85 | 4,27 | 1,17 | 5,86 | 1,71 | 8,55 |
| männlich | stehen Schaumstoff AG | 70 | 74 | 1,63 | 8,15 | 1,32 | 6,62 | 1,59 | 7,97 | 2,34 | 11,69 |
| männlich | stehen Schaumstoff AG | 75 | 79 | 1,38 | 6,92 | 1,15 | 5,73 | 2,20 | 10,99 | 2,80 | 14,01 |
| männlich | stehen Schaumstoff AG | 80 | 84 | 1,67 | 8,36 | 1,82 | 9,12 | 1,29 | 6,44 | 1,25 | 6,27 |
| männlich | stehen Schaumstoff AG | 85 | 90 | 2,20 | 11,00 | 2,55 | 12,75 | 1,65 | 8,24 | 1,89 | 9,45 |
| männlich | stehen Schaumstoff 1 Bein | 15 | 19 | 1,52 | 7,58 | 1,54 | 7,68 | 1,34 | 6,71 | 1,25 | 6,27 |
| männlich | stehen Schaumstoff 1 Bein | 20 | 24 | 1,48 | 7,38 | 1,52 | 7,61 | 1,49 | 7,43 | 1,58 | 7,90 |
| männlich | stehen Schaumstoff 1 Bein | 25 | 29 | 1,95 | 9,76 | 1,93 | 9,63 | 2,08 | 10,42 | 2,31 | 11,56 |
| männlich | stehen Schaumstoff 1 Bein | 30 | 34 | 3,06 | 15,28 | 2,32 | 11,58 | 2,15 | 10,73 | 2,23 | 11,14 |
| männlich | stehen Schaumstoff 1 Bein | 35 | 39 | 2,94 | 14,71 | 2,81 | 14,03 | 2,02 | 10,11 | 2,57 | 12,83 |
| männlich | stehen Schaumstoff 1 Bein | 40 | 44 | 3,55 | 17,75 | 3,45 | 17,23 | 3,68 | 18,42 | 4,93 | 24,64 |
| männlich | stehen Schaumstoff 1 Bein | 45 | 49 | 3,86 | 19,32 | 3,50 | 17,49 | 4,05 | 20,25 | 3,76 | 18,82 |
| männlich | stehen Schaumstoff 1 Bein | 50 | 54 | 6,43 | 32,14 | 6,43 | 32,14 | 6,45 | 32,25 | 5,87 | 29,34 |
| männlich | stehen Schaumstoff 1 Bein | 55 | 60 | 9,01 | 45,05 | 8,64 | 43,19 | 6,70 | 33,48 | 8,53 | 42,64 |

| Geschlecht | Aufgabe | von | bis | von | bis | von | bis | von | bis | von | bis |
|---|---|---|---|---|---|---|---|---|---|---|---|
| männlich | gehen tandem Schaumstoff | 15 | 19 | 5,14 | 25,71 | 5,37 | 26,87 | 6,24 | 31,22 | 6,96 | 34,79 |
| männlich | gehen tandem Schaumstoff | 20 | 24 | 5,80 | 29,01 | 4,30 | 21,48 | 5,99 | 29,94 | 6,84 | 34,22 |
| männlich | gehen tandem Schaumstoff | 25 | 29 | 7,38 | 36,91 | 4,61 | 23,06 | 7,55 | 37,74 | 9,21 | 46,05 |
| männlich | gehen tandem Schaumstoff | 30 | 34 | 8,17 | 40,83 | 5,11 | 25,53 | 6,50 | 32,48 | 9,87 | 49,37 |
| männlich | gehen tandem Schaumstoff | 35 | 39 | 7,67 | 38,35 | 5,90 | 29,50 | 5,96 | 29,80 | 9,39 | 46,93 |
| männlich | gehen tandem Schaumstoff | 40 | 44 | 6,86 | 34,28 | 6,26 | 31,29 | 7,16 | 35,82 | 12,08 | 60,41 |
| männlich | gehen tandem Schaumstoff | 45 | 49 | 8,32 | 41,61 | 6,87 | 34,37 | 8,57 | 42,87 | 12,26 | 61,28 |
| männlich | gehen tandem Schaumstoff | 50 | 54 | 8,35 | 41,75 | 10,57 | 52,86 | 5,19 | 25,97 | 6,85 | 34,23 |
| männlich | gehen tandem Schaumstoff | 55 | 59 | 9,62 | 48,10 | 12,10 | 60,51 | 10,15 | 50,73 | 17,54 | 87,69 |
| männlich | gehen tandem Schaumstoff | 60 | 64 | 7,52 | 37,60 | 7,81 | 39,04 | 7,48 | 37,38 | 10,03 | 50,16 |
| männlich | gehen tandem Schaumstoff | 65 | 69 | 9,02 | 45,10 | 9,85 | 49,24 | 12,49 | 62,44 | 14,27 | 71,36 |
| männlich | gehen tandem Schaumstoff | 70 | 74 | 7,36 | 36,80 | 6,09 | 30,47 | 9,95 | 49,77 | 12,52 | 62,61 |
| männlich | gehen tandem Schaumstoff | 75 | 79 | 8,13 | 40,66 | 7,77 | 38,83 | 11,13 | 55,66 | 11,05 | 55,27 |
| männlich | gehen tandem Schaumstoff | 80 | 84 | 9,45 | 47,25 | 14,03 | 70,16 | 7,78 | 38,90 | 10,44 | 52,20 |
| männlich | gehen tandem Schaumstoff | 85 | 90 | 9,03 | 45,17 | 12,81 | 64,07 | 11,31 | 56,54 | 9,53 | 47,64 |
| männlich | gehen Kopfkreisen | 15 | 19 | 4,81 | 24,06 | 5,42 | 27,09 | 6,20 | 30,99 | 4,84 | 24,18 |
| männlich | gehen Kopfkreisen | 20 | 24 | 6,54 | 32,72 | 6,07 | 30,37 | 7,61 | 38,05 | 7,00 | 35,00 |
| männlich | gehen Kopfkreisen | 25 | 29 | 7,19 | 35,93 | 6,38 | 31,89 | 7,71 | 38,57 | 9,20 | 46,00 |
| männlich | gehen Kopfkreisen | 30 | 34 | 8,30 | 41,48 | 6,66 | 33,28 | 8,36 | 41,81 | 9,19 | 45,97 |
| männlich | gehen Kopfkreisen | 35 | 39 | 6,69 | 33,43 | 6,18 | 30,90 | 6,84 | 34,19 | 9,74 | 48,69 |
| männlich | gehen Kopfkreisen | 40 | 44 | 8,98 | 44,91 | 5,87 | 29,36 | 6,83 | 34,16 | 10,76 | 53,82 |
| männlich | gehen Kopfkreisen | 45 | 49 | 7,97 | 39,86 | 5,89 | 29,47 | 9,36 | 46,82 | 9,41 | 47,06 |
| männlich | gehen Kopfkreisen | 50 | 54 | 6,64 | 33,21 | 7,60 | 37,98 | 9,07 | 45,37 | 11,04 | 55,18 |
| männlich | gehen Kopfkreisen | 55 | 59 | 8,93 | 44,66 | 9,54 | 47,70 | 8,78 | 43,92 | 12,66 | 63,31 |
| männlich | gehen Kopfkreisen | 60 | 64 | 6,49 | 32,43 | 6,25 | 31,24 | 6,77 | 33,83 | 8,78 | 43,91 |
| männlich | gehen Kopfkreisen | 65 | 69 | 5,61 | 28,07 | 6,60 | 33,01 | 8,70 | 43,49 | 10,36 | 51,81 |
| männlich | gehen Kopfkreisen | 70 | 74 | 6,20 | 30,99 | 8,57 | 42,84 | 7,98 | 39,91 | 8,23 | 41,15 |
| männlich | gehen Kopfkreisen | 75 | 79 | 5,61 | 28,07 | 5,82 | 29,11 | 9,03 | 45,17 | 7,98 | 39,89 |
| männlich | gehen Kopfkreisen | 80 | 84 | 6,31 | 31,54 | 6,75 | 33,74 | 7,45 | 37,25 | 7,83 | 39,17 |
| männlich | gehen Kopfkreisen | 85 | 90 | 7,08 | 35,39 | 8,43 | 42,14 | 7,93 | 39,67 | 8,10 | 40,50 |
| | | | | | | | | | | | |

| | | Alter | | rechts | | links | | vorwärts | | rückwärts | |
|---|---|---|---|---|---|---|---|---|---|---|---|
| Geschlecht | Aufgabe | von | bis | von | bis | von | bis | von | bis | von | bis |
| männlich | gehen Kopfnicken | 15 | 19 | 5,12 | 25,61 | 6,34 | 31,71 | 6,66 | 33,30 | 8,34 | 41,70 |
| männlich | gehen Kopfnicken | 20 | 24 | 6,91 | 34,53 | 6,72 | 33,59 | 8,20 | 41,02 | 9,24 | 46,19 |
| männlich | gehen Kopfnicken | 25 | 29 | 7,70 | 38,52 | 5,62 | 28,08 | 6,49 | 32,46 | 10,16 | 50,78 |
| männlich | gehen Kopfnicken | 30 | 34 | 6,10 | 30,50 | 5,67 | 28,34 | 7,26 | 36,32 | 8,89 | 44,43 |
| männlich | gehen Kopfnicken | 35 | 39 | 7,06 | 35,28 | 5,76 | 28,80 | 6,98 | 34,90 | 9,16 | 45,79 |
| männlich | gehen Kopfnicken | 40 | 44 | 9,39 | 46,94 | 6,33 | 31,64 | 9,29 | 46,46 | 11,19 | 55,93 |
| männlich | gehen Kopfnicken | 45 | 49 | 7,04 | 35,19 | 4,95 | 24,74 | 7,09 | 35,43 | 9,80 | 48,98 |
| männlich | gehen Kopfnicken | 50 | 54 | 6,64 | 33,18 | 8,28 | 41,42 | 4,82 | 24,08 | 5,92 | 29,59 |
| männlich | gehen Kopfnicken | 55 | 59 | 8,23 | 41,15 | 8,27 | 41,33 | 7,77 | 38,87 | 11,46 | 57,30 |
| männlich | gehen Kopfnicken | 60 | 64 | 6,01 | 30,07 | 4,87 | 24,33 | 5,81 | 29,04 | 7,51 | 37,56 |
| männlich | gehen Kopfnicken | 65 | 69 | 6,27 | 31,35 | 5,47 | 27,36 | 7,12 | 35,60 | 10,31 | 51,53 |
| männlich | gehen Kopfnicken | 70 | 74 | 5,72 | 28,59 | 4,36 | 21,78 | 6,63 | 33,15 | 8,27 | 41,36 |
| männlich | gehen Kopfnicken | 75 | 79 | 5,28 | 26,41 | 5,34 | 26,72 | 7,31 | 36,57 | 8,12 | 40,58 |
| männlich | gehen Kopfnicken | 80 | 84 | 5,98 | 29,90 | 6,70 | 33,51 | 4,04 | 20,22 | 5,30 | 26,49 |
| männlich | gehen Kopfnicken | 85 | 90 | 5,41 | 27,04 | 6,25 | 31,26 | 4,89 | 24,45 | 5,24 | 26,21 |
| männlich | gehen AG | 15 | 19 | 4,73 | 23,63 | 5,37 | 26,87 | 7,06 | 35,28 | 7,90 | 39,51 |
| männlich | gehen AG | 20 | 24 | 5,61 | 28,04 | 4,77 | 23,87 | 6,01 | 30,04 | 6,35 | 31,76 |
| männlich | gehen AG | 25 | 29 | 8,00 | 39,98 | 5,26 | 26,28 | 6,21 | 31,05 | 9,02 | 45,12 |
| männlich | gehen AG | 30 | 34 | 7,24 | 36,21 | 4,78 | 23,89 | 6,57 | 32,86 | 8,33 | 41,63 |
| männlich | gehen AG | 35 | 39 | 6,40 | 32,02 | 4,32 | 21,59 | 5,75 | 28,75 | 8,33 | 41,66 |
| männlich | gehen AG | 40 | 44 | 8,47 | 42,37 | 4,50 | 22,50 | 7,69 | 38,46 | 11,56 | 57,78 |
| männlich | gehen AG | 45 | 49 | 7,06 | 35,28 | 5,26 | 26,32 | 7,44 | 37,19 | 9,77 | 48,87 |
| männlich | gehen AG | 50 | 54 | 6,92 | 34,61 | 8,31 | 41,53 | 5,37 | 26,83 | 6,06 | 30,30 |
| männlich | gehen AG | 55 | 59 | 7,29 | 36,46 | 8,69 | 43,45 | 7,14 | 35,72 | 10,96 | 54,81 |
| männlich | gehen AG | 60 | 64 | 5,92 | 29,61 | 4,35 | 21,77 | 5,20 | 26,00 | 7,92 | 39,61 |
| männlich | gehen AG | 65 | 69 | 5,62 | 28,10 | 4,81 | 24,05 | 7,75 | 38,76 | 9,08 | 45,38 |
| männlich | gehen AG | 70 | 74 | 5,10 | 25,52 | 3,76 | 18,81 | 5,76 | 28,82 | 7,62 | 38,09 |
| männlich | gehen AG | 75 | 79 | 4,42 | 22,11 | 4,47 | 22,33 | 6,86 | 34,28 | 7,27 | 36,33 |
| männlich | gehen AG | 80 | 84 | 5,97 | 29,84 | 6,25 | 31,27 | 7,35 | 36,76 | 7,81 | 39,07 |
| männlich | gehen AG | 85 | 90 | 5,91 | 29,57 | 6,95 | 34,73 | 6,50 | 32,48 | 8,27 | 41,37 |
| männlich | gehen über Hindernisse | 15 | 19 | 7,65 | 38,27 | 10,61 | 53,04 | 11,60 | 58,02 | 10,43 | 52,14 |
| männlich | gehen über Hindernisse | 20 | 24 | 8,22 | 41,12 | 12,07 | 60,34 | 12,21 | 61,04 | 11,03 | 55,17 |
| männlich | gehen über Hindernisse | 25 | 29 | 7,99 | 39,94 | 8,20 | 40,98 | 11,02 | 55,12 | 12,64 | 63,19 |
| männlich | gehen über Hindernisse | 30 | 34 | 9,72 | 48,62 | 9,46 | 47,29 | 14,27 | 71,37 | 15,31 | 76,56 |
| männlich | gehen über Hindernisse | 35 | 39 | 8,69 | 43,45 | 9,75 | 48,75 | 13,96 | 69,79 | 13,64 | 68,20 |
| männlich | gehen über Hindernisse | 40 | 44 | 13,38 | 66,90 | 13,73 | 68,67 | 13,49 | 67,43 | 17,12 | 85,61 |
| männlich | gehen über Hindernisse | 45 | 49 | 10,70 | 53,48 | 11,80 | 58,98 | 14,27 | 71,36 | 15,50 | 77,48 |
| männlich | gehen über Hindernisse | 50 | 54 | 9,24 | 46,20 | 14,42 | 72,12 | 8,80 | 44,02 | 9,54 | 47,71 |
| männlich | gehen über Hindernisse | 55 | 59 | 15,69 | 78,43 | 16,26 | 81,31 | 22,71 | 113,57 | 20,75 | 103,74 |
| männlich | gehen über Hindernisse | 60 | 64 | 10,31 | 51,54 | 13,12 | 65,58 | 13,32 | 66,60 | 15,00 | 74,99 |
| männlich | gehen über Hindernisse | 65 | 69 | 12,41 | 62,06 | 14,27 | 71,36 | 12,96 | 64,81 | 15,71 | 78,54 |
| männlich | gehen über Hindernisse | 70 | 74 | 13,45 | 67,24 | 14,81 | 74,04 | 11,25 | 56,24 | 11,81 | 59,03 |
| männlich | gehen über Hindernisse | 75 | 79 | 13,34 | 66,69 | 16,16 | 80,81 | 16,94 | 84,69 | 19,03 | 95,14 |
| männlich | gehen über Hindernisse | 80 | 84 | 12,00 | 60,00 | 12,05 | 60,23 | 14,37 | 71,87 | 12,15 | 60,77 |
| männlich | gehen über Hindernisse | 85 | 90 | 10,55 | 52,74 | 10,64 | 53,19 | 12,99 | 64,95 | 13,18 | 65,88 |
| männlich | gehen AO | 15 | 19 | 5,21 | 26,04 | 4,59 | 22,97 | 7,89 | 39,45 | 5,14 | 25,71 |
| männlich | gehen AO | 20 | 24 | 6,41 | 32,03 | 5,39 | 26,95 | 7,47 | 37,36 | 8,66 | 43,30 |
| männlich | gehen AO | 25 | 29 | 8,85 | 44,23 | 6,00 | 29,99 | 7,48 | 37,40 | 9,78 | 48,88 |
| männlich | gehen AO | 30 | 34 | 8,76 | 43,79 | 5,67 | 28,33 | 7,50 | 37,48 | 10,00 | 49,98 |
| männlich | gehen AO | 35 | 39 | 7,55 | 37,73 | 5,62 | 28,09 | 6,14 | 30,68 | 9,21 | 46,05 |
| männlich | gehen AO | 40 | 44 | 10,96 | 54,82 | 6,90 | 34,50 | 10,15 | 50,73 | 13,47 | 67,37 |
| männlich | gehen AO | 45 | 49 | 8,28 | 41,38 | 5,75 | 28,73 | 8,73 | 43,64 | 10,05 | 50,24 |
| männlich | gehen AO | 50 | 54 | 7,41 | 37,03 | 8,47 | 42,35 | 4,77 | 23,86 | 6,18 | 30,90 |
| männlich | gehen AO | 55 | 59 | 8,49 | 42,46 | 7,83 | 39,13 | 8,23 | 41,14 | 12,59 | 62,94 |
| männlich | gehen AO | 60 | 64 | 6,08 | 30,40 | 4,12 | 20,62 | 5,40 | 27,01 | 7,62 | 38,12 |
| männlich | gehen AO | 65 | 69 | 5,97 | 29,83 | 4,31 | 21,56 | 7,04 | 35,22 | 9,18 | 45,88 |
| männlich | gehen AO | 70 | 74 | 6,40 | 32,01 | 4,58 | 22,92 | 6,45 | 32,24 | 7,98 | 39,92 |
| männlich | gehen AO | 75 | 79 | 4,94 | 24,70 | 3,92 | 19,59 | 6,89 | 34,45 | 8,13 | 40,65 |
| männlich | gehen AO | 80 | 84 | 5,75 | 28,73 | 5,88 | 29,40 | 7,13 | 35,66 | 8,19 | 40,94 |
| männlich | gehen AO | 85 | 90 | 4,81 | 24,07 | 5,94 | 29,72 | 8,10 | 40,49 | 7,93 | 39,67 |
| | | | | | | | | | | | |

| | | Alter | | rechts | | links | | vorwärts | | rückwärts | |
|---|---|---|---|---|---|---|---|---|---|---|---|
| Geschlecht | Aufgabe | von | bis | von | bis | | von bis von | | | bis von | bis |
| männlich | hinsetzen | 20 | 24 | 14,40 | 71,98 | 9,96 | 49,80 | 7,86 | 39,32 | 12,88 | 64,40 |
| männlich | hinsetzen | 25 | 29 | 13,57 | 67,86 | 8,50 | 42,49 | 7,65 | 38,23 | 11,63 | 58,16 |
| männlich | hinsetzen | 30 | 34 | 10,32 | 51,59 | 9,56 | 47,78 | 11,53 | 57,63 | 15,03 | 75,13 |
| männlich | hinsetzen | 35 | 39 | 14,75 | 73,77 | 12,12 | 60,61 | 11,81 | 59,07 | 15,33 | 76,64 |
| männlich | hinsetzen | 40 | 44 | 11,75 | 58,77 | 12,37 | 61,86 | 6,61 | 33,04 | 13,17 | 65,83 |
| männlich | hinsetzen | 45 | 49 | 14,80 | 74,02 | 9,28 | 46,42 | 10,57 | 52,86 | 15,33 | 76,67 |
| männlich | hinsetzen | 50 | 54 | 11,39 | 56,93 | 15,15 | 75,75 | 10,86 | 54,30 | 15,02 | 75,12 |
| männlich | hinsetzen | 55 | 59 | 10,34 | 51,69 | 13,10 | 65,49 | 10,00 | 50,00 | 19,39 | 96,93 |
| männlich | hinsetzen | 60 | 64 | 11,21 | 56,06 | 11,52 | 57,58 | 10,40 | 52,01 | 13,29 | 66,47 |
| männlich | hinsetzen | 65 | 69 | 10,89 | 54,43 | 12,17 | 60,83 | 13,50 | 67,49 | 15,31 | 76,56 |
| männlich | hinsetzen | 70 | 74 | 14,25 | 71,27 | 11,48 | 57,40 | 10,58 | 52,89 | 14,31 | 71,56 |
| männlich | hinsetzen | 75 | 79 | 12,13 | 60,64 | 11,00 | 54,99 | 10,45 | 52,27 | 14,73 | 73,66 |
| männlich | hinsetzen | 80 | 84 | 8,12 | 40,61 | 12,81 | 64,07 | 11,73 | 58,63 | 16,66 | 83,30 |
| männlich | hinsetzen | 85 | 90 | 6,98 | 34,89 | 11,85 | 59,24 | 8,65 | 43,25 | 12,57 | 62,86 |
| männlich | aufstehen | 20 | 24 | 9,64 | 48,20 | 16,58 | 82,91 | 18,25 | 91,27 | 11,52 | 57,61 |
| männlich | aufstehen | 25 | 29 | 9,38 | 46,92 | 16,37 | 81,84 | 13,84 | 69,18 | 6,30 | 31,50 |
| männlich | aufstehen | 30 | 34 | 7,13 | 35,66 | 12,57 | 62,83 | 18,54 | 92,68 | 8,14 | 40,69 |
| männlich | aufstehen | 35 | 39 | 9,29 | 46,43 | 15,32 | 76,61 | 16,93 | 84,65 | 8,30 | 41,48 |
| männlich | aufstehen | 40 | 44 | 10,79 | 53,94 | 12,20 | 60,98 | 10,98 | 54,90 | 7,56 | 37,82 |
| männlich | aufstehen | 45 | 49 | 8,50 | 42,48 | 16,84 | 84,22 | 15,35 | 76,73 | 7,47 | 37,36 |
| männlich | aufstehen | 50 | 54 | 17,01 | 85,04 | 11,99 | 59,93 | 19,25 | 96,26 | 10,45 | 52,27 |
| männlich | aufstehen | 55 | 59 | 11,76 | 58,80 | 11,81 | 59,05 | 18,41 | 92,04 | 9,67 | 48,36 |
| männlich | aufstehen | 60 | 64 | 11,42 | 57,12 | 12,60 | 62,99 | 13,10 | 65,51 | 9,11 | 45,55 |
| männlich | aufstehen | 65 | 69 | 11,10 | 55,50 | 13,29 | 66,44 | 17,73 | 88,63 | 14,29 | 71,45 |
| männlich | aufstehen | 70 | 74 | 11,81 | 59,05 | 18,28 | 91,42 | 18,18 | 90,89 | 11,20 | 56,01 |
| männlich | aufstehen | 75 | 79 | 9,78 | 48,92 | 15,52 | 77,59 | 15,50 | 77,48 | 11,08 | 55,40 |
| männlich | aufstehen | 80 | 84 | 14,67 | 73,35 | 11,79 | 58,96 | 18,80 | 94,01 | 13,35 | 66,76 |
| männlich | aufstehen | 85 | 90 | 13,76 | 68,79 | 13,42 | 67,10 | 15,25 | 76,26 | 13,30 | 66,48 |

| Geschleht | Aufgabe | | von bis | von | bis | von | bis | von | bis | von | bis |
|---|---|---|---|---|---|---|---|---|---|---|---|
| weiblich | stehen AO | 15 | 19 | 0,48 | 2,39 | 0,16 | 0,81 | 0,50 | 2,49 | 0,48 | 2,39 |
| weiblich | stehen AO | 20 | 24 | 0,58 | 2,88 | 0,19 | 0,96 | 0,30 | 1,49 | 0,65 | 3,23 |
| weiblich | stehen AO | 25 | 29 | 0,50 | 2,50 | 0,17 | 0,87 | 0,28 | 1,38 | 0,65 | 3,25 |
| weiblich | stehen AO | 30 | 34 | 0,37 | 1,83 | 0,38 | 1,90 | 0,50 | 2,49 | 0,31 | 1,55 |
| weiblich | stehen AO | 35 | 39 | 0,68 | 3,41 | 0,53 | 2,64 | 0,65 | 3,26 | 1,02 | 5,08 |
| weiblich | stehen AO | 40 | 44 | 0,45 | 2,27 | 0,24 | 1,20 | 0,32 | 1,60 | 0,67 | 3,33 |
| weiblich | stehen AO | 45 | 49 | 0,58 | 2,91 | 0,10 | 0,50 | 0,28 | 1,41 | 0,71 | 3,53 |
| weiblich | stehen AO | 50 | 54 | 0,65 | 3,24 | 0,27 | 1,33 | 0,52 | 2,59 | 0,96 | 4,80 |
| weiblich | stehen AO | 55 | 59 | 0,42 | 2,08 | 1,02 | 5,11 | 0,96 | 4,78 | 1,03 | 5,16 |
| weiblich | stehen AO | 60 | 64 | 0,61 | 3,03 | 0,63 | 3,15 | 1,14 | 5,69 | 0,96 | 4,78 |
| weiblich | stehen AO | 65 | 69 | 0,79 | 3,97 | 0,42 | 2,09 | 0,49 | 2,43 | 0,77 | 3,87 |
| weiblich | stehen AO | 70 | 74 | 0,55 | 2,73 | 0,51 | 2,56 | 0,66 | 3,29 | 0,76 | 3,79 |
| weiblich | stehen AO | 75 | 79 | 0,58 | 2,92 | 0,43 | 2,14 | 0,55 | 2,74 | 0,88 | 4,40 |
| weiblich | stehen AO | 80 | 84 | 0,48 | 2,42 | 0,33 | 1,65 | 0,70 | 3,48 | 0,72 | 3,59 |
| weiblich | stehen AO | 85 | 90 | 0,64 | 3,18 | 0,35 | 1,76 | 0,78 | 3,92 | 0,68 | 3,38 |
| weiblich | stehen AG | 15 | 19 | 0,43 | 2,16 | 0,43 | 2,16 | 0,45 | 2,27 | 0,75 | 3,77 |
| weiblich | stehen AG | 20 | 24 | 0,51 | 2,56 | 0,21 | 1,07 | 0,47 | 2,37 | 0,80 | 4,00 |
| weiblich | stehen AG | 25 | 29 | 0,75 | 3,76 | 0,23 | 1,14 | 0,41 | 2,04 | 1,14 | 5,72 |
| weiblich | stehen AG | 30 | 34 | 0,82 | 4,08 | 0,59 | 2,95 | 0,62 | 3,11 | 0,42 | 2,08 |
| weiblich | stehen AG | 35 | 39 | 0,61 | 3,06 | 0,39 | 1,95 | 0,28 | 1,39 | 0,73 | 3,67 |
| weiblich | stehen AG | 40 | 44 | 0,48 | 2,39 | 0,26 | 1,31 | 0,49 | 2,43 | 0,84 | 4,21 |
| weiblich | stehen AG | 45 | 49 | 0,64 | 3,20 | 0,08 | 0,38 | 0,24 | 1,18 | 0,84 | 4,18 |
| weiblich | stehen AG | 50 | 54 | 0,54 | 2,70 | 0,20 | 1,02 | 0,34 | 1,69 | 0,67 | 3,35 |
| weiblich | stehen AG | 55 | 59 | 0,44 | 2,19 | 0,33 | 1,65 | 0,44 | 2,20 | 0,50 | 2,52 |
| weiblich | stehen AG | 60 | 64 | 0,59 | 2,93 | 0,40 | 1,98 | 0,49 | 2,46 | 0,64 | 3,18 |
| weiblich | stehen AG | 65 | 69 | 0,48 | 2,39 | 0,36 | 1,79 | 0,41 | 2,03 | 0,56 | 2,81 |
| weiblich | stehen AG | 70 | 74 | 0,58 | 2,88 | 0,54 | 2,69 | 0,72 | 3,60 | 0,79 | 3,96 |
| weiblich | stehen AG | 75 | 79 | 0,46 | 2,31 | 0,37 | 1,86 | 0,43 | 2,13 | 0,60 | 2,98 |
| weiblich | stehen AG | 80 | 84 | 0,55 | 2,75 | 0,35 | 1,76 | 0,72 | 3,59 | 0,70 | 3,48 |
| weiblich | stehen AG | 85 | 90 | 0,57 | 2,85 | 0,33 | 1,65 | 0,83 | 4,14 | 0,70 | 3,48 |
| | | | | | | | | | | | |

| | | Alter | | rechts | | links | | vorwärts | | rückwärts | |
|---|---|---|---|---|---|---|---|---|---|---|---|
| Geschlecht | Aufgabe | von | bis | von | bis | von | bis | von | bis | von | bis |
| weiblich | stehen 1 Bein AO | 15 | 19 | 1,60 | 8,01 | 1,06 | 5,31 | 1,52 | 7,58 | 1,52 | 7,62 |
| weiblich | stehen 1 Bein AO | 20 | 24 | 1,63 | 8,16 | 1,16 | 5,80 | 1,95 | 9,73 | 1,99 | 9,94 |
| weiblich | stehen 1 Bein AO | 25 | 29 | 1,30 | 6,51 | 0,86 | 4,31 | 1,01 | 5,05 | 1,50 | 7,52 |
| weiblich | stehen 1 Bein AO | 30 | 34 | 1,59 | 7,95 | 2,44 | 12,18 | 1,41 | 7,06 | 1,86 | 9,29 |
| weiblich | stehen 1 Bein AO | 35 | 39 | 2,79 | 13,97 | 2,99 | 14,95 | 4,12 | 20,58 | 3,28 | 16,42 |
| weiblich | stehen 1 Bein AO | 40 | 44 | 2,10 | 10,52 | 2,15 | 10,74 | 2,26 | 11,28 | 1,80 | 8,99 |
| weiblich | stehen 1 Bein AO | 45 | 49 | 3,64 | 18,22 | 1,79 | 8,95 | 2,78 | 13,91 | 2,43 | 12,17 |
| weiblich | stehen 1 Bein AO | 50 | 54 | 1,97 | 9,87 | 1,74 | 8,71 | 2,19 | 10,97 | 2,27 | 11,33 |
| weiblich | stehen 1 Bein AO | 55 | 59 | 4,76 | 23,78 | 3,54 | 17,70 | 4,79 | 23,95 | 3,43 | 17,13 |
| weiblich | stehen 1 Bein AO | 60 | 64 | 1,43 | 7,14 | 1,29 | 6,43 | 1,94 | 9,71 | 1,77 | 8,86 |
| weiblich | stehen 1 Bein AO | 65 | 69 | 2,67 | 13,36 | 2,72 | 13,61 | 3,50 | 17,52 | 3,40 | 16,98 |
| weiblich | stehen 1 Bein AO | 70 | 74 | 3,58 | 17,88 | 3,74 | 18,70 | 4,40 | 21,98 | 4,39 | 21,94 |
| weiblich | stehen 1 Bein AO | 75 | 79 | 4,98 | 24,91 | 5,83 | 29,14 | 7,94 | 39,68 | 6,02 | 30,10 |
| weiblich | stehen 1 Bein AO | 80 | 84 | 3,23 | 16,16 | 2,80 | 14,01 | 3,13 | 15,66 | 2,53 | 12,64 |
| weiblich | stehen 1 Bein AO | 85 | 90 | 6,67 | 33,35 | 4,37 | 21,87 | 5,23 | 26,16 | 3,15 | 15,77 |
| weiblich | stehen 1 Bein AG | 15 | 19 | 5,69 | 28,46 | 6,91 | 34,56 | 8,79 | 43,95 | 6,00 | 29,99 |
| weiblich | stehen 1 Bein AG | 20 | 24 | 5,52 | 27,60 | 6,41 | 32,05 | 7,42 | 37,09 | 6,36 | 31,80 |
| weiblich | stehen 1 Bein AG | 25 | 29 | 4,27 | 21,34 | 3,47 | 17,33 | 6,42 | 32,09 | 5,50 | 27,51 |
| weiblich | stehen 1 Bein AG | 30 | 34 | 3,70 | 18,49 | 4,39 | 21,94 | 4,80 | 24,02 | 3,69 | 18,43 |
| weiblich | stehen 1 Bein AG | 35 | 39 | 4,62 | 23,10 | 4,75 | 23,74 | 5,56 | 27,79 | 5,57 | 27,85 |
| weiblich | stehen 1 Bein AG | 40 | 44 | 8,13 | 40,67 | 8,40 | 42,00 | 8,44 | 42,21 | 9,76 | 48,81 |
| weiblich | stehen 1 Bein AG | 45 | 49 | 5,61 | 28,06 | 5,06 | 25,28 | 5,36 | 26,82 | 6,66 | 33,29 |
| weiblich | stehen 1 Bein AG | 50 | 54 | 7,25 | 36,24 | 6,29 | 31,43 | 10,10 | 50,49 | 8,95 | 44,74 |
| weiblich | stehen 1 Bein AG | 55 | 60 | 4,52 | 22,59 | 5,82 | 29,09 | 6,89 | 34,45 | 3,97 | 19,86 |
| weiblich | gehen tandem | 15 | 19 | 6,21 | 31,05 | 5,51 | 27,53 | 6,38 | 31,92 | 5,84 | 29,18 |
| weiblich | gehen tandem | 20 | 24 | 6,78 | 33,91 | 3,97 | 19,85 | 7,63 | 38,14 | 7,09 | 35,43 |
| weiblich | gehen tandem | 25 | 29 | 6,42 | 32,11 | 3,75 | 18,74 | 5,28 | 26,40 | 7,20 | 36,01 |
| weiblich | gehen tandem | 30 | 34 | 6,76 | 33,78 | 7,32 | 36,61 | 6,21 | 31,06 | 7,17 | 35,83 |
| weiblich | gehen tandem | 35 | 39 | 6,04 | 30,22 | 4,01 | 20,05 | 4,70 | 23,48 | 8,19 | 40,94 |
| weiblich | gehen tandem | 40 | 44 | 5,38 | 26,89 | 3,80 | 18,98 | 5,28 | 26,39 | 8,06 | 40,30 |
| weiblich | gehen tandem | 45 | 49 | 6,24 | 31,18 | 5,04 | 25,22 | 5,80 | 29,00 | 8,73 | 43,66 |
| weiblich | gehen tandem | 50 | 54 | 5,74 | 28,68 | 4,65 | 23,25 | 5,25 | 26,26 | 7,53 | 37,66 |
| weiblich | gehen tandem | 55 | 59 | 7,65 | 38,23 | 6,16 | 30,82 | 6,59 | 32,96 | 8,07 | 40,37 |
| weiblich | gehen tandem | 60 | 64 | 5,56 | 27,79 | 4,75 | 23,74 | 5,25 | 26,26 | 6,49 | 32,43 |
| weiblich | gehen tandem | 65 | 69 | 6,37 | 31,85 | 5,20 | 26,02 | 5,85 | 29,25 | 7,51 | 37,54 |
| weiblich | gehen tandem | 70 | 74 | 6,59 | 32,93 | 5,34 | 26,70 | 7,14 | 35,70 | 8,17 | 40,85 |
| weiblich | gehen tandem | 75 | 79 | 7,08 | 35,39 | 6,69 | 33,46 | 7,76 | 38,82 | 9,15 | 45,77 |
| weiblich | gehen tandem | 80 | 84 | 9,53 | 47,63 | 6,02 | 30,11 | 8,22 | 41,10 | 6,72 | 33,61 |
| weiblich | gehen tandem | 85 | 90 | 8,55 | 42,75 | 5,23 | 26,16 | 7,34 | 36,71 | 8,56 | 42,80 |
| weiblich | stehen Schaumstoff AO | 15 | 19 | 0,84 | 4,22 | 0,38 | 1,88 | 0,56 | 2,82 | 0,91 | 4,55 |
| weiblich | stehen Schaumstoff AO | 20 | 24 | 0,66 | 3,30 | 0,42 | 2,10 | 0,54 | 2,68 | 1,31 | 6,54 |
| weiblich | stehen Schaumstoff AO | 25 | 29 | 0,72 | 3,60 | 0,47 | 2,35 | 0,57 | 2,83 | 0,93 | 4,64 |
| weiblich | stehen Schaumstoff AO | 30 | 34 | 0,99 | 4,96 | 1,12 | 5,59 | 0,80 | 3,98 | 1,08 | 5,42 |
| weiblich | stehen Schaumstoff AO | 35 | 39 | 1,37 | 6,85 | 1,58 | 7,92 | 1,30 | 6,52 | 1,47 | 7,37 |
| weiblich | stehen Schaumstoff AO | 40 | 44 | 0,80 | 3,98 | 0,41 | 2,06 | 0,69 | 3,47 | 1,05 | 5,25 |
| weiblich | stehen Schaumstoff AO | 45 | 49 | 1,05 | 5,26 | 0,41 | 2,04 | 0,44 | 2,22 | 1,11 | 5,57 |
| weiblich | stehen Schaumstoff AO | 50 | 54 | 1,05 | 5,26 | 1,67 | 8,36 | 0,65 | 3,24 | 1,63 | 8,14 |
| weiblich | stehen Schaumstoff AO | 55 | 59 | 1,43 | 7,13 | 1,62 | 8,11 | 2,33 | 11,65 | 2,13 | 10,65 |
| weiblich | stehen Schaumstoff AO | 60 | 64 | 0,98 | 4,92 | 0,71 | 3,56 | 0,93 | 4,63 | 1,17 | 5,84 |
| weiblich | stehen Schaumstoff AO | 65 | 69 | 0,99 | 4,94 | 0,71 | 3,53 | 0,73 | 3,66 | 1,20 | 6,02 |
| weiblich | stehen Schaumstoff AO | 70 | 74 | 0,93 | 4,63 | 0,93 | 4,65 | 0,94 | 4,70 | 1,26 | 6,29 |
| weiblich | stehen Schaumstoff AO | 75 | 79 | 0,98 | 4,90 | 0,68 | 3,40 | 0,92 | 4,61 | 1,21 | 6,05 |
| weiblich | stehen Schaumstoff AO | 80 | 84 | 1,37 | 6,87 | 0,88 | 4,40 | 1,28 | 6,38 | 1,08 | 5,39 |
| weiblich | stehen Schaumstoff AO | 85 | 90 | 1,16 | 5,82 | 0,74 | 3,68 | 1,06 | 5,28 | 1,19 | 5,96 |
| | | | | | | | | | | | |

| | | Alter | | rechts | | links | | vorwärts | | rückwärts | |
|---|---|---|---|---|---|---|---|---|---|---|---|
| Geschlecht | Aufgabe | von | bis | von | bis | von | bis | von | bis | von | bis |
| weiblich | stehen Schaumstoff AG | 15 | 19 | 1,30 | 6,48 | 0,64 | 3,18 | 0,92 | 4,62 | 0,83 | 4,17 |
| weiblich | stehen Schaumstoff AG | 20 | 24 | 0,81 | 4,07 | 0,67 | 3,36 | 1,11 | 5,57 | 1,08 | 5,38 |
| weiblich | stehen Schaumstoff AG | 25 | 29 | 0,87 | 4,36 | 0,53 | 2,67 | 0,97 | 4,85 | 1,34 | 6,71 |
| weiblich | stehen Schaumstoff AG | 30 | 34 | 0,92 | 4,60 | 1,36 | 6,82 | 0,95 | 4,74 | 0,71 | 3,57 |
| weiblich | stehen Schaumstoff AG | 35 | 39 | 0,91 | 4,56 | 1,85 | 9,24 | 0,90 | 4,50 | 1,30 | 6,49 |
| weiblich | stehen Schaumstoff AG | 40 | 44 | 1,42 | 7,11 | 1,04 | 5,19 | 1,73 | 8,65 | 1,62 | 8,11 |
| weiblich | stehen Schaumstoff AG | 45 | 49 | 1,21 | 6,06 | 0,31 | 1,56 | 0,70 | 3,52 | 1,19 | 5,94 |
| weiblich | stehen Schaumstoff AG | 50 | 54 | 1,86 | 9,31 | 0,99 | 4,97 | 1,29 | 6,45 | 1,79 | 8,97 |
| weiblich | stehen Schaumstoff AG | 55 | 59 | 0,80 | 4,01 | 0,77 | 3,85 | 0,92 | 4,62 | 1,17 | 5,87 |
| weiblich | stehen Schaumstoff AG | 60 | 64 | 1,36 | 6,78 | 1,17 | 5,84 | 1,33 | 6,64 | 1,58 | 7,91 |
| weiblich | stehen Schaumstoff AG | 65 | 69 | 1,86 | 9,28 | 1,38 | 6,91 | 1,83 | 9,16 | 2,36 | 11,82 |
| weiblich | stehen Schaumstoff AG | 70 | 74 | 1,29 | 6,43 | 1,14 | 5,72 | 1,27 | 6,37 | 1,56 | 7,79 |
| weiblich | stehen Schaumstoff AG | 75 | 79 | 1,92 | 9,58 | 1,46 | 7,29 | 1,66 | 8,29 | 1,92 | 9,60 |
| weiblich | stehen Schaumstoff AG | 80 | 84 | 2,09 | 10,44 | 1,67 | 8,36 | 2,20 | 11,00 | 1,85 | 9,23 |
| weiblich | stehen Schaumstoff AG | 85 | 90 | 1,54 | 7,70 | 1,11 | 5,55 | 1,61 | 8,03 | 1,84 | 9,20 |
| weiblich | stehen Schaumstoff 1 Bein | 15 | 19 | 2,24 | 11,21 | 2,95 | 14,73 | 2,02 | 10,11 | 2,37 | 11,87 |
| weiblich | stehen Schaumstoff 1 Bein | 20 | 24 | 1,95 | 9,74 | 2,98 | 14,90 | 2,22 | 11,11 | 2,74 | 13,72 |
| weiblich | stehen Schaumstoff 1 Bein | 25 | 29 | 2,92 | 14,59 | 2,77 | 13,84 | 3,14 | 15,72 | 2,81 | 14,04 |
| weiblich | stehen Schaumstoff 1 Bein | 30 | 34 | 5,07 | 25,35 | 3,44 | 17,19 | 2,73 | 13,65 | 4,02 | 20,10 |
| weiblich | stehen Schaumstoff 1 Bein | 35 | 39 | 3,62 | 18,10 | 4,45 | 22,25 | 5,19 | 25,94 | 5,92 | 29,60 |
| weiblich | stehen Schaumstoff 1 Bein | 40 | 44 | 4,82 | 24,09 | 5,73 | 28,65 | 5,31 | 26,56 | 4,76 | 23,82 |
| weiblich | stehen Schaumstoff 1 Bein | 45 | 49 | 4,97 | 24,85 | 5,13 | 25,67 | 5,59 | 27,97 | 4,97 | 24,86 |
| weiblich | stehen Schaumstoff 1 Bein | 50 | 54 | 5,12 | 25,61 | 4,54 | 22,69 | 5,88 | 29,39 | 5,18 | 25,89 |
| weiblich | stehen Schaumstoff 1 Bein | 55 | 60 | 5,29 | 26,46 | 4,91 | 24,57 | 5,87 | 29,37 | 4,15 | 20,73 |
| weiblich | gehen tandem Schaumstoff | 15 | 19 | 8,30 | 41,49 | 7,68 | 38,40 | 7,63 | 38,13 | 11,21 | 56,04 |
| weiblich | gehen tandem Schaumstoff | 20 | 24 | 7,05 | 35,23 | 5,20 | 26,00 | 9,62 | 48,10 | 17,54 | 87,70 |
| weiblich | gehen tandem Schaumstoff | 25 | 29 | 10,95 | 54,77 | 7,19 | 35,94 | 7,36 | 36,82 | 11,47 | 57,35 |
| weiblich | gehen tandem Schaumstoff | 30 | 34 | 7,72 | 38,62 | 9,52 | 47,60 | 7,25 | 36,24 | 8,52 | 42,60 |
| weiblich | gehen tandem Schaumstoff | 35 | 39 | 10,53 | 52,64 | 8,58 | 42,90 | 9,95 | 49,76 | 11,55 | 57,73 |
| weiblich | gehen tandem Schaumstoff | 40 | 44 | 7,41 | 37,05 | 6,62 | 33,10 | 5,89 | 29,46 | 8,14 | 40,68 |
| weiblich | gehen tandem Schaumstoff | 45 | 49 | 7,48 | 37,38 | 5,97 | 29,86 | 7,14 | 35,70 | 9,05 | 45,25 |
| weiblich | gehen tandem Schaumstoff | 50 | 54 | 8,12 | 40,60 | 11,40 | 57,01 | 8,51 | 42,57 | 9,49 | 47,43 |
| weiblich | gehen tandem Schaumstoff | 55 | 59 | 7,88 | 39,38 | 7,94 | 39,72 | 7,99 | 39,94 | 9,15 | 45,75 |
| weiblich | gehen tandem Schaumstoff | 60 | 64 | 7,11 | 35,55 | 7,06 | 35,29 | 8,65 | 43,25 | 9,61 | 48,04 |
| weiblich | gehen tandem Schaumstoff | 65 | 69 | 7,30 | 36,49 | 6,82 | 34,11 | 8,89 | 44,45 | 10,00 | 50,01 |
| weiblich | gehen tandem Schaumstoff | 70 | 74 | 8,19 | 40,93 | 8,01 | 40,06 | 10,20 | 51,02 | 11,46 | 57,32 |
| weiblich | gehen tandem Schaumstoff | 75 | 79 | 7,65 | 38,26 | 6,32 | 31,61 | 9,63 | 48,13 | 11,21 | 56,03 |
| weiblich | gehen tandem Schaumstoff | 80 | 84 | 14,29 | 71,43 | 7,61 | 38,03 | 10,31 | 51,54 | 8,06 | 40,28 |
| weiblich | gehen tandem Schaumstoff | 85 | 90 | 8,39 | 41,93 | 8,56 | 42,80 | 7,84 | 39,18 | 8,35 | 41,76 |
| weiblich | gehen Kopfkreisen | 15 | 19 | 8,84 | 44,18 | 8,31 | 41,54 | 10,02 | 50,12 | 8,43 | 42,15 |
| weiblich | gehen Kopfkreisen | 20 | 24 | 8,42 | 42,12 | 6,17 | 30,84 | 9,20 | 45,98 | 9,28 | 46,42 |
| weiblich | gehen Kopfkreisen | 25 | 29 | 8,03 | 40,13 | 6,90 | 34,51 | 6,91 | 34,56 | 9,12 | 45,60 |
| weiblich | gehen Kopfkreisen | 30 | 34 | 6,35 | 31,76 | 6,81 | 34,07 | 7,67 | 38,35 | 9,35 | 46,73 |
| weiblich | gehen Kopfkreisen | 35 | 39 | 7,36 | 36,82 | 6,40 | 32,00 | 8,28 | 41,41 | 11,32 | 56,58 |
| weiblich | gehen Kopfkreisen | 40 | 44 | 7,06 | 35,29 | 5,82 | 29,09 | 8,09 | 40,45 | 7,85 | 39,25 |
| weiblich | gehen Kopfkreisen | 45 | 49 | 7,19 | 35,94 | 5,20 | 26,02 | 8,85 | 44,23 | 8,44 | 42,19 |
| weiblich | gehen Kopfkreisen | 50 | 54 | 6,12 | 30,60 | 5,77 | 28,84 | 8,98 | 44,92 | 8,55 | 42,73 |
| weiblich | gehen Kopfkreisen | 55 | 59 | 7,62 | 38,11 | 6,60 | 33,02 | 7,76 | 38,79 | 7,87 | 39,33 |
| weiblich | gehen Kopfkreisen | 60 | 64 | 5,48 | 27,38 | 5,13 | 25,65 | 6,19 | 30,97 | 7,34 | 36,71 |
| weiblich | gehen Kopfkreisen | 65 | 69 | 5,83 | 29,15 | 5,73 | 28,66 | 6,85 | 34,26 | 8,29 | 41,46 |
| weiblich | gehen Kopfkreisen | 70 | 74 | 6,29 | 31,44 | 5,87 | 29,34 | 7,96 | 39,80 | 7,88 | 39,39 |
| weiblich | gehen Kopfkreisen | 75 | 79 | 6,54 | 32,68 | 7,31 | 36,56 | 8,73 | 43,63 | 9,69 | 48,46 |
| weiblich | gehen Kopfkreisen | 80 | 84 | 7,26 | 36,32 | 8,08 | 40,38 | 10,06 | 50,28 | 8,42 | 42,09 |
| weiblich | gehen Kopfkreisen | 85 | 90 | 7,15 | 35,77 | 8,32 | 41,60 | 9,92 | 49,61 | 8,38 | 41,92 |
| | | | | | | | | | | | |

| | | Alter | | rechts | | links | | vorwärts | | rückwärts | |
|---|---|---|---|---|---|---|---|---|---|---|---|
| Geschlecht | Aufgabe | von | bis | von | bis | von | bis | von | bis | von | bis |
| weiblich | gehen Kopfnicken | 15 | 19 | 8,02 | 40,11 | 5,03 | 25,17 | 7,12 | 35,61 | 9,83 | 49,17 |
| weiblich | gehen Kopfnicken | 20 | 24 | 7,93 | 39,63 | 5,84 | 29,22 | 11,82 | 59,08 | 13,70 | 68,50 |
| weiblich | gehen Kopfnicken | 25 | 29 | 8,24 | 41,22 | 6,67 | 33,36 | 6,21 | 31,06 | 10,02 | 50,12 |
| weiblich | gehen Kopfnicken | 30 | 34 | 7,34 | 36,69 | 7,48 | 37,40 | 8,43 | 42,13 | 8,91 | 44,53 |
| weiblich | gehen Kopfnicken | 35 | 39 | 7,24 | 36,20 | 8,86 | 44,31 | 7,72 | 38,60 | 11,63 | 58,17 |
| weiblich | gehen Kopfnicken | 40 | 44 | 6,31 | 31,56 | 5,76 | 28,81 | 5,88 | 29,41 | 7,07 | 35,36 |
| weiblich | gehen Kopfnicken | 45 | 49 | 8,05 | 40,26 | 4,73 | 23,67 | 7,13 | 35,66 | 7,72 | 38,60 |
| weiblich | gehen Kopfnicken | 50 | 54 | 5,87 | 29,34 | 4,76 | 23,79 | 6,93 | 34,67 | 7,41 | 37,05 |
| weiblich | gehen Kopfnicken | 55 | 59 | 6,35 | 31,74 | 5,34 | 26,70 | 6,52 | 32,58 | 7,36 | 36,80 |
| weiblich | gehen Kopfnicken | 60 | 64 | 5,48 | 27,38 | 4,36 | 21,79 | 4,91 | 24,54 | 6,22 | 31,08 |
| weiblich | gehen Kopfnicken | 65 | 69 | 5,79 | 28,93 | 4,68 | 23,40 | 6,84 | 34,22 | 7,82 | 39,12 |
| weiblich | gehen Kopfnicken | 70 | 74 | 5,42 | 27,08 | 5,42 | 27,09 | 6,80 | 33,99 | 7,29 | 36,43 |
| weiblich | gehen Kopfnicken | 75 | 79 | 5,96 | 29,78 | 6,32 | 31,58 | 8,39 | 41,96 | 9,15 | 45,76 |
| weiblich | gehen Kopfnicken | 80 | 84 | 6,96 | 34,78 | 5,15 | 25,77 | 6,83 | 34,17 | 8,85 | 44,23 |
| weiblich | gehen Kopfnicken | 85 | 90 | 6,66 | 33,30 | 4,57 | 22,86 | 8,93 | 44,67 | 9,09 | 45,44 |
| weiblich | gehen AG | 15 | 19 | 5,90 | 29,51 | 5,37 | 26,87 | 6,36 | 31,82 | 6,30 | 31,49 |
| weiblich | gehen AG | 20 | 24 | 8,07 | 40,35 | 7,07 | 35,37 | 6,99 | 34,94 | 9,28 | 46,42 |
| weiblich | gehen AG | 25 | 29 | 7,86 | 39,28 | 4,71 | 23,57 | 6,88 | 34,38 | 10,31 | 51,56 |
| weiblich | gehen AG | 30 | 34 | 7,18 | 35,89 | 7,04 | 35,21 | 7,09 | 35,45 | 8,31 | 41,54 |
| weiblich | gehen AG | 35 | 39 | 8,01 | 40,07 | 4,99 | 24,97 | 7,17 | 35,87 | 9,96 | 49,82 |
| weiblich | gehen AG | 40 | 44 | 5,94 | 29,69 | 4,82 | 24,09 | 5,56 | 27,82 | 7,25 | 36,23 |
| weiblich | gehen AG | 45 | 49 | 7,15 | 35,75 | 4,72 | 23,58 | 5,77 | 28,85 | 7,41 | 37,06 |
| weiblich | gehen AG | 50 | 54 | 6,14 | 30,71 | 4,70 | 23,52 | 6,36 | 31,81 | 6,64 | 33,19 |
| weiblich | gehen AG | 55 | 59 | 6,18 | 30,92 | 4,86 | 24,29 | 5,53 | 27,63 | 6,00 | 29,99 |
| weiblich | gehen AG | 60 | 64 | 5,53 | 27,63 | 4,42 | 22,09 | 5,32 | 26,61 | 6,44 | 32,20 |
| weiblich | gehen AG | 65 | 69 | 5,02 | 25,08 | 4,67 | 23,34 | 6,25 | 31,25 | 7,77 | 38,84 |
| weiblich | gehen AG | 70 | 74 | 5,19 | 25,93 | 4,25 | 21,24 | 5,79 | 28,97 | 6,38 | 31,88 |
| weiblich | gehen AG | 75 | 79 | 5,38 | 26,90 | 4,37 | 21,87 | 6,33 | 31,65 | 7,53 | 37,66 |
| weiblich | gehen AG | 80 | 84 | 5,52 | 27,59 | 4,90 | 24,51 | 7,34 | 36,71 | 8,34 | 41,70 |
| weiblich | gehen AG | 85 | 90 | 7,18 | 35,88 | 4,36 | 21,79 | 5,98 | 29,90 | 7,87 | 39,34 |
| weiblich | gehen über Hindernisse | 15 | 19 | 10,70 | 53,52 | 11,12 | 55,61 | 16,88 | 84,39 | 15,58 | 77,91 |
| weiblich | gehen über Hindernisse | 20 | 24 | 12,11 | 60,57 | 12,25 | 61,27 | 17,58 | 87,91 | 15,89 | 79,47 |
| weiblich | gehen über Hindernisse | 25 | 29 | 13,28 | 66,39 | 12,33 | 61,67 | 13,86 | 69,29 | 16,29 | 81,44 |
| weiblich | gehen über Hindernisse | 30 | 34 | 13,33 | 66,65 | 13,79 | 68,96 | 14,75 | 73,77 | 14,62 | 73,09 |
| weiblich | gehen über Hindernisse | 35 | 39 | 9,67 | 48,33 | 9,72 | 48,62 | 19,60 | 98,02 | 20,91 | 104,57 |
| weiblich | gehen über Hindernisse | 40 | 44 | 10,01 | 50,07 | 12,83 | 64,14 | 15,07 | 75,33 | 11,51 | 57,54 |
| weiblich | gehen über Hindernisse | 45 | 49 | 11,11 | 55,57 | 13,88 | 69,38 | 18,32 | 91,62 | 18,46 | 92,29 |
| weiblich | gehen über Hindernisse | 50 | 54 | 9,13 | 45,63 | 11,67 | 58,34 | 20,26 | 101,29 | 17,80 | 89,00 |
| weiblich | gehen über Hindernisse | 55 | 59 | 11,94 | 59,71 | 15,78 | 78,89 | 21,06 | 105,31 | 17,77 | 88,87 |
| weiblich | gehen über Hindernisse | 60 | 64 | 15,32 | 76,62 | 19,06 | 95,31 | 13,38 | 66,92 | 13,35 | 66,75 |
| weiblich | gehen über Hindernisse | 65 | 69 | 15,26 | 76,32 | 17,73 | 88,66 | 15,26 | 76,30 | 16,41 | 82,07 |
| weiblich | gehen über Hindernisse | 70 | 74 | 13,74 | 68,71 | 15,61 | 78,07 | 16,04 | 80,21 | 18,91 | 94,53 |
| weiblich | gehen über Hindernisse | 75 | 79 | 13,03 | 65,14 | 18,35 | 91,75 | 15,32 | 76,59 | 15,73 | 78,66 |
| weiblich | gehen über Hindernisse | 80 | 84 | 11,67 | 58,35 | 18,08 | 90,38 | 13,14 | 65,72 | 13,82 | 69,12 |
| weiblich | gehen über Hindernisse | 85 | 90 | 12,60 | 63,02 | 17,04 | 85,22 | 13,97 | 69,84 | 16,29 | 81,43 |
| weiblich | gehen AO | 15 | 19 | 8,65 | 43,25 | 6,05 | 30,27 | 8,32 | 41,60 | 8,12 | 40,61 |
| weiblich | gehen AO | 20 | 24 | 10,09 | 50,47 | 7,01 | 35,07 | 8,19 | 40,95 | 9,87 | 49,35 |
| weiblich | gehen AO | 25 | 29 | 10,22 | 51,08 | 6,08 | 30,41 | 7,10 | 35,51 | 9,36 | 46,79 |
| weiblich | gehen AO | 30 | 34 | 7,20 | 35,98 | 8,11 | 40,57 | 8,34 | 41,69 | 10,24 | 51,21 |
| weiblich | gehen AO | 35 | 39 | 9,54 | 47,72 | 6,56 | 32,79 | 8,58 | 42,91 | 9,88 | 49,38 |
| weiblich | gehen AO | 40 | 44 | 7,34 | 36,72 | 4,63 | 23,16 | 6,67 | 33,36 | 8,21 | 41,07 |
| weiblich | gehen AO | 45 | 49 | 9,27 | 46,35 | 6,45 | 32,25 | 6,87 | 34,35 | 8,59 | 42,93 |
| weiblich | gehen AO | 50 | 54 | 7,92 | 39,61 | 5,90 | 29,51 | 9,63 | 48,16 | 9,27 | 46,37 |
| weiblich | gehen AO | 55 | 59 | 7,28 | 36,41 | 5,52 | 27,58 | 6,96 | 34,78 | 7,44 | 37,18 |
| weiblich | gehen AO | 60 | 64 | 6,75 | 33,75 | 4,46 | 22,31 | 6,39 | 31,93 | 7,39 | 36,95 |
| weiblich | gehen AO | 65 | 69 | 6,01 | 30,07 | 4,51 | 22,54 | 7,19 | 35,95 | 7,92 | 39,61 |
| weiblich | gehen AO | 70 | 74 | 6,10 | 30,49 | 5,04 | 25,22 | 7,23 | 36,15 | 8,05 | 40,23 |
| weiblich | gehen AO | 75 | 79 | 4,79 | 23,95 | 4,37 | 21,86 | 6,58 | 32,90 | 7,93 | 39,67 |
| weiblich | gehen AO | 80 | 84 | 6,44 | 32,21 | 4,68 | 23,40 | 7,30 | 36,48 | 8,56 | 42,80 |
| weiblich | gehen AO | 85 | 90 | 6,95 | 34,73 | 4,86 | 24,29 | 7,10 | 35,49 | 7,90 | 39,48 |
| | | | | | | | | | | | |

| | | Alter | | rechts | | links | | vorwärts | | rückwärts | |
|---|---|---|---|---|---|---|---|---|---|---|---|
| Geschlecht | Aufgabe | von | bis | von | bis | von | bis | von | bis | von | bis |
| weiblich | hinsetzen | 20 | 24 | 10,24 | 51,20 | 9,82 | 49,12 | 6,10 | 30,52 | 15,41 | 77,03 |
| weiblich | hinsetzen | 25 | 29 | 10,47 | 52,34 | 9,36 | 46,79 | 8,92 | 44,62 | 12,60 | 62,99 |
| weiblich | hinsetzen | 30 | 34 | 12,00 | 59,98 | 12,49 | 62,45 | 9,25 | 46,26 | 11,10 | 55,52 |
| weiblich | hinsetzen | 35 | 39 | 11,34 | 56,68 | 10,50 | 52,48 | 11,62 | 58,11 | 14,80 | 73,99 |
| weiblich | hinsetzen | 40 | 44 | 10,64 | 53,20 | 11,57 | 57,87 | 12,17 | 60,83 | 12,69 | 63,43 |
| weiblich | hinsetzen | 45 | 49 | 13,91 | 69,56 | 12,67 | 63,37 | 8,17 | 40,86 | 11,32 | 56,62 |
| weiblich | hinsetzen | 50 | 54 | 11,76 | 58,81 | 11,84 | 59,21 | 14,62 | 73,12 | 12,89 | 64,43 |
| weiblich | hinsetzen | 55 | 59 | 14,37 | 71,85 | 12,02 | 60,10 | 13,16 | 65,81 | 19,63 | 98,16 |
| weiblich | hinsetzen | 60 | 64 | 12,41 | 62,07 | 12,92 | 64,59 | 11,67 | 58,35 | 12,04 | 60,18 |
| weiblich | hinsetzen | 65 | 69 | 11,95 | 59,76 | 11,34 | 56,70 | 12,63 | 63,16 | 14,50 | 72,51 |
| weiblich | hinsetzen | 70 | 74 | 11,21 | 56,05 | 11,32 | 56,60 | 11,86 | 59,29 | 15,09 | 75,45 |
| weiblich | hinsetzen | 75 | 79 | 14,72 | 73,61 | 12,12 | 60,58 | 13,02 | 65,09 | 18,35 | 91,75 |
| weiblich | hinsetzen | 80 | 84 | 11,72 | 58,62 | 9,06 | 45,28 | 10,85 | 54,23 | 14,07 | 70,33 |
| weiblich | hinsetzen | 85 | 90 | 11,70 | 58,52 | 9,10 | 45,50 | 8,53 | 42,63 | 12,68 | 63,41 |
| weiblich | aufstehen | 20 | 24 | 5,20 | 26,01 | 10,03 | 50,17 | 13,37 | 66,85 | 3,98 | 19,88 |
| weiblich | aufstehen | 25 | 29 | 7,76 | 38,78 | 17,51 | 87,54 | 15,29 | 76,45 | 6,36 | 31,79 |
| weiblich | aufstehen | 30 | 34 | 17,46 | 87,30 | 15,89 | 79,43 | 12,65 | 63,25 | 8,81 | 44,07 |
| weiblich | aufstehen | 35 | 39 | 10,02 | 50,09 | 14,86 | 74,28 | 16,05 | 80,26 | 10,19 | 50,96 |
| weiblich | aufstehen | 40 | 44 | 11,00 | 55,01 | 15,34 | 76,71 | 16,69 | 83,46 | 11,20 | 56,01 |
| weiblich | aufstehen | 45 | 49 | 10,63 | 53,15 | 16,89 | 84,47 | 13,93 | 69,65 | 8,18 | 40,88 |
| weiblich | aufstehen | 50 | 54 | 11,18 | 55,92 | 16,72 | 83,61 | 17,04 | 85,20 | 12,63 | 63,16 |
| weiblich | aufstehen | 55 | 59 | 10,72 | 53,61 | 16,75 | 83,77 | 26,01 | 130,03 | 13,55 | 67,73 |
| weiblich | aufstehen | 60 | 64 | 10,42 | 52,12 | 14,13 | 70,67 | 14,75 | 73,73 | 11,88 | 59,38 |
| weiblich | aufstehen | 65 | 69 | 11,15 | 55,74 | 16,00 | 79,99 | 19,55 | 97,74 | 13,42 | 67,09 |
| weiblich | aufstehen | 70 | 74 | 10,59 | 52,96 | 15,02 | 75,09 | 18,51 | 92,54 | 12,32 | 61,59 |
| weiblich | aufstehen | 75 | 79 | 10,71 | 53,56 | 16,15 | 80,75 | 19,70 | 98,48 | 12,42 | 62,08 |
| weiblich | aufstehen | 80 | 84 | 11,49 | 57,47 | 15,51 | 77,53 | 19,90 | 99,51 | 13,04 | 65,22 |
| weiblich | aufstehen | 85 | 90 | 11,06 | 55,28 | 16,41 | 82,04 | 18,30 | 91,49 | 14,10 | 70,50 |

**Tabelle 2: Bereich der Normwerte für die Schwankungsgeschwindigkeit des Körperschwerpunktes (Winkelgeschwindigkeit in °/s) bei verschiedenen Altersgruppen und Übungen sowie beider Geschlechter (AO = Augen offen, AG = Augen geschlossen). Es werden rotatorische Schwankungen betrachtet.**

| | | Alter | | Schwankung seitwärts | | Schwankung vor/zurück | |
|---|---|---|---|---|---|---|---|
| Geschlecht | Aufgabe | von | bis | von | bis | von | bis |
| männlich | stehen AO | 15 | 19 | 0,05 | 0,23 | 0,09 | 0,44 |
| männlich | stehen AO | 20 | 24 | 0,17 | 0,83 | 0,16 | 0,78 |
| männlich | stehen AO | 25 | 29 | 0,13 | 0,67 | 0,16 | 0,82 |
| männlich | stehen AO | 30 | 34 | 0,18 | 0,90 | 0,24 | 1,21 |
| männlich | stehen AO | 35 | 39 | 0,16 | 0,81 | 0,12 | 0,61 |
| männlich | stehen AO | 40 | 44 | 0,16 | 0,82 | 0,20 | 1,02 |
| männlich | stehen AO | 45 | 49 | 0,18 | 0,92 | 0,27 | 1,34 |
| männlich | stehen AO | 50 | 54 | 0,14 | 0,68 | 0,11 | 0,53 |
| männlich | stehen AO | 55 | 59 | 0,13 | 0,66 | 0,20 | 1,01 |
| männlich | stehen AO | 60 | 64 | 0,11 | 0,53 | 0,15 | 0,75 |
| männlich | stehen AO | 65 | 69 | 0,10 | 0,52 | 0,20 | 1,01 |
| männlich | stehen AO | 70 | 74 | 0,13 | 0,66 | 0,16 | 0,81 |
| männlich | stehen AO | 75 | 79 | 0,13 | 0,67 | 0,20 | 1,01 |
| männlich | stehen AO | 80 | 84 | 0,12 | 0,61 | 0,09 | 0,44 |
| männlich | stehen AO | 85 | 90 | 0,13 | 0,66 | 0,12 | 0,61 |
| männlich | stehen AG | 15 | 19 | 0,05 | 0,23 | 0,07 | 0,33 |
| männlich | stehen AG | 20 | 24 | 0,17 | 0,87 | 0,16 | 0,81 |
| männlich | stehen AG | 25 | 29 | 0,15 | 0,75 | 0,20 | 1,01 |
| männlich | stehen AG | 30 | 34 | 0,20 | 1,01 | 0,29 | 1,44 |
| männlich | stehen AG | 35 | 39 | 0,13 | 0,65 | 0,14 | 0,68 |
| männlich | stehen AG | 40 | 44 | 0,18 | 0,91 | 0,21 | 1,04 |
| männlich | stehen AG | 45 | 49 | 0,19 | 0,94 | 0,30 | 1,49 |
| männlich | stehen AG | 50 | 54 | 0,22 | 1,10 | 0,14 | 0,70 |
| männlich | stehen AG | 55 | 59 | 0,16 | 0,82 | 0,21 | 1,04 |
| männlich | stehen AG | 60 | 64 | 0,13 | 0,67 | 0,17 | 0,86 |
| männlich | stehen AG | 65 | 69 | 0,11 | 0,55 | 0,20 | 1,01 |
| männlich | stehen AG | 70 | 74 | 0,13 | 0,67 | 0,19 | 0,95 |
| männlich | stehen AG | 75 | 79 | 0,14 | 0,68 | 0,23 | 1,17 |
| männlich | stehen AG | 80 | 84 | 0,14 | 0,71 | 0,11 | 0,56 |
| männlich | stehen AG | 85 | 90 | 0,13 | 0,66 | 0,13 | 0,66 |
| männlich | stehen 1 Bein AO | 15 | 19 | 0,11 | 0,56 | 0,13 | 0,66 |
| männlich | stehen 1 Bein AO | 20 | 24 | 0,24 | 1,18 | 0,30 | 1,48 |
| männlich | stehen 1 Bein AO | 25 | 29 | 0,22 | 1,11 | 0,29 | 1,43 |
| männlich | stehen 1 Bein AO | 30 | 34 | 0,22 | 1,12 | 0,33 | 1,66 |
| männlich | stehen 1 Bein AO | 35 | 39 | 0,34 | 1,70 | 0,32 | 1,60 |
| männlich | stehen 1 Bein AO | 40 | 44 | 0,47 | 2,35 | 0,47 | 2,33 |
| männlich | stehen 1 Bein AO | 45 | 49 | 0,34 | 1,68 | 0,42 | 2,10 |
| männlich | stehen 1 Bein AO | 50 | 54 | 0,46 | 2,30 | 0,33 | 1,65 |
| männlich | stehen 1 Bein AO | 55 | 59 | 0,31 | 1,55 | 0,38 | 1,89 |
| männlich | stehen 1 Bein AO | 60 | 64 | 0,57 | 2,84 | 0,47 | 2,35 |
| männlich | stehen 1 Bein AO | 65 | 69 | 0,55 | 2,77 | 0,63 | 3,17 |
| männlich | stehen 1 Bein AO | 70 | 74 | 0,53 | 2,63 | 0,64 | 3,21 |
| männlich | stehen 1 Bein AO | 75 | 79 | 0,82 | 4,08 | 0,87 | 4,37 |
| männlich | stehen 1 Bein AO | 80 | 84 | 0,59 | 2,97 | 0,31 | 1,55 |
| männlich | stehen 1 Bein AO | 85 | 90 | 0,29 | 1,43 | 0,29 | 1,43 |
| männlich | stehen 1 Bein AG | 15 | 19 | 0,15 | 0,77 | 0,15 | 0,77 |
| männlich | stehen 1 Bein AG | 20 | 24 | 0,60 | 2,98 | 0,69 | 3,47 |
| männlich | stehen 1 Bein AG | 25 | 29 | 0,56 | 2,80 | 0,69 | 3,44 |
| männlich | stehen 1 Bein AG | 30 | 34 | 0,63 | 3,15 | 0,72 | 3,60 |
| männlich | stehen 1 Bein AG | 35 | 39 | 0,87 | 4,35 | 0,81 | 4,07 |
| männlich | stehen 1 Bein AG | 40 | 44 | 0,75 | 3,73 | 0,86 | 4,78 |
| männlich | stehen 1 Bein AG | 45 | 49 | 1,14 | 5,71 | 1,52 | 7,60 |
| männlich | stehen 1 Bein AG | 50 | 54 | 1,77 | 8,83 | 2,51 | 12,54 |
| männlich | stehen 1 Bein AG | 55 | 60 | 2,39 | 11,95 | 3,50 | 17,49 |
| männlich | gehen tandem | 15 | 19 | 0,95 | 4,73 | 1,49 | 7,47 |
| männlich | gehen tandem | 20 | 24 | 1,25 | 6,27 | 1,51 | 7,53 |
| männlich | gehen tandem | 25 | 29 | 1,41 | 7,03 | 1,63 | 8,17 |
| männlich | gehen tandem | 30 | 34 | 1,42 | 7,09 | 1,67 | 8,35 |
| männlich | gehen tandem | 35 | 39 | 1,38 | 6,91 | 1,65 | 8,26 |
| männlich | gehen tandem | 40 | 44 | 1,41 | 7,05 | 1,91 | 9,56 |
| männlich | gehen tandem | 45 | 49 | 1,50 | 7,52 | 1,94 | 9,71 |
| männlich | gehen tandem | 50 | 54 | 1,48 | 7,40 | 1,31 | 6,57 |
| männlich | gehen tandem | 55 | 59 | 1,46 | 7,30 | 1,79 | 8,96 |
| männlich | gehen tandem | 60 | 64 | 1,45 | 7,27 | 1,66 | 8,32 |
| männlich | gehen tandem | 65 | 69 | 1,51 | 7,55 | 1,92 | 9,60 |
| männlich | gehen tandem | 70 | 74 | 1,27 | 6,37 | 1,56 | 7,78 |
| männlich | gehen tandem | 75 | 79 | 1,50 | 7,52 | 1,90 | 9,51 |
| männlich | gehen tandem | 80 | 84 | 1,66 | 8,30 | 1,39 | 6,93 |
| männlich | gehen tandem | 85 | 90 | 1,74 | 8,69 | 1,10 | 5,49 |
| männlich | stehen Schaumstoff AO | 15 | 19 | 0,09 | 0,44 | 0,09 | 0,44 |
| männlich | stehen Schaumstoff AO | 20 | 24 | 0,20 | 0,98 | 0,20 | 1,02 |
| männlich | stehen Schaumstoff AO | 25 | 29 | 0,19 | 0,97 | 0,28 | 1,39 |
| männlich | stehen Schaumstoff AO | 30 | 34 | 0,25 | 1,23 | 0,38 | 1,88 |
| männlich | stehen Schaumstoff AO | 35 | 39 | 0,22 | 1,09 | 0,23 | 1,16 |
| männlich | stehen Schaumstoff AO | 40 | 44 | 0,21 | 1,03 | 0,25 | 1,24 |
| männlich | stehen Schaumstoff AO | 45 | 49 | 0,22 | 1,12 | 0,34 | 1,69 |
| männlich | stehen Schaumstoff AO | 50 | 54 | 0,37 | 1,87 | 0,27 | 1,35 |
| männlich | stehen Schaumstoff AO | 55 | 59 | 0,26 | 1,32 | 0,33 | 1,64 |
| männlich | stehen Schaumstoff AO | 60 | 64 | 0,21 | 1,04 | 0,29 | 1,46 |
| männlich | stehen Schaumstoff AO | 65 | 69 | 0,19 | 0,93 | 0,30 | 1,49 |
| männlich | stehen Schaumstoff AO | 70 | 74 | 0,21 | 1,06 | 0,30 | 1,48 |
| männlich | stehen Schaumstoff AO | 75 | 79 | 0,21 | 1,04 | 0,32 | 1,58 |
| männlich | stehen Schaumstoff AO | 80 | 84 | 0,24 | 1,22 | 0,18 | 0,89 |
| männlich | stehen Schaumstoff AO | 85 | 90 | 0,31 | 1,54 | 0,18 | 0,89 |
| männlich | stehen Schaumstoff AG | 15 | 19 | 0,09 | 0,44 | 0,11 | 0,56 |
| männlich | stehen Schaumstoff AG | 20 | 24 | 0,19 | 0,97 | 0,18 | 0,92 |
| männlich | stehen Schaumstoff AG | 25 | 29 | 0,25 | 1,26 | 0,31 | 1,54 |
| männlich | stehen Schaumstoff AG | 30 | 34 | 0,26 | 1,28 | 0,37 | 1,83 |
| männlich | stehen Schaumstoff AG | 35 | 39 | 0,25 | 1,26 | 0,27 | 1,36 |
| männlich | stehen Schaumstoff AG | 40 | 44 | 0,24 | 1,21 | 0,25 | 1,25 |
| männlich | stehen Schaumstoff AG | 45 | 49 | 0,25 | 1,25 | 0,36 | 1,82 |
| männlich | stehen Schaumstoff AG | 50 | 54 | 0,41 | 2,04 | 0,30 | 1,52 |
| männlich | stehen Schaumstoff AG | 55 | 59 | 0,31 | 1,55 | 0,37 | 1,86 |
| männlich | stehen Schaumstoff AG | 60 | 64 | 0,31 | 1,53 | 0,35 | 1,76 |
| männlich | stehen Schaumstoff AG | 65 | 69 | 0,24 | 1,20 | 0,35 | 1,75 |
| männlich | stehen Schaumstoff AG | 70 | 74 | 0,31 | 1,54 | 0,44 | 2,22 |
| männlich | stehen Schaumstoff AG | 75 | 79 | 0,29 | 1,43 | 0,35 | 1,76 |
| männlich | stehen Schaumstoff AG | 80 | 84 | 0,33 | 1,65 | 0,26 | 1,32 |
| männlich | stehen Schaumstoff AG | 85 | 90 | 0,40 | 1,98 | 0,40 | 1,98 |
| männlich | stehen Schaumstoff 1 Bein | 15 | 19 | 0,18 | 0,89 | 0,13 | 0,66 |
| männlich | stehen Schaumstoff 1 Bein | 20 | 24 | 0,27 | 1,36 | 0,29 | 1,46 |
| männlich | stehen Schaumstoff 1 Bein | 25 | 29 | 0,35 | 1,77 | 0,44 | 2,19 |
| männlich | stehen Schaumstoff 1 Bein | 30 | 34 | 0,50 | 2,49 | 0,49 | 2,46 |
| männlich | stehen Schaumstoff 1 Bein | 35 | 39 | 0,57 | 2,84 | 0,51 | 2,53 |
| männlich | stehen Schaumstoff 1 Bein | 40 | 44 | 0,56 | 2,79 | 0,52 | 2,62 |
| männlich | stehen Schaumstoff 1 Bein | 45 | 49 | 0,61 | 3,04 | 0,71 | 3,54 |
| männlich | stehen Schaumstoff 1 Bein | 50 | 54 | 0,84 | 4,21 | 0,90 | 4,51 |
| männlich | stehen Schaumstoff 1 Bein | 55 | 60 | 1,32 | 6,60 | 1,14 | 5,72 |

| Geschlecht | Aufgabe Aufgabe | von | bis | von | bis | von | bis |
|---|---|---|---|---|---|---|---|
| männlich | gehen tandem Schaumstoff | 15 | 19 | 0,90 | 4,50 | 0,81 | 4,07 |
| männlich | gehen tandem Schaumstoff | 20 | 24 | 1,35 | 6,75 | 1,53 | 7,65 |
| männlich | gehen tandem Schaumstoff | 25 | 29 | . 1,40 | 7,02 | 1,60 | 8,01 |
| männlich | gehen tandem Schaumstoff | 30 | 34 | 1,63 | 8,16 | 2,17 | 10,83 |
| männlich | gehen tandem Schaumstoff | 35 | 39 | 1,75 | 8,76 | 1,98 | 9,91 |
| männlich | gehen tandem Schaumstoff | 40 | 44 | 1,54 | 7,70 | 2,09 | 10,43 |
| männlich | gehen tandem Schaumstoff | 45 | 49 | 1,89 | 9,44 | 2,43 | 12,14 |
| männlich | gehen tandem Schaumstoff | 50 | 54 | 2,17 | 10,85 | 1,60 | 8,01 |
| männlich | gehen tandem Schaumstoff | 55 | 59 | 2,52 | 12,62 | 3,17 | 15,87 |
| männlich | gehen tandem Schaumstoff | 60 | 64 | 1,76 | 8,81 | 1,94 | 9,71 |
| männlich | gehen tandem Schaumstoff | 65 | 69 | 2,02 | 10,12 | 2,49 | 12,47 |
| männlich | gehen tandem Schaumstoff | 70 | 74 | 1,44 | 7,19 | 2,06 | 10,29 |
| männlich | gehen tandem Schaumstoff | 75 | 79 | 1,56 | 7,82 | 2,33 | 11,65 |
| männlich | gehen tandem Schaumstoff | 80 | 84 | 2,01 | 10,06 | 1,50 | 7,52 |
| männlich | gehen tandem Schaumstoff | 85 | 90 | 2,00 | 10,01 | 1,25 | 6,27 |
| männlich | gehen Kopfkreisen | 15 | 19 | 2,00 | 10,01 | 0,83 | 4,17 |
| männlich | gehen Kopfkreisen | 20 | 24 | 1,54 | 7,72 | 1,81 | 9,07 |
| männlich | gehen Kopfkreisen | 25 | 29 | 1,74 | 8,71 | 2,28 | 11,41 |
| männlich | gehen Kopfkreisen | 30 | 34 | 1,79 | 8,94 | 2,51 | 12,55 |
| männlich | gehen Kopfkreisen | 35 | 39 | 1,61 | 8,03 | 2,47 | 12,35 |
| männlich | gehen Kopfkreisen | 40 | 44 | 1,75 | 8,73 | 2,57 | 12,83 |
| männlich | gehen Kopfkreisen | 45 | 49 | 1,76 | 8,81 | 2,33 | 11,63 |
| männlich | gehen Kopfkreisen | 50 | 54 | 1,99 | 9,96 | 1,26 | 6,28 |
| männlich | gehen Kopfkreisen | 55 | 59 | 2,09 | 10,44 | 2,44 | 12,21 |
| männlich | gehen Kopfkreisen | 60 | 64 | 1,69 | 8,43 | 2,07 | 10,33 |
| männlich | gehen Kopfkreisen | 65 | 69 | 1,58 | 7,92 | 2,05 | 10,23 |
| männlich | gehen Kopfkreisen | 70 | 74 | 1,52 | 7,58 | 1,66 | 8,30 |
| männlich | gehen Kopfkreisen | 75 | 79 | 1,48 | 7,38 | 2,19 | 10,96 |
| männlich | gehen Kopfkreisen | 80 | 84 | 1,47 | 7,37 | 1,50 | 7,52 |
| männlich | gehen Kopfkreisen | 85 | 90 | 1,53 | 7,64 | 1,44 | 7,20 |

| Geschlecht | Aufgabe | von | bis | von | bis | von | bis |
|---|---|---|---|---|---|---|---|
| männlich | gehen Kopfnicken | 15 | 19 | 1,56 | 7,80 | 1,43 | 7,14 |
| männlich | gehen Kopfnicken | 20 | 24 | 1,73 | 8,66 | 2,21 | 11,04 |
| männlich | gehen Kopfnicken | 25 | 29 | 1,80 | 8,98 | 2,35 | 11,74 |
| männlich | gehen Kopfnicken | 30 | 34 | 1,84 | 9,22 | 2,63 | 13,14 |
| männlich | gehen Kopfnicken | 35 | 39 | 1,70 | 8,51 | 2,41 | 12,03 |
| männlich | gehen Kopfnicken | 40 | 44 | 2,00 | 10,00 | 3,62 | 18,10 |
| männlich | gehen Kopfnicken | 45 | 49 | 1,63 | 8,17 | 2,53 | 12,67 |
| männlich | gehen Kopfnicken | 50 | 54 | 1,98 | 9,88 | 1,59 | 7,96 |
| männlich | gehen Kopfnicken | 55 | 59 | 2,11 | 10,54 | 2,73 | 13,67 |
| männlich | gehen Kopfnicken | 60 | 64 | 1,57 | 7,83 | 1,76 | 8,78 |
| männlich | gehen Kopfnicken | 65 | 69 | 1,74 | 8,70 | 2,31 | 11,53 |
| männlich | gehen Kopfnicken | 70 | 74 | 1,43 | 7,14 | 1,95 | 9,74 |
| männlich | gehen Kopfnicken | 75 | 79 | 1,41 | 7,07 | 2,25 | 11,25 |
| männlich | gehen Kopfnicken | 80 | 84 | 1,78 | 8,90 | 1,43 | 7,14 |
| männlich | gehen Kopfnicken | 85 | 90 | 1,49 | 7,47 | 1,53 | 7,64 |
| männlich | gehen AG | 15 | 19 | 1,32 | 6,60 | 1,47 | 7,37 |
| männlich | gehen AG | 20 | 24 | 1,36 | 6,82 | 1,61 | 8,04 |
| männlich | gehen AG | 25 | 29 | 1,95 | 9,77 | 2,16 | 10,81 |
| männlich | gehen AG | 30 | 34 | 1,64 | 8,22 | 2,44 | 12,18 |
| männlich | gehen AG | 35 | 39 | 1,64 | 8,19 | 2,02 | 10,08 |
| männlich | gehen AG | 40 | 44 | 1,61 | 8,07 | 3,38 | 16,89 |
| männlich | gehen AG | 45 | 49 | 1,73 | 8,64 | 2,57 | 12,84 |
| männlich | gehen AG | 50 | 54 | 2,04 | 10,18 | 1,56 | 7,79 |
| männlich | gehen AG | 55 | 59 | 2,22 | 11,08 | 3,16 | 15,79 |
| männlich | gehen AG | 60 | 64 | 1,42 | 7,11 | 1,79 | 8,93 |
| männlich | gehen AG | 65 | 69 | 1,47 | 7,34 | 2,27 | 11,36 |
| männlich | gehen AG | 70 | 74 | 1,20 | 5,98 | 1,70 | 8,48 |
| männlich | gehen AG | 75 | 79 | 1,22 | 6,12 | 1,91 | 9,55 |
| männlich | gehen AG | 80 | 84 | 1,46 | 7,31 | 1,32 | 6,59 |
| männlich | gehen AG | 85 | 90 | 1,94 | 9,68 | 1,49 | 7,47 |
| männlich | gehen über Hindernisse | 15 | 19 | 1,45 | 7,26 | 2,24 | 11,21 |
| männlich | gehen über Hindernisse | 20 | 24 | 2,61 | 13,04 | 3,26 | 16,29 |
| männlich | gehen über Hindernisse | 25 | 29 | 2,39 | 11,93 | 3,64 | 18,18 |
| männlich | gehen über Hindernisse | 30 | 34 | 2,96 | 14,79 | 3,60 | 18,00 |
| männlich | gehen über Hindernisse | 35 | 39 | 3,28 | 16,38 | 4,10 | 20,48 |
| männlich | gehen über Hindernisse | 40 | 44 | 4,18 | 20,91 | 4,12 | 20,58 |
| männlich | gehen über Hindernisse | 45 | 49 | 3,33 | 16,66 | 3,62 | 18,11 |
| männlich | gehen über Hindernisse | 50 | 54 | 2,58 | 12,91 | 3,21 | 16,07 |
| männlich | gehen über Hindernisse | 55 | 59 | 3,06 | 15,30 | 4,18 | 20,88 |
| männlich | gehen über Hindernisse | 60 | 64 | 3,27 | 16,33 | 3,63 | 18,16 |
| männlich | gehen über Hindernisse | 65 | 69 | 3,14 | 15,71 | 3,57 | 17,85 |
| männlich | gehen über Hindernisse | 70 | 74 | 3,19 | 15,94 | 2,87 | 14,35 |
| männlich | gehen über Hindernisse | 75 | 79 | 3,08 | 15,42 | 3,37 | 16,85 |
| männlich | gehen über Hindernisse | 80 | 84 | 3,12 | 15,60 | 3,34 | 16,71 |
| männlich | gehen über Hindernisse | 85 | 90 | 2,66 | 13,29 | 2,99 | 14,94 |
| männlich | gehen AO | 15 | 19 | 2,35 | 11,76 | 1,61 | 8,03 |
| männlich | gehen AO | 20 | 24 | 1,59 | 7,96 | 2,26 | 11,30 |
| männlich | gehen AO | 25 | 29 | 2,08 | 10,42 | 2,30 | 11,48 |
| männlich | gehen AO | 30 | 34 | 1,99 | 9,97 | 2,81 | 14,06 |
| männlich | gehen AO | 35 | 39 | 2,20 | 11,01 | 2,35 | 11,73 |
| männlich | gehen AO | 40 | 44 | 2,24 | 11,18 | 4,04 | 20,18 |
| männlich | gehen AO | 45 | 49 | 2,03 | 10,17 | 3,00 | 15,00 |
| männlich | gehen AO | 50 | 54 | 2,23 | 11,16 | 1,63 | 8,16 |
| männlich | gehen AO | 55 | 59 | 1,92 | 9,58 | 1,75 | 8,73 |
| männlich | gehen AO | 60 | 64 | 1,60 | 8,01 | 1,86 | 9,30 |
| männlich | gehen AO | 65 | 69 | 1,52 | 7,58 | 2,38 | 11,91 |
| männlich | gehen AO | 70 | 74 | 1,47 | 7,33 | 1,96 | 9,78 |
| männlich | gehen AO | 75 | 79 | 1,21 | 6,04 | 2,19 | 10,95 |
| männlich | gehen AO | 80 | 84 | 1,56 | 7,80 | 1,62 | 8,08 |
| männlich | gehen AO | 85 | 90 | 1,93 | 9,67 | 1,59 | 7,97 |
| männlich | hinsetzen | 20 | 24 | 5,19 | 25,95 | 3,84 | 19,18 |
| männlich | hinsetzen | 25 | 29 | 4,19 | 20,94 | 3,39 | 16,96 |
| männlich | hinsetzen | 30 | 34 | 3,72 | 18,58 | 5,25 | 26,25 |
| männlich | hinsetzen | 35 | 39 | 5,06 | 25,32 | 4,07 | 20,37 |
| männlich | hinsetzen | 40 | 44 | 5,14 | 25,69 | 3,65 | 18,24 |
| männlich | hinsetzen | 45 | 49 | 4,82 | 24,12 | 4,34 | 21,69 |
| männlich | hinsetzen | 50 | 54 | 4,13 | 20,65 | 4,79 | 23,93 |
| männlich | hinsetzen | 55 | 59 | 4,96 | 24,78 | 5,14 | 25,70 |
| männlich | hinsetzen | 60 | 64 | 4,86 | 24,32 | 4,48 | 22,39 |
| männlich | hinsetzen | 65 | 69 | 5,50 | 27,49 | 6,41 | 32,03 |
| männlich | hinsetzen | 70 | 74 | 5,29 | 26,43 | 4,66 | 23,30 |
| männlich | hinsetzen | 75 | 79 | 4,80 | 24,00 | 4,99 | 24,97 |
| männlich | hinsetzen | 80 | 84 | 5,04 | 25,22 | 3,95 | 19,73 |
| männlich | hinsetzen | 85 | 90 | 3,08 | 15,38 | 2,79 | 13,96 |
| männlich | aufstehen | 20 | 24 | 5,94 | 29,68 | 6,22 | 31,11 |
| männlich | aufstehen | 25 | 29 | 5,22 | 26,12 | 3,08 | 15,39 |
| männlich | aufstehen | 30 | 34 | 3,38 | 16,89 | 5,16 | 25,78 |
| männlich | aufstehen | 35 | 39 | 5,61 | 28,05 | 4,57 | 22,87 |
| männlich | aufstehen | 40 | 44 | 4,45 | 22,27 | 4,35 | 21,74 |
| männlich | aufstehen | 45 | 49 | 5,23 | 26,16 | 3,55 | 17,76 |
| männlich | aufstehen | 50 | 54 | 3,99 | 19,94 | 5,60 | 28,02 |
| männlich | aufstehen | 55 | 59 | 3,98 | 19,90 | 6,45 | 32,25 |
| männlich | aufstehen | 60 | 64 | 4,99 | 24,97 | 3,33 | 16,67 |
| männlich | aufstehen | 65 | 69 | 5,11 | 25,55 | 5,42 | 27,08 |
| männlich | aufstehen | 70 | 74 | 5,85 | 29,26 | 5,20 | 26,01 |
| männlich | aufstehen | 75 | 79 | 5,22 | 26,12 | 4,76 | 23,81 |
| männlich | aufstehen | 80 | 84 | 5,54 | 27,69 | 5,49 | 27,47 |
| männlich | aufstehen | 85 | 90 | 4,49 | 22,47 | 5,62 | 28,08 |
| weiblich | stehen AO | 15 | 19 | 0,15 | 0,75 | 0,15 | 0,75 |
| weiblich | stehen AO | 20 | 24 | 0,16 | 0,80 | 0,24 | 1,20 |
| weiblich | stehen AO | 25 | 29 | 0,19 | 0,94 | 0,29 | 1,44 |
| weiblich | stehen AO | 30 | 34 | 0,18 | 0,89 | 0,20 | 1,02 |
| weiblich | stehen AO | 35 | 39 | 0,16 | 0,78 | 0,22 | 1,08 |
| weiblich | stehen AO | 40 | 44 | 0,19 | 0,96 | 0,23 | 1,17 |
| weiblich | stehen AO | 45 | 49 | 0,24 | 1,22 | 0,27 | 1,33 |
| weiblich | stehen AO | 50 | 54 | 0,23 | 1,14 | 0,20 | 1,00 |
| weiblich | stehen AO | 55 | 59 | 0,15 | 0,77 | 0,15 | 0,77 |
| weiblich | stehen AO | 60 | 64 | 0,11 | 0,53 | 0,15 | 0,75 |
| weiblich | stehen AO | 65 | 69 | 0,13 | 0,63 | 0,16 | 0,80 |
| weiblich | stehen AO | 70 | 74 | 0,13 | 0,63 | 0,16 | 0,80 |
| weiblich | stehen AO | 75 | 79 | 0,13 | 0,64 | 0,16 | 0,79 |
| weiblich | stehen AO | 80 | 84 | 0,11 | 0,56 | 0,11 | 0,56 |
| weiblich | stehen AO | 85 | 90 | 0,13 | 0,66 | 0,11 | 0,56 |
| weiblich | stehen AG | 15 | 19 | 0,19 | 0,93 | *0,20* | 0,98 |
| weiblich | stehen AG | 20 | 24 | 0,19 | 0,97 | 0,27 | 1,36 |
| weiblich | stehen AG | 25 | 29 | 0,21 | 1,07 | 0,34 | 1,71 |
| weiblich | stehen AG | 30 | 34 | 0,21 | 1,04 | 0,19 | 0,95 |
| weiblich | stehen AG | 35 | 39 | 0,14 | 0,69 | 0,20 | 0,99 |
| weiblich | stehen AG | 40 | 44 | 0,19 | 0,96 | 0,24 | 1,19 |
| weiblich | stehen AG | 45 | 49 | 0,27 | 1,37 | 0,29 | 1,44 |
| weiblich | stehen AG | 50 | 54 | 0,32 | 1,61 | 0,37 | 1,85 |
| weiblich | stehen AG | 55 | 59 | 0,14 | 0,71 | 0,14 | 0,68 |
| weiblich | stehen AG | 60 | 64 | 0,11 | 0,53 | 0,13 | 0,64 |
| weiblich | stehen AG | 65 | 69 | 0,14 | 0,71 | 0,18 | 0,89 |
| weiblich | stehen AG | 70 | 74 | 0,15 | 0,76 | 0,17 | 0,83 |
| weiblich | stehen AG | 75 | 79 | 0,13 | 0,64 | 0,16 | 0,81 |
| weiblich | stehen AG | 80 | 84 | 0,13 | 0,66 | 0,11 | 0,56 |
| weiblich | stehen AG | 85 | 90 | 0,13 | 0,66 | 0,09 | 0,44 |
| weiblich | stehen 1 Bein AO | 15 | 19 | 0,25 | 1,26 | 0,28 | 1,41 |
| weiblich | stehen 1 Bein AO | 20 | 24 | 0,26 | 1,30 | 0,44 | 2,20 |
| weiblich | stehen 1 Bein AO | 25 | 29 | 0,27 | 1,35 | 0,41 | 2,05 |
| weiblich | stehen 1 Bein AO | 30 | 34 | 0,32 | 1,58 | 0,31 | 1,55 |
| weiblich | stehen 1 Bein AO | 35 | 39 | 0,31 | 1,57 | 0,39 | 1,97 |
| weiblich | stehen 1 Bein AO | 40 | 44 | 0,28 | 1,39 | 0,31 | 1,57 |
| weiblich | stehen 1 Bein AO | 45 | 49 | 0,41 | 2,04 | 0,41 | 2,07 |
| weiblich | stehen 1 Bein AO | 50 | 54 | 0,36 | 1,82 | 0,40 | 1,99 |
| weiblich | stehen 1 Bein AO | 55 | 59 | 0,38 | 1,89 | 0,42 | 2,08 |
| weiblich | stehen 1 Bein AO | 60 | 64 | 0,23 | 1,13 | 0,29 | 1,43 |
| weiblich | stehen 1 Bein AO | 65 | 69 | 0,39 | 1,97 | 0,49 | 2,46 |
| weiblich | stehen 1 Bein AO | 70 | 74 | 0,59 | 2,96 | 0,62 | 3,10 |
| weiblich | stehen 1 Bein AO | 75 | 79 | 0,71 | 3,55 | 0,82 | 4,11 |
| weiblich | stehen 1 Bein AO | 80 | 84 | 0,44 | 2,20 | 0,31 | 1,54 |
| weiblich | stehen 1 Bein AO | 85 | 90 | 0,48 | 2,42 | 0,43 | 2,15 |
| weiblich | stehen 1 Bein AG | 15 | 19 | 0,74 | 3,71 | 0,77 | 3,86 |
| weiblich | stehen 1 Bein AG | 20 | 24 | 0,77 | 3,84 | 0,72 | 3,59 |
| weiblich | stehen 1 Bein AG | 25 | 29 | 0,58 | 2,92 | 0,84 | 4,19 |
| weiblich | stehen 1 Bein AG | 30 | 34 | 0,58 | 2,91 | 0,54 | 2,71 |
| weiblich | stehen 1 Bein AG | 35 | 39 | 1,03 | 5,16 | 0,98 | 4,92 |
| weiblich | stehen 1 Bein AG | 40 | 44 | 0,77 | 3,86 | 0,94 | 4,68 |
| weiblich | stehen 1 Bein AG | 45 | 49 | 0,66 | 3,32 | 0,77 | 3,85 |
| weiblich | stehen 1 Bein AG | 50 | 54 | 0,89 | 4,47 | 1,25 | 6,23 |
| weiblich | stehen 1 Bein AG | 55 | 60 | 0,86 | 4,31 | 0,81 | 4,03 |
| weiblich | gehen tandem | 15 | 19 | 1,30 | 6,51 | 1,33 | 6,66 |
| weiblich | gehen tandem | 20 | 24 | 1,36 | 6,80 | 2,07 | 10,34 |
| weiblich | gehen tandem | 25 | 29 | 1,44 | 7,20 | 1,45 | 7,24 |
| weiblich | gehen tandem | 30 | 34 | 1,65 | 8,24 | 1,59 | 7,97 |
| weiblich | gehen tandem | 35 | 39 | 1,31 | 6,53 | 1,48 | 7,40 |
| weiblich | gehen tandem | 40 | 44 | 1,25 | 6,25 | 1,57 | 7,85 |
| weiblich | gehen tandem | 45 | 49 | 1,37 | 6,87 | 1,71 | 8,54 |
| weiblich | gehen tandem | 50 | 54 | 1,32 | 6,61 | 1,55 | 7,74 |
| weiblich | gehen tandem | 55 | 59 | 1,70 | 8,51 | 1,76 | 8,78 |
| weiblich | gehen tandem | 60 | 64 | 1,41 | 7,03 | 1,43 | 7,16 |
| weiblich | gehen tandem | 65 | 69 | 1,35 | 6,76 | 1,57 | 7,84 |
| weiblich | gehen tandem | 70 | 74 | 1,45 | 7,24 | 1,67 | 8,37 |
| weiblich | gehen tandem | 75 | 79 | 1,58 | 7,92 | 2,00 | 10,00 |
| weiblich | gehen tandem | 80 | 84 | 1,76 | 8,79 | 1,43 | 7,14 |
| weiblich | gehen tandem | 85 | 90 | 1,87 | 9,35 | 1,32 | 6,60 |
| weiblich | stehen Schaumstoff AO | 15 | 19 | 0,30 | 1,49 | 0,27 | 1,34 |
| weiblich | stehen Schaumstoff AO | 20 | 24 | 0,28 | 1,39 | 0,36 | 1,81 |
| weiblich | stehen Schaumstoff AO | 25 | 29 | 0,27 | 1,35 | 0,44 | 2,19 |
| weiblich | stehen Schaumstoff AO | 30 | 34 | 0,30 | 1,49 | 0,22 | 1,12 |
| weiblich | stehen Schaumstoff AO | 35 | 39 | 0,29 | 1,45 | 0,34 | 1,69 |
| weiblich | stehen Schaumstoff AO | 40 | 44 | 0,25 | 1,25 | 0,30 | 1,51 |
| weiblich | stehen Schaumstoff AO | 45 | 49 | 0,33 | 1,67 | 0,40 | 1,98 |
| weiblich | stehen Schaumstoff AO | 50 | 54 | 0,31 | 1,56 | 0,29 | 1,44 |
| weiblich | stehen Schaumstoff AO | 55 | 59 | 0,34 | 1,69 | 0,39 | 1,94 |
| weiblich | stehen Schaumstoff AO | 60 | 64 | 0,18 | 0,88 | 0,22 | 1,10 |
| weiblich | stehen Schaumstoff AO | 65 | 69 | 0,21 | 1,03 | 0,27 | 1,36 |
| weiblich | stehen Schaumstoff AO | 70 | 74 | 0,21 | 1,06 | 0,25 | 1,27 |
| weiblich | stehen Schaumstoff AO | 75 | 79 | 0,22 | 1,09 | 0,27 | 1,37 |
| weiblich | stehen Schaumstoff AO | 80 | 84 | 0,22 | 1,10 | 0,18 | 0,89 |
| weiblich | stehen Schaumstoff AO | 85 | 90 | 0,24 | 1,22 | 0,18 | 0,89 |

| Geschlecht | Aufgabe | von | bis | von | | | bis |
|---|---|---|---|---|---|---|---|
| weiblich | stehen Schaumstoff AG | 15 | 19 | 0,33 | 1,65 | 0,45 | 2,27 |
| weiblich | stehen Schaumstoff AG | 20 | 24 | 0,25 | 1,25 | 0,34 | 1,70 |
| weiblich | stehen Schaumstoff AG | 25 | 29 | 0,29 | 1,47 | 0,49 | 2,44 |
| weiblich | stehen Schaumstoff AG | 30 | 34 | 0,34 | 1,68 | 0,28 | 1,39 |
| weiblich | stehen Schaumstoff AG | 35 | 39 | 0,28 | 1,39 | 0,32 | 1,62 |
| weiblich | stehen Schaumstoff AG | 40 | 44 | 0,26 | 1,28 | 0,34 | 1,72 |
| weiblich | stehen Schaumstoff AG | 45 | 49 | 0,33 | 1,67 | 0,41 | 2,06 |
| weiblich | stehen Schaumstoff AG | 50 | 54 | 0,41 | 2,05 | 0,39 | 1,94 |
| weiblich | stehen Schaumstoff AG | 55 | 59 | 0,23 | 1,16 | 0,26 | 1,30 |
| weiblich | stehen Schaumstoff AG | 60 | 64 | 0,24 | 1,20 | 0,31 | 1,53 |
| weiblich | stehen Schaumstoff AG | 65 | 69 | 0,28 | 1,41 | 0,38 | 1,91 |
| weiblich | stehen Schaumstoff AG | 70 | 74 | 0,30 | 1,49 | 0,33 | 1,67 |
| weiblich | stehen Schaumstoff AG | 75 | 79 | 0,29 | 1,46 | 0,39 | 1,96 |
| weiblich | stehen Schaumstoff AG | 80 | 84 | 0,38 | 1,88 | 0,35 | 1,76 |
| weiblich | stehen Schaumstoff AG | 85 | 90 | 0,29 | 1,43 | 0,29 | 1,43 |
| weiblich | stehen Schaumstoff 1 Bein | 15 | 19 | 0,38 | 1,88 | 0,46 | 2,31 |
| weiblich | stehen Schaumstoff 1 Bein | 20 | 24 | 0,31 | 1,57 | 0,55 | 2,73 |
| weiblich | stehen Schaumstoff 1 Bein | 25 | 29 | 0,41 | 2,03 | 0,55 | 2,73 |
| weiblich | stehen Schaumstoff 1 Bein | 30 | 34 | 0,58 | 2,91 | 0,51 | 2,55 |
| weiblich | stehen Schaumstoff 1 Bein | 35 | 39 | 0,68 | 3,40 | 0,90 | 4,50 |
| weiblich | stehen Schaumstoff 1 Bein | 40 | 44 | 0,59 | 2,94 | 0,58 | 2,92 |
| weiblich | stehen Schaumstoff 1 Bein | 45 | 49 | 0,48 | 2,39 | 0,56 | 2,79 |
| weiblich | stehen Schaumstoff 1 Bein | 50 | 54 | 0,85 | 4,24 | 0,82 | 4,09 |
| weiblich | stehen Schaumstoff 1 Bein | 55 | 60 | 0,76 | 3,81 | 0,72 | 3,58 |

| Geschlecht | Aufgabe | von | bis | von | bis | von | bis |
|---|---|---|---|---|---|---|---|
| weiblich | gehen tandem Schaumstoff | 15 | 19 | 2,02 | 10,11 | 2,28 | 11,39 |
| weiblich | gehen tandem Schaumstoff | 20 | 24 | 1,78 | 8,92 | 2,73 | 13,67 |
| weiblich | gehen tandem Schaumstoff | 25 | 29 | 1,92 | 9,61 | 2,10 | 10,48 |
| weiblich | gehen tandem Schaumstoff | 30 | 34 | 2,34 | 11,72 | 2,17 | 10,85 |
| weiblich | gehen tandem Schaumstoff | 35 | 39 | 1,82 | 9,11 | 2,04 | 10,22 |
| weiblich | gehen tandem Schaumstoff | 40 | 44 | 1,58 | 7,89 | 1,88 | 9,39 |
| weiblich | gehen tandem Schaumstoff | 45 | 49 | 1,73 | 8,66 | 1,95 | 9,74 |
| weiblich | gehen tandem Schaumstoff | 50 | 54 | 1,90 | 9,48 | 2,11 | 10,57 |
| weiblich | gehen tandem Schaumstoff | 55 | 59 | 1,83 | 9,16 | 2,09 | 10,44 |
| weiblich | gehen tandem Schaumstoff | 60 | 64 | 1,61 | 8,07 | 2,01 | 10,04 |
| weiblich | gehen tandem Schaumstoff | 65 | 69 | 1,71 | 8,55 | 2,14 | 10,69 |
| weiblich | gehen tandem Schaumstoff | 70 | 74 | 1,78 | 8,91 | 2,21 | 11,05 |
| weiblich | gehen tandem Schaumstoff | 75 | 79 | 1,86 | 9,28 | 2,18 | 10,92 |
| weiblich | gehen tandem Schaumstoff | 80 | 84 | 2,79 | 13,95 | 1,91 | 9,56 |
| weiblich | gehen tandem Schaumstoff | 85 | 90 | 1,97 | 9,83 | 1,56 | 7,80 |
| weiblich | gehen Kopfkreisen | 15 | 19 | 1,88 | 9,41 | 1,77 | 8,87 |
| weiblich | gehen Kopfkreisen | 20 | 24 | 1,98 | 9,89 | 2,21 | 11,05 |
| weiblich | gehen Kopfkreisen | 25 | 29 | 1,85 | 9,23 | 2,28 | 11,41 |
| weiblich | gehen Kopfkreisen | 30 | 34 | 2,16 | 10,80 | 2,07 | 10,33 |
| weiblich | gehen Kopfkreisen | 35 | 39 | 1,65 | 8,24 | 2,13 | 10,64 |
| weiblich | gehen Kopfkreisen | 40 | 44 | 1,64 | 8,18 | 1,95 | 9,74 |
| weiblich | gehen Kopfkreisen | 45 | 49 | 1,23 | 6,13 | 1,54 | 7,68 |
| weiblich | gehen Kopfkreisen | 50 | 54 | 1,46 | 7,32 | 1,95 | 9,77 |
| weiblich | gehen Kopfkreisen | 55 | 59 | 1,83 | 9,13 | 2,02 | 10,08 |
| weiblich | gehen Kopfkreisen | 60 | 64 | 1,37 | 6,84 | 1,63 | 8,15 |
| weiblich | gehen Kopfkreisen | 65 | 69 | 1,39 | 6,93 | 1,83 | 9,13 |
| weiblich | gehen Kopfkreisen | 70 | 74 | 1,44 | 7,21 | 1,73 | 8,64 |
| weiblich | gehen Kopfkreisen | 75 | 79 | 1,73 | 8,67 | 1,97 | 9,83 |
| weiblich | gehen Kopfkreisen | 80 | 84 | 1,74 | 8,69 | 1,40 | 6,98 |
| weiblich | gehen Kopfkreisen | 85 | 90 | 1,83 | 9,17 | 1,37 | 6,87 |
| weiblich | gehen Kopfnicken | 15 | 19 | 2,57 | 12,86 | 1,82 | 9,12 |
| weiblich | gehen Kopfnicken | 20 | 24 | 2,12 | 10,61 | 2,12 | 10,58 |
| weiblich | gehen Kopfnicken | 25 | 29 | 2,05 | 10,25 | 2,48 | 12,39 |
| weiblich | gehen Kopfnicken | 30 | 34 | 2,04 | 10,20 | 2,31 | 11,57 |
| weiblich | gehen Kopfnicken | 35 | 39 | 1,59 | 7,97 | 2,32 | 11,61 |
| weiblich | gehen Kopfnicken | 40 | 44 | 1,71 | 8,54 | 2,03 | 10,16 |
| weiblich | gehen Kopfnicken | 45 | 49 | 1,64 | 8,22 | 2,13 | 10,66 |
| weiblich | gehen Kopfnicken | 50 | 54 | 1,67 | 8,36 | 2,14 | 10,70 |
| weiblich | gehen Kopfnicken | 55 | 59 | 1,65 | 8,23 | 2,02 | 10,11 |
| weiblich | gehen Kopfnicken | 60 | 64 | 1,36 | 6,80 | 1,74 | 8,69 |
| weiblich | gehen Kopfnicken | 65 | 69 | 1,42 | 7,09 | 2,07 | 10,37 |
| weiblich | gehen Kopfnicken | 70 | 74 | 1,46 | 7,28 | 1,80 | 9,02 |
| weiblich | gehen Kopfnicken | 75 | 79 | 1,56 | 7,80 | 2,07 | 10,34 |
| weiblich | gehen Kopfnicken | 80 | 84 | 1,94 | 9,68 | 1,55 | 7,75 |
| weiblich | gehen Kopfnicken | 85 | 90 | 1,87 | 9,35 | 1,39 | 6,93 |
| weiblich | gehen AG | 15 | 19 | 2,09 | 10,44 | 1,89 | 9,45 |
| weiblich | gehen AG | 20 | 24 | 2,36 | 11,78 | 2,30 | 11,49 |
| weiblich | gehen AG | 25 | 29 | 1,52 | 7,58 | 2,37 | 11,84 |
| weiblich | gehen AG | 30 | 34 | 1,98 | 9,92 | 2,41 | 12,06 |
| weiblich | gehen AG | 35 | 39 | 1,95 | 9,74 | 2,48 | 12,39 |
| weiblich | gehen AG | 40 | 44 | 1,61 | 8,07 | 1,71 | 8,57 |
| weiblich | gehen AG | 45 | 49 | 1,49 | 7,47 | 1,75 | 8,74 |
| weiblich | gehen AG | 50 | 54 | 1,54 | 7,71 | 1,96 | 9,81 |
| weiblich | gehen AG | 55 | 59 | 1,51 | 7,53 | 1,66 | 8,32 |
| weiblich | gehen AG | 60 | 64 | 1,34 | 6,68 | 1,75 | 8,77 |
| weiblich | gehen AG | 65 | 69 | 1,32 | 6,60 | 1,93 | 9,64 |
| weiblich | gehen AG | 70 | 74 | 1,29 | 6,45 | 1,66 | 8,31 |
| weiblich | gehen AG | 75 | 79 | 1,28 | 6,38 | 2,01 | 10,04 |
| weiblich | gehen AG | 80 | 84 | 1,61 | 8,03 | 1,49 | 7,47 |
| weiblich | gehen AG | 85 | 90 | 1,76 | 8,79 | 1,33 | 6,65 |
| weiblich | gehen über Hindernisse | 15 | 19 | 5,13 | 25,67 | 5,29 | 26,43 |
| weiblich | gehen über Hindernisse | 20 | 24 | 4,41 | 22,03 | 6,52 | 32,62 |
| weiblich | gehen über Hindernisse | 25 | 29 | 3,14 | 15,70 | 4,44 | 22,18 |
| weiblich | gehen über Hindernisse | 30 | 34 | 3,62 | 18,09 | 4,28 | 21,38 |
| weiblich | gehen über Hindernisse | 35 | 39 | 2,80 | 13,98 | 5,27 | 26,35 |
| weiblich | gehen über Hindernisse | 40 | 44 | 3,01 | 15,06 | 3,39 | 16,93 |
| weiblich | gehen über Hindernisse | 45 | 49 | 2,80 | 13,99 | 4,25 | 21,25 |
| weiblich | gehen über Hindernisse | 50 | 54 | 2,72 | 13,62 | 4,71 | 23,53 |
| weiblich | gehen über Hindernisse | 55 | 59 | 3,80 | 18,98 | 4,13 | 20,66 |
| weiblich | gehen über Hindernisse | 60 | 64 | 4,11 | 20,54 | 2,99 | 14,97 |
| weiblich | gehen über Hindernisse | 65 | 69 | 4,30 | 21,51 | 3,35 | 16,74 |
| weiblich | gehen über Hindernisse | 70 | 74 | 3,95 | 19,76 | 3,67 | 18,37 |
| weiblich | gehen über Hindernisse | 75 | 79 | 4,06 | 20,28 | 3,69 | 18,44 |
| weiblich | gehen über Hindernisse | 80 | 84 | 3,45 | 17,25 | 4,29 | 21,44 |
| weiblich | gehen über Hindernisse | 85 | 90 | 3,40 | 16,98 | 4,25 | 21,26 |
| weiblich | gehen AO | 15 | 19 | 2,37 | 11,84 | 2,69 | 13,47 |
| weiblich | gehen AO | 20 | 24 | 2,71 | 13,53 | 2,87 | 14,35 |
| weiblich | gehen AO | 25 | 29 | 2,23 | 11,16 | 2,76 | 13,78 |
| weiblich | gehen AO | 30 | 34 | 2,24 | 11,20 | 2,58 | 12,89 |
| weiblich | gehen AO | 35 | 39 | 2,02 | 10,08 | 2,43 | 12,13 |
| weiblich | gehen AO | 40 | 44 | 1,53 | 7,65 | 2,10 | 10,49 |
| weiblich | gehen AO | 45 | 49 | 1,92 | 9,58 | 2,25 | 11,25 |
| weiblich | gehen AO | 50 | 54 | 2,01 | 10,06 | 2,77 | 13,84 |
| weiblich | gehen AO | 55 | 59 | 1,90 | 9,50 | 2,13 | 10,67 |
| weiblich | gehen AO | 60 | 64 | 1,60 | 7,98 | 1,99 | 9,96 |
| weiblich | gehen AO | 65 | 69 | 1,49 | 7,45 | 2,29 | 11,46 |
| weiblich | gehen AO | 70 | 74 | 1,50 | 7,51 | 2,10 | 10,52 |
| weiblich | gehen AO | 75 | 79 | 1,45 | 7,27 | 2,03 | 10,14 |
| weiblich | gehen AO | 80 | 84 | 1,96 | 9,78 | 1,58 | 7,91 |
| weiblich | gehen AO | 85 | 90 | 2,20 | 11,00 | 1,54 | 7,70 |
| weiblich | hinsetzen | 20 | 24 | 2,72 | 13,62 | 3,44 | 17,22 |
| weiblich | hinsetzen | 25 | 29 | 4,97 | 24,84 | 4,37 | 21,86 |
| weiblich | hinsetzen | 30 | 34 | 5,13 | 25,67 | 3,90 | 19,49 |
| weiblich | hinsetzen | 35 | 39 | 3,91 | 19,53 | 5,29 | 26,43 |
| weiblich | hinsetzen | 40 | 44 | 5,63 | 28,15 | 5,17 | 25,86 |
| weiblich | hinsetzen | 45 | 49 | 5,42 | 27,08 | 3,66 | 18,29 |
| weiblich | hinsetzen | 50 | 54 | 5,72 | 28,60 | 6,29 | 31,43 |
| weiblich | hinsetzen | 55 | 59 | 6,01 | 30,06 | 7,21 | 36,07 |
| weiblich | hinsetzen | 60 | 64 | 5,83 | 29,15 | 4,98 | 24,89 |
| weiblich | hinsetzen | 65 | 69 | 4,99 | 24,94 | 5,79 | 28,95 |
| weiblich | hinsetzen | 70 | 74 | 4,33 | 21,66 | 4,89 | 24,43 |
| weiblich | hinsetzen | 75 | 79 | 5,26 | 26,29 | 5,28 | 26,38 |
| weiblich | hinsetzen | 80 | 84 | 5,96 | 29,78 | 5,54 | 27,72 |
| weiblich | hinsetzen | 85 | 90 | 5,95 | 29,73 | 5,27 | 26,33 |
| weiblich | aufstehen | 20 | 24 | 2,03 | 10,16 | 3,69 | 18,43 |
| weiblich | aufstehen | 25 | 29 | 4,94 | 24,70 | 3,78 | 18,89 |
| weiblich | aufstehen | 30 | 34 | 5,59 | 27,97 | 3,36 | 16,80 |
| weiblich | aufstehen | 35 | 39 | 4,46 | 22,32 | 4,99 | 24,93 |
| weiblich | aufstehen | 40 | 44 | 4,96 | 24,78 | 5,35 | 26,77 |
| weiblich | aufstehen | 45 | 49 | 4,45 | 22,23 | 3,66 | 18,30 |
| weiblich | aufstehen | 50 | 54 | 5,83 | 29,17 | 6,14 | 30,70 |
| weiblich | aufstehen | 55 | 59 | 5,64 | 28,19 | 6,94 | 34,71 |
| weiblich | aufstehen | 60 | 64 | 5,06 | 25,31 | 5,21 | 26,06 |
| weiblich | aufstehen | 65 | 69 | 5,60 | 28,02 | 6,07 | 30,33 |
| weiblich | aufstehen | 70 | 74 | 5,16 | 25,80 | 6,19 | 30,93 |
| weiblich | aufstehen | 75 | 79 | 5,40 | 27,02 | 5,99 | 29,95 |
| weiblich | aufstehen | 80 | 84 | 3,30 | 16,49 | 3,78 | 18,90 |
| weiblich | aufstehen | 85 | 90 | 3,23 | 16,16 | 3,21 | 16,05 |

## Patentansprüche

1. Vorrichtung zur Durchführung eines adaptiven Gleichgewichtstrainings umfassend
a) eine Prozessoreinheit,
b) mindestens einen Sensor zur Messung der Veränderung einer Körperposition und/oder eines Bewegungsmusters des Trägers der Vorrichtung, welcher Messdaten an die Prozessoreinheit überträgt, wobei die Prozessoreinheit konfiguriert ist aus den Messdaten des mindestens einen Sensors Schwankungswerte zu bestimmen und wobei der mindestens eine Sensor und die Prozessoreinheit in einem gemeinsamen Gehäuse vorliegen
c) ein Aufforderungsmodul, wobei auf der Prozessoreinheit Informationen über ein Übungsprogramm umfassend mehrere Bewegungsmuster gespeichert vorliegt und die Prozessoreinheit dazu konfiguriert ist mittels des Aufforderungsmodules eine Ausgabe von Anweisungen für das Übungsprogramm umfassend mehrere Bewegungsmuster an den Träger der Vorrichtung vorzunehmen
d) mindestens einen Signalgeber zur Ausgabe eines Feedbacksignals, wobei die Prozesseinheit konfiguriert ist, während eines Übungsprogrammes mittels des Signalgebers ein Feedbacksignal zu erzeugen, falls die Schwankungswerte eine Feedbackschwelle überschreiten,
wobei die Prozessoreinheit dazu konfiguriert ist während einer ersten Durchführung eines Übungsprogrammes die Schwankungswerte für die Bewegungsmuster zu speichern und anhand der gespeicherten Schwankungswerte die Feedbackschwelle für eine spätere zweite Durchführung eines Übungsprogramms zu berechnen, und während der zweiten Durchführung des Übungsprogrammes ein Feedbacksignal mittels des Signalgebers zu erzeugen, falls die Schwankungswerte die berechnete Feedbackschwelle überschreiten, wobei ein Zählerelement eine Anzahl von Durchführungen des Übungsprogrammes zählt und dadurch festgestellt wird, ob die Schwankungswerte zu einer ersten oder einer zweiten Durchführung des Übungsprogrammes gehören.
wobei die Veränderung einer Körperposition und/oder eines Bewegungsmusters des Trägers der Vorrichtung dreidimensional im Raum als Veränderung der Winkelgeschwindigkeit und/oder der Beschleunigung von Vorwärts-, Rückwärts- und Seitwärtsbewegungen des Trägers nahe am Körperschwerpunkt des Trägers bestimmt wird und die Schwankungswerte eine Schwankungsauslenkung, eine Schwankungsgeschwindigkeit oder Schwankungsbeschleunigung umfassen.

2. Vorrichtung gemäß einem der vorherigen Ansprüche
**dadurch gekennzeichnet, dass**
die Schwankungswerte eine Winkelgeschwindigkeit in Bezug auf Vorwärts-, Rückwärts- und Seitwärtsbewegungen des Trägers entsprechen und für jede der Vorwärts-, Rückwärts- und Seitwärtsbewegungen eine Feedbackschwelle berechnet wird.

3. Vorrichtung gemäß einem der vorherigen Ansprüche
**dadurch gekennzeichnet, dass**
auf der Prozessoreinheit maximale Normwerte von Schwankungswerten für jeweilige Bewegungsmuster gespeichert vorliegen, wobei bevorzugt die Prozessoreinheit konfiguriert ist während der ersten Durchführung des Übungsprogrammes die Feedbackschwelle anhand der maximalen Normwerte festzulegen.

4. Vorrichtung gemäß einem der vorherigen Anspruch
**dadurch gekennzeichnet, dass**
die Prozessoreinheit konfiguriert ist während einer zweiten Durchführung des Übungsprogrammes die Feedbackschwelle anhand der Schwankungswerte einer vorherigen ersten Durchführung eines Übungsprogrammes zu berechnen, wobei die Feedbackschwelle bevorzugt einem Wert zwischen dem Median 40 aller Schwankungswerte und dem Median 80 aller Schwankungswerte, besonders bevorzugt dem Median 60 der Schwankungswerte entspricht.

5. Vorrichtung gemäß dem vorherigen Anspruch
**dadurch gekennzeichnet, dass**
die Prozessoreinheit konfiguriert ist zu gewährleisten, dass die Feedbackschwelle stets zwischen 50% und 200% der maximalen Normwerte liegt, indem die Feedbackschwelle auf 50% der maximalen Normwerte hochgesetzt wird, falls die zuvor berechnete Feedbackschwelle weniger als 50% der maximalen Normwerte betrug und die Feedbackschwelle auf einen Wert von 200% der maximalen Normwerte heruntergesetzt wird, falls die zuvor berechnete Feedbackschwelle mehr als 200% der maximalen Normwerte betrug.

6. Vorrichtung gemäß einem oder mehreren der vorherigen Ansprüche
**dadurch gekennzeichnet, dass**
der mindestens eine Signalgeber ausgesucht ist aus der Gruppe bestehend aus einem Lautsprecher, einer optischen Anzeige, einer Elektrode und einem Vibrationsstimulator, wobei das Feedbacksignal bevorzugt ausgewählt ist aus der Gruppe bestehend aus einem visuellen, einem taktilem, einem vibrotaktilem, einem galvanischem und einem gustatorischen Reiz.

7. Vorrichtung gemäß einem oder mehreren der vorherigen Ansprüche
**dadurch gekennzeichnet, dass**
das Feedbacksignal in mehreren Intensitätsstufen ausgegeben werden kann, wobei bevorzugt eine einfache Überschreitung der Feedbackschwelle eine erste Stufe auslöst, eine 1,5fache Überschreitung der Feedbackschwelle eine zweite Stufe und eine zweifache Überschreitung der Feedbackschwelle eine dritte Stufe.

8. Vorrichtung einem oder mehreren der vorherigen Ansprüche
**dadurch gekennzeichnet, dass**
die Ausgabe der Anweisungen für ein Übungsprogramm visuell oder akustisch erfolgt.

9. Vorrichtung einem oder mehreren der vorherigen Ansprüche
**dadurch gekennzeichnet, dass**
die Vorrichtung ein Eingabemodul zur Vornahme von Eingaben durch den Träger der Vorrichtung umfasst, welche zur Auswertung an die Prozessoreinheit übermittelt werden, wobei die Eingaben bevorzugt Spracheingaben, Gesteneingaben, Geräuscheingaben oder Tasteingaben umfassen und/oder wobei ein Träger der Vorrichtung bevorzugt mittels der Eingaben auf die Anweisungen zur Durchführung eines Übungsprograms reagieren kann, in dem der Träger bevorzugt eine Zustimmung, Ablehnung oder Unterbrechung signalisiert, und die Prozessoreinheit dafür konfiguriert ist, den Verlauf der Durchführung des Übungsprogrammes anhand der Eingaben anzupassen.

10. Vorrichtung einem oder mehreren der vorherigen Ansprüche
**dadurch gekennzeichnet, dass**
die Prozesseinheit dazu konfiguriert ist während der Durchführung eines ersten Übungsprogrammes die Schwankungswerte für die Bewegungsmuster zu speichern und anhand der gespeicherte Schwankungswerte Übungen für ein späteres zweites Übungsprogramm auszuwählen.

11. Vorrichtung gemäß einem der vorherigen Ansprüche
**dadurch gekennzeichnet, dass**
eine Auswahl der Übungen für das Übungsprogramm anhand eines Vergleichs der Schwankungswerte bei der Durchführung des ersten Übungsprogrammes mit Normwerten erfolgt, wobei bevorzugt diejenigen Übungen ausgewählt werden bei denen ein Median 50 der gemessenen Schwankungswerte höher ist als der Median 95 der Median 50 Werte aller Probanden (Normwerte) für eine jeweilige Alters- oder Geschlechtsgruppe.

12. Vorrichtung gemäß einem oder mehreren der vorherigen Ansprüche,
**dadurch gekennzeichnet, dass**
bei einer Durchführung des Übungsprogramms anhand einer Ähnlichkeitsanalyse mit einer vorhergehenden Durchführung des Übungsprogrammes eine Rückmeldung über die korrekte Ausführung der Übungen des Übungsprogrammes ausgegeben wird.

13. Vorrichtung gemäß einem oder mehreren der vorherigen Ansprüche
**dadurch gekennzeichnet, dass**
mindestens ein Sensor zur Messung der Veränderung einer Körperposition und/oder eines Bewegungsmusters des Trägers der Vorrichtung Gyrometer und/oder Beschleunigungssensoren umfasst, wobei der mindestens einen Sensor zur Messung der Veränderung einer Körperposition und/oder eines Bewegungsmusters des Trägers bevorzugt mehrere orthogonal zueinanderstehende Gyrometer und/oder Beschleunigungssensoren aufweist, welche die Veränderung der Winkelgeschwindigkeit und/oder die Beschleunigung von Vorwärts-, Rückwärts- und Seitwärtsbewegungen des Körpers bestimmen.

14. Kit zur Durchführung eines adaptiven Gleichgewichtstrainings umfassend eine
i. Vorrichtung umfassend
a. Prozessoreinheit
b. mindestens einen Sensor zur Messung der Veränderung einer Körperposition und/oder eines Bewegungsmusters des Trägers der Vorrichtung, welcher Messdaten an die Prozessoreinheit übertragen kann,
c. ein Aufforderungsmodul zur Ausgabe von Anweisungen für ein Übungsprogramm umfassend mehrere Bewegungsmuster an den Träger der Vorrichtung
d. mindestens einen Signalgeber zur Ausgabe eines Feedbacksignals
ii. ein Computerprogrammprodukt zur Installation auf der Prozessoreinheit der Vorrichtung, welches Befehle umfasst zur
a. Ausgabe von Anweisungen für ein Übungsprogramm umfassend mehrere Bewegungsmuster an den Träger der Vorrichtung,
b. Messung der Veränderung einer Körperposition und/oder eines Bewegungsmusters des Trägers der Vorrichtung durch mindestens einen Sensor während der Durchführung des Übungsprogramms und Bestimmung der Schwankungswerte aus den Messdaten
c. Ausgabe eines Feedbacksignals durch den Signalgeber während des Übungsprogrammes, falls die Schwankungswerte eine Feedbackschwelle überschreiten, wobei während der ersten Durchführung des Übungsprogrammes die Schwankungswerte für die Bewegungsmuster gespeichert werden und die Feedbackschwelle für die zweite Durchführung des Übungsprogrammes anhand der gespeicherten Schwankungswerte berechnet werden, und während der zweiten Durchführung des Übungsprogrammes das Feedbacksignal mittels des Signalgebers erzeugt wird, falls die Schwankungswerte die berechnete Feedbackschwelle überschreiten, wobei ein Zählerelement eine Anzahl von Durchführungen des Übungsprogrammes zählt und dadurch festgestellt wird, ob die Schwankungswerte zu einer ersten oder einer zweiten Durchführung des Übungsprogrammes gehören.

15. Computerprogrammprodukt zur Installation auf einer Vorrichtung umfassend
a. eine Prozessoreinheit
b. mindestens einen Sensor zur Messung der Veränderung einer Körperposition und/oder eines Bewegungsmusters des Trägers der Vorrichtung, welcher Messdaten an die Prozessoreinheit übertragen kann,
c. ein Aufforderungsmodul zur Ausgabe von Anweisungen für ein Übungsprogramm umfassend mehrere Bewegungsmuster an den Träger der Vorrichtung
d. mindestens einen Signalgeber zur Ausgabe eines Feedbacksignals
wobei das Computerprogrammprodukt Befehle aufweist zur Durchführung eines adaptiven Gleichgewichtstrainings umfassend die Verfahrensschritte
a. Ausgabe von Anweisungen für ein Übungsprogramm umfassend mehrere Bewegungsmuster an einen Träger der Vorrichtung,
b. Messung der Veränderung einer Körperposition und/oder eines Bewegungsmusters des Trägers der Vorrichtung durch mindestens einen Sensor während der Durchführung des Übungsprogramms und Bestimmung der Schwankungswerte aus den Messdaten
c. Ausgabe eines Feedbacksignals durch den Signalgeber während des Übungsprogrammes, falls die Schwankungswerte eine Feedbackschwelle überschreiten, wobei während der ersten Durchführung des Übungsprogrammes die Schwankungswerte für die Bewegungsmuster gespeichert werden und die Feedbackschwelle für die zweite Durchführung des Übungsprogrammes anhand der gespeicherten Schwankungswerte berechnet werden und während der zweiten Durchführung des Übungsprogrammes das Feedbacksignal mittels des Signalgebers erzeugt wird, falls die Schwankungswerte die berechnete Feedbackschwelle überschreiten, wobei ein Zählerelement eine Anzahl von Durchführungen des Übungsprogrammes zählt und dadurch festgestellt wird, ob die Schwankungswerte zu einer ersten oder einer zweiten Durchführung des Übungsprogrammes gehören.

## Claims

1. A device for performing adaptive balance training, comprising
a) a processor unit,
b) at least one sensor for measuring the change in a body position and/or a movement pattern of the wearer of the device, which sensor transmits measurement data to the processor unit, wherein the processor unit is configured to determine fluctuation values from the measurement data of the at least one sensor, and wherein the at least one sensor and the processor unit are present in a common housing
c) a request module, wherein information about an exercise program comprising a plurality of movement patterns is stored on the processor unit, and the processor unit is configured to use the request module to output instructions for the exercise program comprising a plurality of movement patterns to the wearer of the device
d) at least one signal generator for outputting a feedback signal, wherein the process unit is configured to generate a feedback signal by means of the signal generator during an exercise program if the fluctuation values exceed a feedback threshold,
wherein the processor unit is configured to store the fluctuation values for the movement patterns during a first performance of an exercise program and to calculate the feedback threshold for a later second performance of an exercise program on the basis of the stored fluctuation values, and to generate a feedback signal by means of the signal generator during the second performance of the exercise program if the fluctuation values exceed the calculated feedback threshold, wherein a counter element counts a number of performances of the exercise program and thereby determines whether the fluctuation values belong to a first or a second performance of the exercise program,
wherein the change in a body position and/or a movement pattern of the wearer of the device is determined three-dimensionally in space as a change in the angular velocity and/or the acceleration of forward, backward, and sideways movements of the wearer close to the center of gravity of the wearer, and the fluctuation values comprise a fluctuation deflection, a fluctuation velocity, or a fluctuation acceleration.

2. The device according to any one of the preceding claims,
**characterized in that**
the fluctuation values correspond to an angular velocity with respect to forward, backward, and sideways movements of the wearer and a feedback threshold is calculated for each of the forward, backward, and sideways movements.

3. The device according to any one of the preceding claims,
**characterized in that**
maximum standard values of fluctuation values for respective movement patterns are stored on the processor unit, wherein the processor unit is preferably configured to determine the feedback threshold on the basis of the maximum standard values during the first performance of the exercise program.

4. The device according to any one of the preceding claims,
**characterized in that**
the processor unit is configured to calculate the feedback threshold on the basis of the fluctuation values of a previous first performance of an exercise program during a second performance of the exercise program, wherein the feedback threshold preferably corresponds to a value between the median 40 of all fluctuation values and the median 80 of all fluctuation values, particularly preferably the median 60 of the fluctuation values.

5. The device according to the preceding claim,
**characterized in that**
the processor unit is configured to ensure that the feedback threshold is always between 50% and 200% of the maximum standard values by raising the feedback threshold to 50% of the maximum standard values if the previously calculated feedback threshold was less than 50% of the maximum standard values and lowering the feedback threshold to a value of 200% of the maximum standard values if the previously calculated feedback threshold was more than 200% of the maximum standard values.

6. The device according to any one or more of the preceding claims,
**characterized in that**
the at least one signal generator is selected from the group consisting of a loudspeaker, an optical display, an electrode, and a vibration stimulator, wherein the feedback signal is preferably selected from the group consisting of a visual, a tactile, a vibro-tactile, a galvanic, and a gustatory stimulus.

7. The device according to any one or more of the preceding claims,
**characterized in that**
the feedback signal can be output in a plurality of intensity stages, wherein preferably a simple exceeding of the feedback threshold triggers a first stage, a 1.5 times exceeding of the feedback threshold triggers a second stage, and a two times exceeding of the feedback threshold triggers a third stage.

8. The device according to any one or more of the preceding claims,
**characterized in that**
the instructions for an exercise program are outputted visually or acoustically.

9. The device according to any one or more of the preceding claims,
**characterized in that**
the device comprises an input module for making inputs by the wearer of the device, which inputs are transmitted to the processor unit for evaluation, wherein the inputs preferably comprise voice inputs, gesture inputs, noise inputs, or key inputs, and/or wherein a wearer of the device can react preferably by means of the inputs to the instructions for performing an exercise program, in which the wearer preferably signals an approval, rejection, or interruption, and the processor unit is configured to adapt the course of performing the exercise program on the basis of the inputs.

10. The device according to any one or more of the preceding claims,
**characterized in that**
the process unit is configured to store the fluctuation values for the movement patterns during the performance of a first exercise program and to select exercises for a later second exercise program on the basis of the stored fluctuation values.

11. The device according to any one of the preceding claims,
**characterized in that**
a selection of the exercises for the exercise program is made on the basis of a comparison of the fluctuation values during the performance of the first exercise program with standard values, wherein preferably those exercises are selected in which a median of 50 of the measured fluctuation values is higher than the median of 95 of the median of 50 values of all subjects (standard values) for a respective age or gender group.

12. The device according to any one or more of the preceding claims,
**characterized in that**
when the exercise program is performed on the basis of a similarity analysis with a previous performance of the exercise program, a feedback is outputted about the correct performance of the exercises of the exercise program.

13. The device according to any one or more of the preceding claims,
**characterized in that**
at least one sensor for measuring the change in a body position and/or a movement pattern of the wearer of the device comprises gyrometers and/or
acceleration sensors, wherein the at least one sensor for measuring the change in a body position and/or a movement pattern of the wearer preferably has a plurality of gyrometers and/or acceleration sensors which are orthogonal to one another and which determine the change in the angular velocity and/or the acceleration of forward, backward, and sideways movements of the body.

14. A kit for performing adaptive balance training, comprising a
i. device, comprising
a. processor unit
b. at least one sensor for measuring the change in a body position and/or a movement pattern of the wearer of the device, which sensor can transmit measurement data to the processor unit,
c. a request module for outputting instructions for an exercise program comprising a plurality of movement patterns to the wearer of the apparatus
d. at least one signal generator for outputting a feedback signal
ii. a computer program product for installation on the processor unit of the device, which comprises commands for
a. ouputting instructions for an exercise program comprising a plurality of movement patterns to the wearer of the device,
b. measuring the change in a body position and/or a movement pattern of the wearer of the device by at least one sensor during the performance of the exercise program and determining the fluctuation values from the measurement data
c. outputting a feedback signal by the signal generator during the exercise program if the fluctuation values exceed a feedback threshold, wherein the fluctuation values for the movement patterns are stored during the first performance of the exercise program and the feedback threshold for the second performance of the exercise program is calculated on the basis of the stored fluctuation values, and during the second performance of the exercise program the feedback signal is generated by means of the signal generator if the fluctuation values exceed the calculated feedback threshold, wherein a counter element counts a number of performances of the exercise program and thereby determines whether the fluctuation values belong to a first or a second performance of the exercise program.

15. A computer program product for installation on a device, comprising
a. a processor unit
b. at least one sensor for measuring the change in a body position and/or a movement pattern of the wearer of the device, which sensor can transmit measurement data to the processor unit,
c. a request module for outputting instructions for an exercise program comprising a plurality of movement patterns to the wearer of the apparatus
d. at least one signal generator for outputting a feedback signal wherein the computer program product has commands for performing an adaptive equilibrium training comprising the method steps
a. ouputting instructions for an exercise program comprising a plurality of movement patterns to a wearer of the device,
b. measuring the change in a body position and/or a movement pattern of the wearer of the device by at least one sensor during the performance of the exercise program and determining the fluctuation values from the measurement data
c. outputting a feedback signal by the signal generator during the exercise program if the fluctuation values exceed a feedback threshold, wherein the fluctuation values for the movement patterns are stored during the first performance of the exercise program and the feedback threshold for the second performance of the exercise program is calculated on the basis of the stored fluctuation values, and during the second performance of the exercise program the feedback signal is generated by means of the signal generator if the fluctuation values exceed the calculated feedback threshold, wherein a counter element counts a number of performances of the exercise program and thereby determines whether the fluctuation values belong to a first or a second performance of the exercise program.

## Revendications

1. Dispositif destiné à la mise en œuvre d'un apprentissage d'équilibre adaptatif, comprenant
a) une unité de traitement,
b) au moins un capteur destiné à mesurer la modification d'une position corporelle et/ou d'un schéma de mouvement du porteur du dispositif, lequel transmet des données de mesure à l'unité de traitement, dans lequel l'unité de traitement est configurée pour déterminer, à partir des données de mesure de l'au moins un capteur, des valeurs de fluctuation, et dans lequel l'au moins un capteur et l'unité de traitement se trouvent dans un boîtier commun
c) un module d'invitation, dans lequel des informations relatives à un programme d'exercices comprenant plusieurs schémas de mouvement sont enregistrées dans l'unité de traitement, et l'unité de traitement est configurée pour, au moyen du module d'invitation, émettre à destination du porteur du dispositif des instructions relatives au programme d'exercices comprenant plusieurs schémas de mouvement
d) au moins un émetteur de signaux destiné à émettre un signal de retour d'information, dans lequel l'unité de traitement est configurée pour générer, au moyen de l'émetteur de signaux, un signal de retour d'information pendant un programme d'exercices si les valeurs de fluctuation dépassent un seuil de retour d'information,
dans lequel l'unité de traitement est configurée pour enregistrer, pendant une première exécution d'un programme d'exercices, les valeurs de fluctuation correspondant aux schémas de mouvement et pour calculer, sur la base des valeurs de fluctuation enregistrées, le seuil de retour d'information pour une seconde exécution ultérieure du programme d'exercices, et pour générer, pendant la seconde exécution du programme d'exercices, un signal de retour d'information au moyen de l'émetteur de signaux si les valeurs de fluctuation dépassent le seuil de retour d'information calculé, dans lequel un élément compteur compte un nombre d'exécutions du programme d'exercices et permet ainsi de déterminer si les valeurs de fluctuation appartiennent à une première ou à une seconde exécution du programme d'exercices,
dans lequel la modification d'une position corporelle et/ou d'un schéma de mouvement du porteur du dispositif est déterminée tridimensionnellement dans l'espace sous la forme d'une modification de la vitesse angulaire et/ou de l'accélération des mouvements vers l'avant, vers l'arrière et latéraux du porteur à proximité du centre de gravité du corps du porteur, et les valeurs de fluctuation comprennent un déplacement de fluctuation, une vitesse de fluctuation ou une accélération de fluctuation.

2. Dispositif selon l'une des revendications précédentes
**caractérisé en ce que**
les valeurs de fluctuation correspondent à une vitesse angulaire relative aux mouvements vers l'avant, vers l'arrière et latéraux du porteur, et **en ce qu'**un seuil de retour d'information est calculé pour chacun des mouvements vers l'avant, vers l'arrière et latéraux.

3. Dispositif selon l'une des revendications précédentes
**caractérisé en ce que**
des valeurs normales maximales des valeurs de fluctuation pour les différents schémas de mouvement sont enregistrées dans l'unité de traitement, dans lequel l'unité de traitement est de préférence configurée pour fixer, pendant la première exécution du programme d'exercices, le seuil de retour d'information sur la base des valeurs normales maximales.

4. Dispositif selon l'une des revendications précédentes
**caractérisé en ce que**
l'unité de traitement est configurée pour calculer, pendant une seconde exécution du programme d'exercices, le seuil de retour d'information sur la base des valeurs de fluctuation d'une première exécution antérieure du programme d'exercices, dans lequel le seuil de retour d'information correspond de préférence à une valeur comprise entre la médiane 40 de toutes les valeurs de fluctuation et la médiane 80 de toutes les valeurs de fluctuation, de manière davantage préférée à la médiane 60 des valeurs de fluctuation.

5. Dispositif selon la revendication précédente
**caractérisé en ce que**
l'unité de traitement est configurée pour garantir que le seuil de retour d'information est toujours compris entre 50 % et 200 % des valeurs de référence maximales, **en ce que** le seuil de retour d'information est porté à 50 % des valeurs de référence maximales si le seuil de retour d'information calculé précédemment est inférieur à 50 % des valeurs de référence maximales, et **en ce que** le seuil de retour d'information est abaissé à une valeur de 200 % des valeurs de référence maximales si le seuil de retour d'information calculé précédemment était supérieur à 200 % des valeurs de référence maximales.

6. Dispositif selon l'une ou plusieurs des revendications précédentes
**caractérisé en ce que**
l'au moins un émetteur de signaux est choisi dans le groupe constitué d'un haut-parleur, d'un dispositif d'affichage optique, d'une électrode et d'un stimulateur vibratoire, dans lequel le signal de retour d'information est de préférence choisi dans le groupe constitué d'un stimulus visuel, d'un stimulus tactile, d'un stimulus vibrotactile, d'un stimulus galvanique et d'un stimulus gustatif.

7. Dispositif selon l'une ou plusieurs des revendications précédentes
**caractérisé en ce que**
le signal de retour d'information peut être émis selon plusieurs niveaux d'intensité, dans lequel une première intensité est de préférence déclenchée lorsque le seuil de retour d'information est simplement dépassé, une deuxième intensité est déclenchée lorsque le seuil de retour d'information est dépassé d'un facteur 1,5, et une troisième intensité est déclenchée lorsque le seuil de retour d'information est dépassé d'un facteur 2.

8. Dispositif selon une ou plusieurs des revendications précédentes
**caractérisé en ce que**
les instructions relatives à un programme d'exercices sont émises visuellement ou acoustiquement.

9. Dispositif selon l'une ou plusieurs des revendications précédentes
**caractérisé en ce que**
le dispositif comprend un module d'entrée destiné à permettre au porteur du dispositif de saisir des entrées, lesquelles sont transmises à l'unité de traitement à des fins d'évaluation, dans lequel les entrées comprennent de préférence des entrées vocales, des entrées gestuelles, des entrées sonores ou des entrées au moyen de touches, et/ou dans lequel un porteur du dispositif peut de préférence, au moyen des entrées, réagir aux instructions relatives à l'exécution d'un programme d'exercices en signalant de préférence une approbation, un refus ou une interruption, et l'unité de traitement est configurée pour adapter le déroulement de l'exécution du programme d'exercices sur la base des entrées.

10. Dispositif selon l'une ou plusieurs des revendications précédentes
**caractérisé en ce que**
l'unité de traitement est configurée pour enregistrer, pendant l'exécution d'un premier programme d'exercices, les valeurs de fluctuation pour les schémas de mouvement et pour sélectionner, sur la base des valeurs de fluctuation enregistrées, des exercices destinés à un second programme d'exercices ultérieur.

11. Dispositif selon l'une des revendications précédentes
**caractérisé en ce**
**qu'**une sélection des exercices pour le programme d'exercices est effectuée sur la base d'une comparaison des valeurs de fluctuation obtenues lors de l'exécution du premier programme d'exercices avec des valeurs de référence, dans lequel les exercices sont de préférence sélectionnés lorsque la médiane 50 des valeurs de fluctuation mesurées est supérieure à la médiane 95 des valeurs de médiane 50 de l'ensemble des sujets d'essai (valeurs de référence) pour un groupe d'âge ou de sexe donné.

12. Dispositif selon l'une des revendications précédentes
**caractérisé en ce que**
lors d'une exécution du programme d'exercices, un retour d'information relatif à l'exécution correcte des exercices du programme d'exercices est émis sur la base d'une analyse de similarité avec une exécution précédente du programme d'exercices.

13. Dispositif selon l'une ou plusieurs des revendications précédentes
**caractérisé en ce**
**qu'**au moins un capteur destiné à mesurer la modification d'une position corporelle et/ou d'un schéma de mouvement du porteur du dispositif comprend un ou plusieurs gyromètres et/ou
un ou plusieurs accéléromètres, dans lequel l'au moins un capteur destiné à mesurer la modification d'une position corporelle et/ou d'un schéma de mouvement du porteur présente de préférence plusieurs gyromètres et/ou plusieurs accéléromètres disposés orthogonalement les uns par rapport aux autres, lesquels déterminent la variation de la vitesse angulaire et/ou l'accélération des mouvements vers l'avant, vers l'arrière et latéraux du corps.

14. Kit destiné à la mise en œuvre d'un apprentissage adaptatif de l'équilibre, comprenant
i. un dispositif comprenant
a. une unité de traitement
b. au moins un capteur destiné à mesurer la modification d'une position corporelle et/ou d'un schéma de mouvement du porteur du dispositif, lequel peut transmettre des données de mesure à l'unité de traitement,
c. un module d'invitation destiné à émettre, à destination du porteur du dispositif, des instructions relatives à un programme d'exercices comprenant plusieurs schémas de mouvement
d. au moins un émetteur de signaux destiné à émettre un signal de retour d'information
ii. un produit-programme d'ordinateur destiné à être installé sur l'unité de traitement du dispositif, lequel comprend des instructions pour
a. émettre, à destination du porteur du dispositif, des instructions relatives à un programme d'exercices comprenant plusieurs schémas de mouvement,
b. mesurer, au moyen d'au moins un capteur pendant l'exécution du programme d'exercices, la modification d'une position corporelle et/ou d'un schéma de mouvement du porteur du dispositif, et déterminer les valeurs de fluctuation à partir des données de mesure
c. émettre un signal de retour d'information au moyen de l'émetteur de signaux pendant le programme d'exercices si les valeurs de fluctuation dépassent un seuil de retour d'information, dans lequel les valeurs de fluctuation pour les schémas de mouvement sont enregistrées pendant la première exécution du programme d'exercices et le seuil de retour d'information est calculé pour la seconde exécution du programme d'exercices sur la base des valeurs de fluctuation enregistrées, et le signal de retour d'information est généré au moyen de l'émetteur de signaux pendant la seconde exécution du programme d'exercices si les valeurs de fluctuation dépassent le seuil de retour d'information calculé, dans lequel un élément compteur compte un nombre d'exécutions du programme d'exercices et permet ainsi de déterminer si les valeurs de fluctuation appartiennent à une première ou à une seconde exécution du programme d'exercices.

15. Produit-programme d'ordinateur destiné à être installé sur un dispositif comprenant
a. une unité de traitement
b. au moins un capteur destiné à mesurer la modification d'une position corporelle et/ou d'un schéma de mouvement du porteur du dispositif, lequel peut transmettre des données de mesure à l'unité de traitement,
c. un module d'invitation destiné à émettre, à destination du porteur du dispositif, des instructions relatives à un programme d'exercices comprenant plusieurs schémas de mouvement
d. au moins un émetteur de signaux destiné à émettre un signal de retour d'information
dans lequel le produit-programme d'ordinateur présente des instructions pour mettre en œuvre un entraînement adaptatif de l'équilibre comprenant les étapes de procédé consistant à
a. émettre, à destination d'un porteur du dispositif, des instructions relatives à un programme d'exercices comprenant plusieurs schémas de mouvement,
b. mesurer, au moyen d'au moins un capteur pendant l'exécution du programme d'exercices, la modification d'une position corporelle et/ou d'un schéma de mouvement du porteur du dispositif, et déterminer les valeurs de fluctuation à partir des données de mesure
c. émettre un signal de retour d'information au moyen de l'émetteur de signaux pendant le programme d'exercices si les valeurs de fluctuation dépassent un seuil de retour d'information, dans lequel les valeurs de fluctuation correspondent aux schémas de mouvement sont enregistrées pendant la première exécution du programme d'exercices et le seuil de retour d'information est calculé pour la seconde exécution du programme d'exercices sur la base des valeurs de fluctuation enregistrées, et le signal de retour d'information est généré au moyen de l'émetteur de signaux pendant la seconde exécution du programme d'exercices si les valeurs de fluctuation dépassent le seuil de retour d'information calculé, dans lequel un élément compteur compte un nombre d'exécutions du programme d'exercices et permet ainsi de déterminer si les valeurs de fluctuation appartiennent à une première ou à une seconde exécution du programme d'exercices.
